# EUROPEAN PATENT SPECIFICATION

(11) **EP 3 464 626 B1**
(45) Date of publication and mention of the grant of the patent: **06.04.2022**
(21) Application number: 17729636.5
(22) Date of filing: 26.05.2017
(51) Int. Cl.: C12Q 1/68, C12Q 1/6844, G16B 25/00, G16B 35/00, G16C 20/60, G16H 15/00, G16H 50/30, G16B 30/00, G16B 30/10

(54) **METHODS FOR DETECTING GENETIC VARIATIONS**
VERFAHREN ZUM NACHWEIS VON GENETISCHEN VARIATIONEN
MÉTHODES DE DÉTECTION DE VARIATIONS GÉNÉTIQUES

(30) Priority: 27.05.2016 US 201662342839 P
(43) Date of publication of application: 10.04.2019
(60) Divisional application: 21215237.5
(73) Proprietor: Sequenom, Inc., San Diego, CA 92121 (US)
(72) Inventor: JENSEN, Taylor Jacob, San Diego California 92131 (US); EHRICH, Mathias, San Diego California 92109 (US); VAN DEN BOOM, Dirk, Encinitas California 92024 (US); TYNAN, John Allen, San Diego California 92128 (US); KIM, Sung Kyun, San Diego California 92129 (US); BURCHAM, Timothy S., Encinitas California 92024 (US); ELLISON, Christopher K., San Diego California 92122 (US); SUN, Youting, San Diego California 92129 (US)
(74) Representative: Vossius & Partner Patentanwälte Rechtsanwälte mbB
(86) International application number: PCT/US2017/034826
(87) International publication number: WO 2017/205826

(56) References cited:
- WO-A2-2008/157264
- US-A1- 2005 037 388
- ARMOUR JOHN A L ET AL: "Accurate, high-throughput typing of copy number variation using paralogue ratios from dispersed repeats", NUCLEIC ACIDS RESEARCH, INFORMATION RETRIEVAL LTD, vol. 35, no. 3, 14 December 2006 (2006-12-14), page E19, XP002473374, ISSN: 0305-1048, DOI: 10.1093/NAR/GKL1089
- WALKER S ET AL: "Multiplex Paralogue Ratio Tests for accurate measurement of multiallelic CNVs", GENOMICS, ACADEMIC PRESS, SAN DIEGO, US, vol. 93, no. 1, 1 January 2009 (2009-01-01), pages 98-103, XP025745539, ISSN: 0888-7543, DOI: 10.1016/J.YGENO.2008.09.004 [retrieved on 2008-10-22]
- C. D. VEAL ET AL: "Automated design of paralogue ratio test assays for the accurate and rapid typing of copy number variation", BIOINFORMATICS., vol. 29, no. 16, 6 June 2013 (2013-06-06), pages 1997-2003, XP055335230, GB ISSN: 1367-4803, DOI: 10.1093/bioinformatics/btt330
- C. K. ELLISON, ET AL: "Using Targeted Sequencing of Paralogous Sequences for Noninvasive Detection of Selected Fetal Aneuploidies.", CLINICAL CHEMISTRY, vol. 62, no. 12, 30 September 2016 (2016-09-30), pages 1621-1629, XP009195289, DOI: 10.1373/clinchem.2016.260034

## Description

### FIELD OF THE INVENTION

The present invention is defined by the claims. Generally described herein are methods, processes, machines and apparatuses for detecting genetic variations. In some cases, the technology described herein relates to non-invasive assessment of a chromosome abnormality, which include, without limitation, prenatal tests for detecting an aneuploidy (e.g., trisomy 21 (Down syndrome) and trisomy 18 (Edward syndrome)) and copy number variations.

### BACKGROUND

Genetic information of living organisms (e.g., animals, plants and microorganisms) and other forms of replicating genetic information (e.g., viruses) is encoded in deoxyribonucleic acid (DNA) or ribonucleic acid (RNA). Genetic information is a succession of nucleotides or modified nucleotides representing the primary structure of chemical or hypothetical nucleic acids. In humans, the complete genome contains about 30,000 genes located on 24 chromosomes (i.e., 22 autosomes, an X chromosome and a Y chromosome; see The Human Genome, T. Strachan, BIOS Scientific Publishers, 1992). Each gene encodes a specific protein, which after expression via transcription and translation fulfills a specific biochemical function within a living cell.

Many medical conditions are caused by one or more genetic variations. Certain genetic variations cause medical conditions that include, for example, hemophilia, thalassemia, Duchenne Muscular Dystrophy (DMD), Huntington's Disease (HD), Alzheimer's Disease and Cystic Fibrosis (CF) (Human Genome Mutations, D. N. Cooper and M. Krawczak, BIOS Publishers, 1993). Such genetic diseases can result from an addition, substitution, or deletion of a single nucleotide in DNA of a particular gene. Certain birth defects are caused by a chromosomal abnormality, also referred to as an aneuploidy, such as Trisomy 21 (Down's Syndrome), Trisomy 13 (Patau Syndrome), Trisomy 18 (Edward's Syndrome), Monosomy X (Turner's Syndrome) and certain sex chromosome aneuploidies such as Klinefelter's Syndrome (XXY), for example. Another genetic variation is fetal gender, which can often be determined based on sex chromosomes X and Y.

Identifying one or more genetic variations (e.g., copy number alterations, single nucleotide variations, chromosome alterations, translocations, deletions, insertions, and the like) or variances can lead to diagnosis of, or determining predisposition to, a particular medical condition. Identifying a genetic variance can result in facilitating a medical decision and/or employing a helpful medical procedure. In certain cases, identification of one or more genetic variations or variances involves the analysis of circulating cell-free nucleic acid. Circulating cell-free nucleic acid (CCF-NA), such as cell-free DNA (CCF-DNA) for example, is composed of DNA fragments that originate from cell death and circulate in peripheral blood. Additionally, cell-free fetal DNA (CFF-DNA) can be detected in the maternal bloodstream and used for various noninvasive prenatal diagnostics.

### SUMMARY

The present invention is defined by the claims. In a first aspect, the invention relates to a method for determining the presence or absence of a genetic variation, comprising: (a) amplifying in a single reaction a plurality of sets of paralogous polynucleotide species from nucleic acid in a sample, the amplifying comprising contacting the nucleic acid with a plurality of amplification primers under amplification conditions to obtain a plurality of amplified sets of paralogous polynucleotide species, wherein (i) each set of the plurality of amplified sets of paralogous polynucleotide species includes paralogous polynucleotide species located on a target chromosome and one or more reference chromosomes; and (ii) each set of the plurality of paralogous polynucleotide species is amplified by a different single pair of amplification primers of the plurality of amplification primers and each primer of the plurality of amplification primers is complementary to less than 20 positions in a human genome; (b) determining, for each set of the plurality of amplified sets of paralogous polynucleotide species the amount of the paralogous polynucleotide species from the target chromosome and the amount of the paralogous polynucleotide species from the one or more reference chromosomes; (c) determining, for each set of the plurality of amplified sets, a paralog ratio between the amount of the paralogous polynucleotide species from the target chromosome and the amount of the paralogous polynucleotide species from the one or more reference chromosomes; (d) determining, for the paralog ratio determined for each set of the plurality of amplified sets of paralogous polynucleotide species, a z-score; (e) determining, for the sample, a sample z-score based on the z-score determined for each set of the plurality of amplified sets of paralogous polynucleotide species; and (f) determining, for the sample, the presence or absence of the genetic variation based on the sample z-score.

In a second aspect, the invention relates to a use of a composition in the method according to the invention, wherein the composition comprises a mixture of a plurality of amplification primer pairs that can amplify the plurality of sets of paralogous polynucleotide species as defined in accordance with the method of the invention, wherein the each primer of the plurality of amplification primer pairs is complementary to less than 20 positions in a human genome, wherein the amplification primers for each of the paralogous polynucleotide species sets produces two equal-sized amplicons with a size between 60 to 100 bp.

In a third aspect, the invention relates to a use of a kit in the method according to the invention, wherein the kit comprises the composition as defined in accordance with the second aspect of the invention, and a DNA polymerase.

In a fourth aspect, the invention relates to a system for determining the presence or absence of a genetic variation, comprising (a) a component for amplifying in a single reaction a plurality of sets of paralogous polynucleotide species from nucleic acid in a sample, the amplifying comprising contacting the nucleic acid with a plurality of amplification primers under amplification conditions to obtain a plurality of amplified sets of paralogous polynucleotide species, wherein (i) each set of the plurality of amplified sets of paralogous polynucleotide species includes paralogous polynucleotide species located on a target chromosome and one or more reference chromosomes; and (ii) each set of the plurality of paralogous polynucleotide species is amplified by a different single pair of amplification primers of the plurality of amplification primers and each primer of the plurality of amplification primers is complementary to less than 20 positions in a human genome; (b) a component for determining, for each set of the plurality of amplified sets of paralogous polynucleotide species, the amount of the paralogous polynucleotide species from the target chromosome and the amount of the paralogous polynucleotide species from the one or more reference chromosomes; (c) a component for: (i) determining, for each set of the plurality of amplified sets, a paralog ratio between the amount of the paralogous polynucleotide species from the target chromosome and the amount of the paralogous polynucleotide species from the one or more reference chromosomes; (ii) determining, for the paralog ratio determined for each set of the plurality of amplified sets of paralogous polynucleotide species, a z-score; and (iii) determining, for the sample, a sample z-score based on the z-score determined for each set of the plurality of amplified sets of paralogous polynucleotide species; and (d) a component for determining, for the sample, the presence or absence of the genetic variation based on the sample z-score.

Provided herein are methods useful for determining the presence or absence of a genetic variation. In some cases the genetic variation is a chromosomal aneuploidy detected in a sample of circulating cell free nucleic acid obtained from a pregnant female. For example, the methods can specifically enrich for a selected set of paralog targets to enable noninvasive detection of fetal trisomy 21 (T21) and trisomy 18 (T18), or other aneuploidies, using cell-free DNA (cfDNA) from maternal plasma. A method often amplifies multiple paralog targets in a single reaction. A method often measures amplified products using massively parallel sequencing and determines deviations from expected (euploid) paralog ratios based on the read depth from each paralog.

The methods provided herein are also useful for the detection of copy number variants (CNVs). CNV's have been associated with a number of diseases and conditions, including but not limited to, autism, reproductive challenges and cancer.

Also provided are systems, machines and computer program products that carry out processes, or parts of processes, described herein. Certain embodiments are described further in the following description, examples, claims and drawings.

### BRIEF DESCRIPTION OF THE DRAWINGS

The drawings illustrate certain embodiments of the technology and are not limiting. For clarity and ease of illustration, the drawings are not made to scale and, in some instances, various aspects may be shown exaggerated or enlarged to facilitate an understanding of particular embodiments.

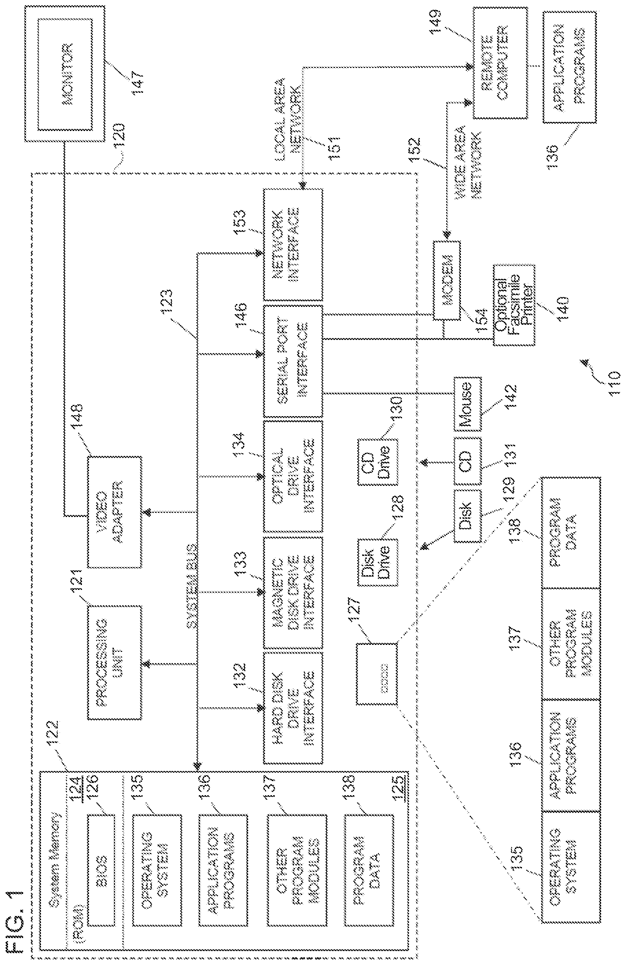

Fig. 1 shows an illustrative example of a system in which certain embodiments of the technology may be implemented.

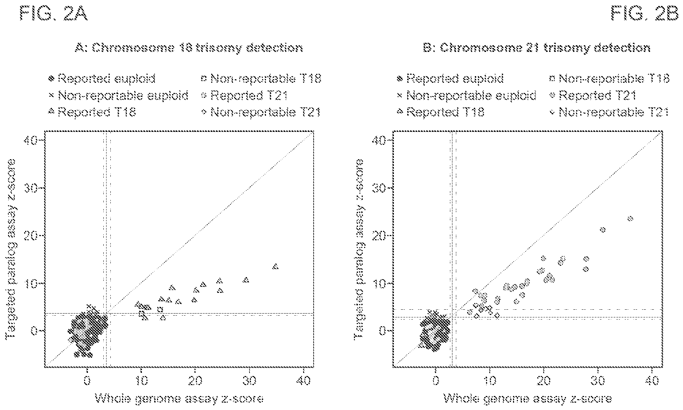

Fig. 2A and 2B shows fetal trisomy classification performance. Comparison of z-scores generated using MaterniT21^{®} PLUS Assay ground truth data versus targeted paralog assay data for (FIG. 2A) fetal chromosome 18 trisomy and (FIG. 2B) fetal chromosome 21 trisomy. Dashed lines represent regional bounds for which no classification was achieved; solid diagonal represents z-score equivalency between the two datasets. The two independent fetal trisomy classifiers are significantly correlated in both cases (F-test, chromosome 21: r²=0.85, p<10⁻¹⁵, chromosome 18: r²=0.79, p<10⁻⁶). The observed classification gap between affected and unaffected samples was greater in whole genome data than in targeted paralog assay data.

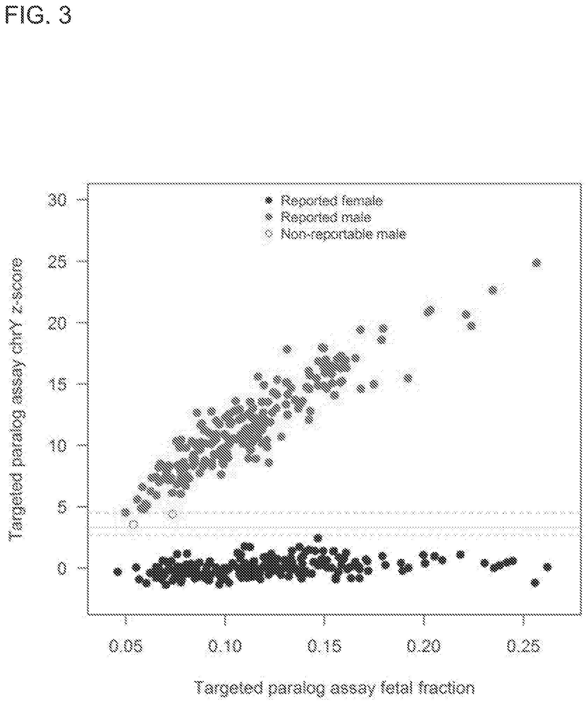

Fig. 3 shows fetal sex classification performance. Data are grouped by fetal sex as determined by the MaterniT21^{®} PLUS Assay ground truth data. Horizontal broken line represents heuristic threshold for fetal sex classification, with points above this line classified as male fetuses. Non-reportable male fetal samples are visible with lower than expected percent of total sequencing reads mapped to chromosome Y assays.

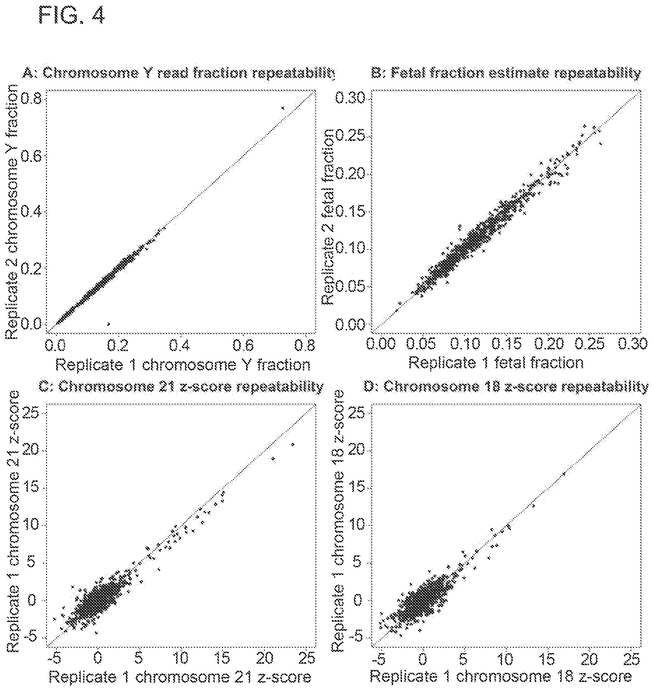

Fig. 4 shows reproducibility of targeted paralog assay data. (A) Comparison of percentage of reads mapped to chromosome Y assays in first versus second evaluation of data (fetal sex classification, r²=0.99, p<10⁻¹⁵, F-test). (B) Comparison of fraction fetal origin DNA estimates in first versus second evaluation of data (r²=0.97, p<10⁻¹⁵, F-test). (C) Comparison of z-scores for fetal chromosome 21 classification in first versus second evaluation of data (r²=0.87, p<10⁻¹⁵, F-test). (D) Comparison of z-scores for fetal chromosome 18 classification in first versus second evaluation of data (r²=0.75, p<10⁻¹⁵, F-test). In all cases, data were found to be highly robust to run-to-run variation observed during sequencing.

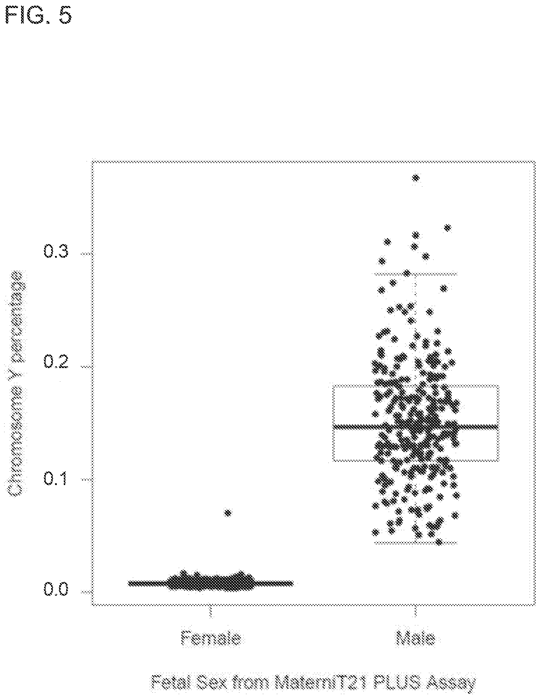

Fig. 5 shows fetal sex classification performance in classification model training dataset. Data are grouped by fetal sex as determined by the MaterniT21^{®} PLUS Assay. The percent of total sequencing reads mapped to chromosome Y assays is significantly greater in male fetus samples than in female fetus samples (p<10-15, Wilcoxon test).

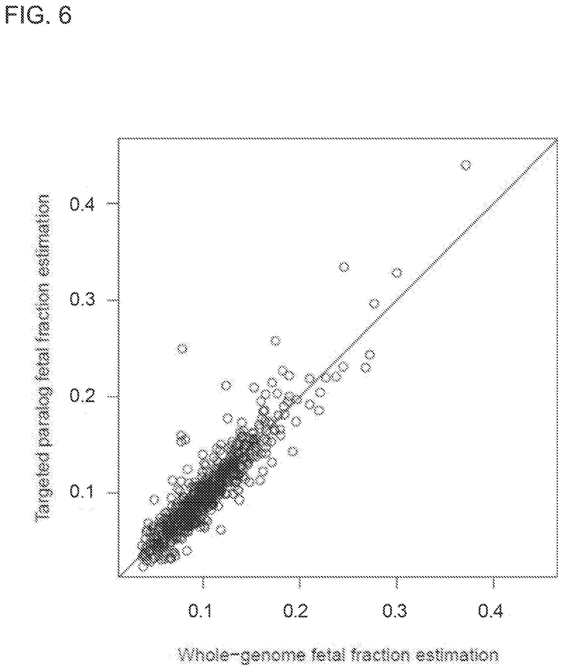

Fig. 6 shows an estimation of fetal fraction in classification model training dataset. Fetal fraction estimated by the MaterniT21^{®} PLUS Assay versus the estimation using the SNP panel integrated as part of the targeted paralog assay. The two independent estimates of fetal fraction are significantly correlated.

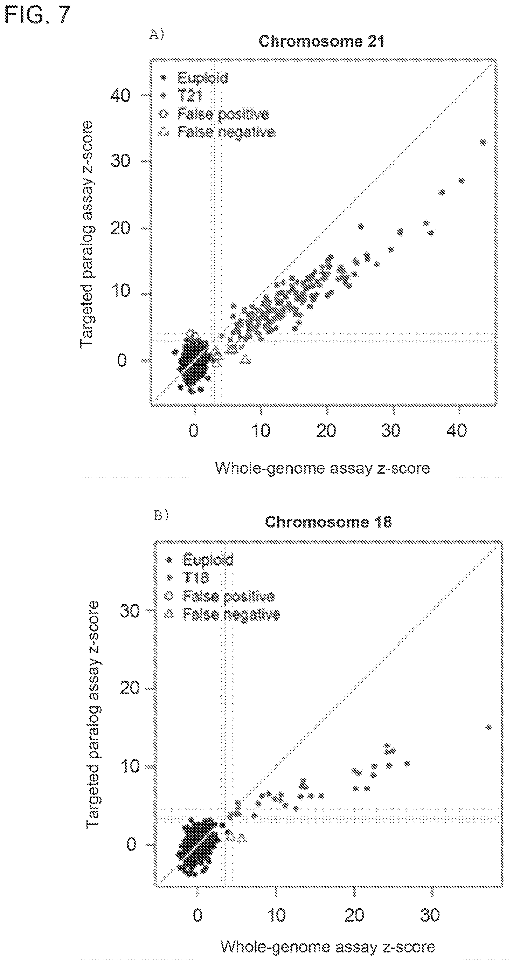

Fig. 7A and 7B shows fetal trisomy classification performance in classification model training dataset. Comparison of z-scores generated using MaterniT21^{®} PLUS Assay data versus targeted paralog assay data for (FIG. 7A) fetal chromosome 21 trisomy and (FIG. 7B) fetal chromosome 18 trisomy. Broken lines represent regional bounds for which no classification can be achieved; solid diagonal represents z-score equivalency between the two datasets. The two independent fetal trisomy classifiers are significantly correlated in both cases (F-test, chromosome 21: r²=0.86, p<10⁻¹⁵. chromosome 18: r2=0.84, p<10⁻¹⁵). In both cases, the magnitude of the z-score classifier is diminished in targeted paralog assay data versus MaterniT21^{®} PLUS Assay data.

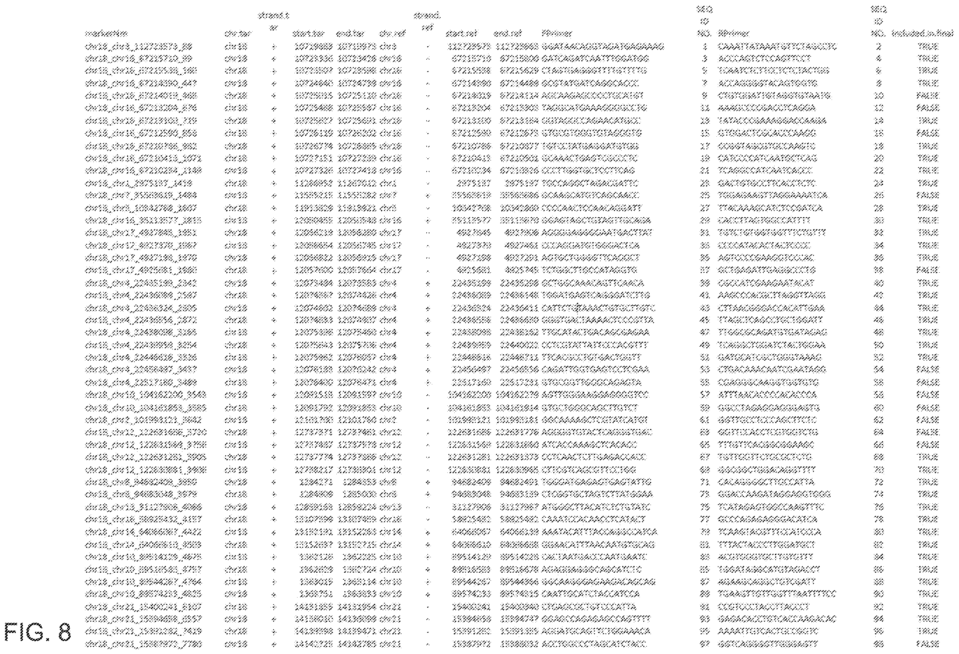

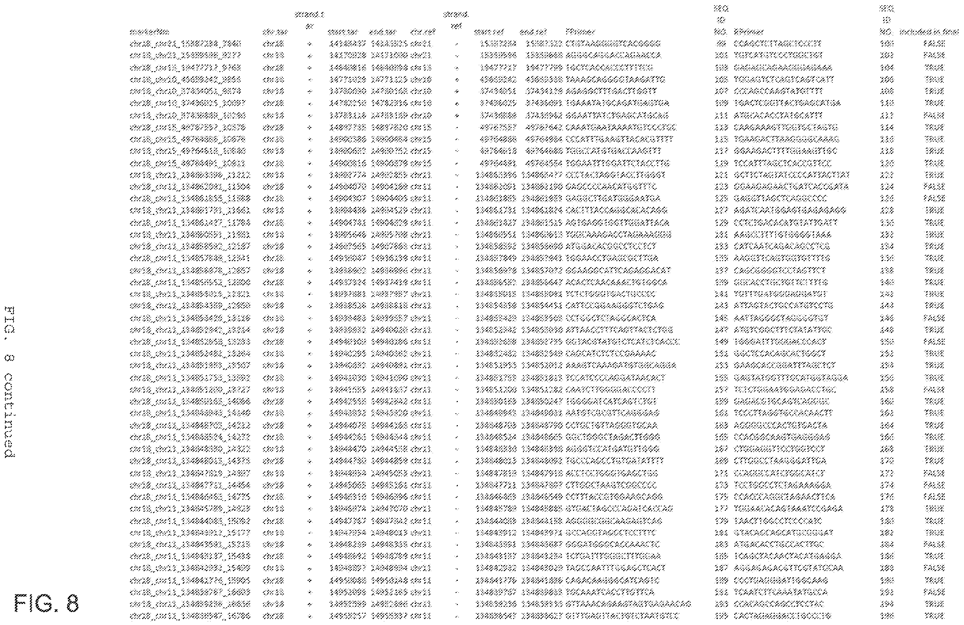

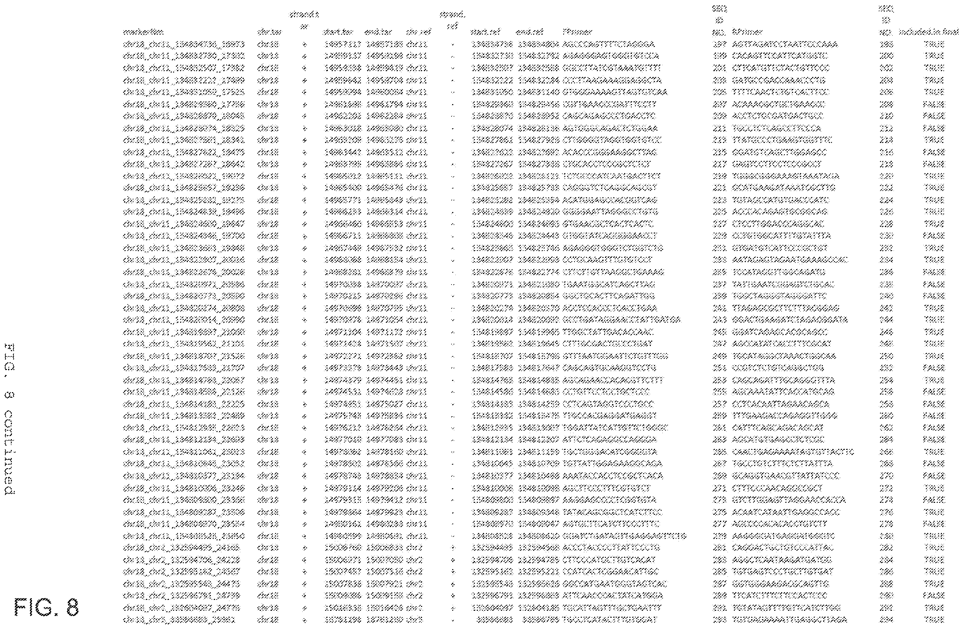

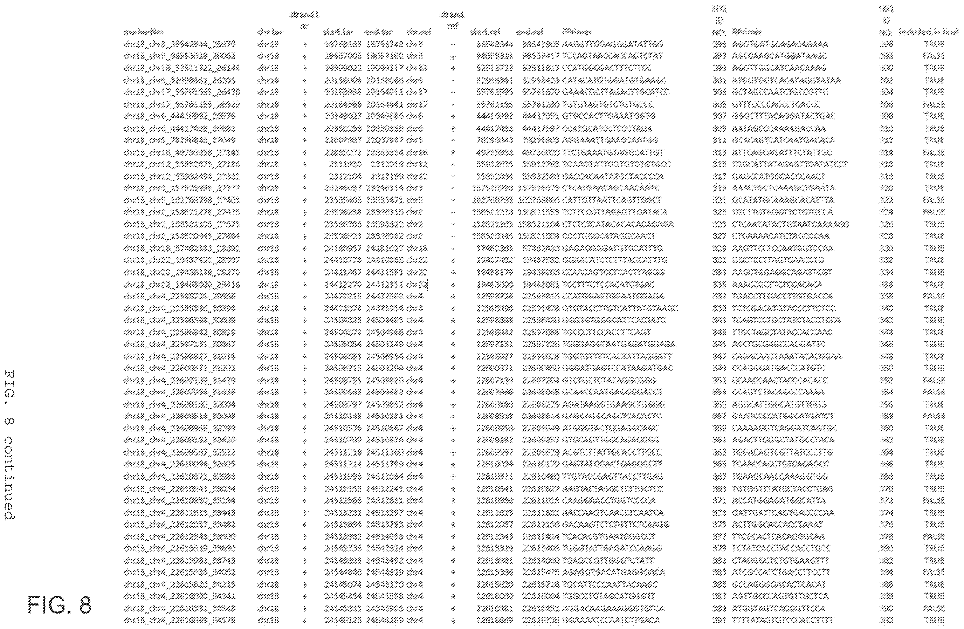

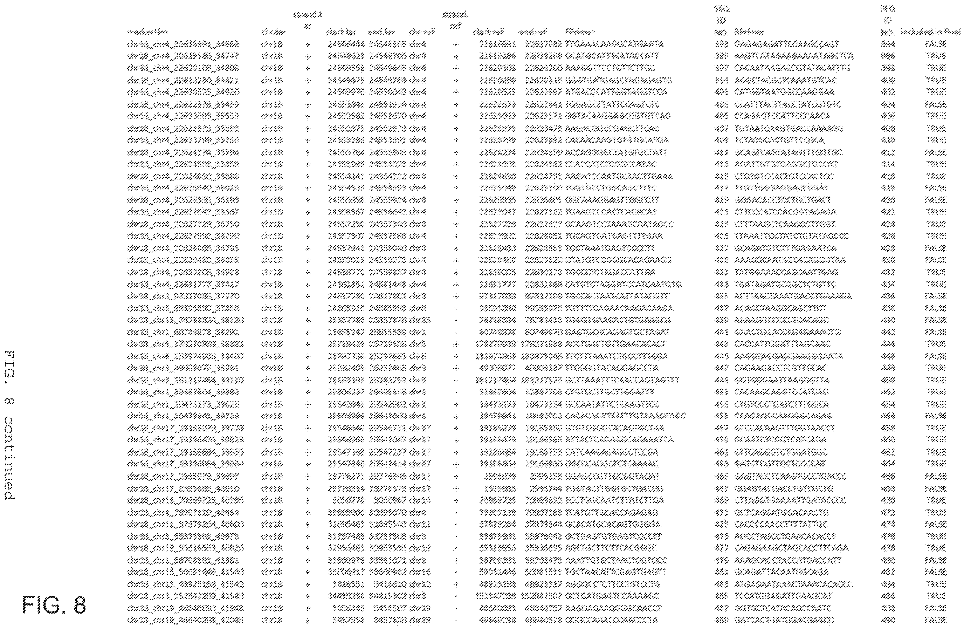

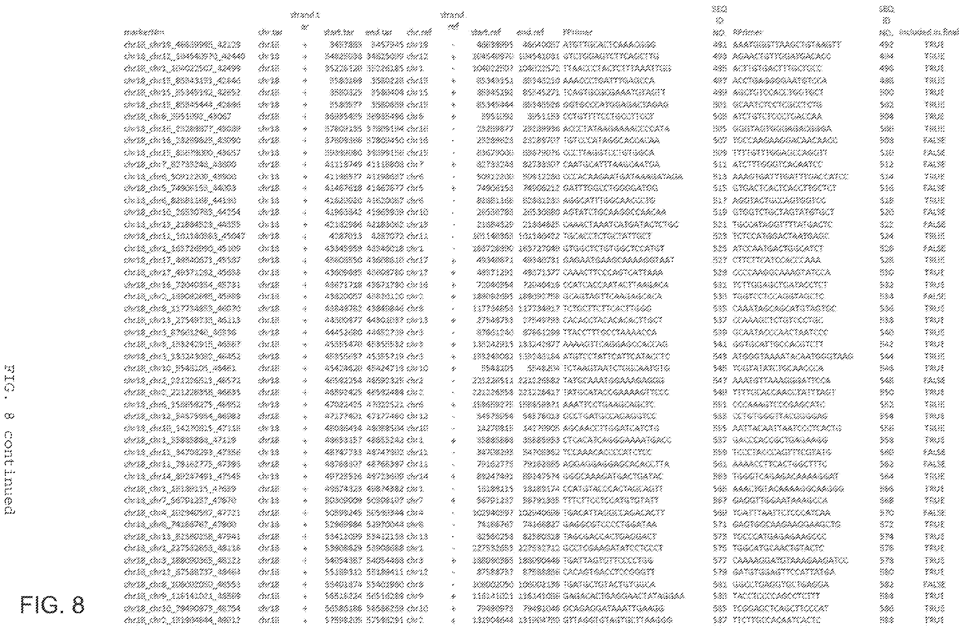

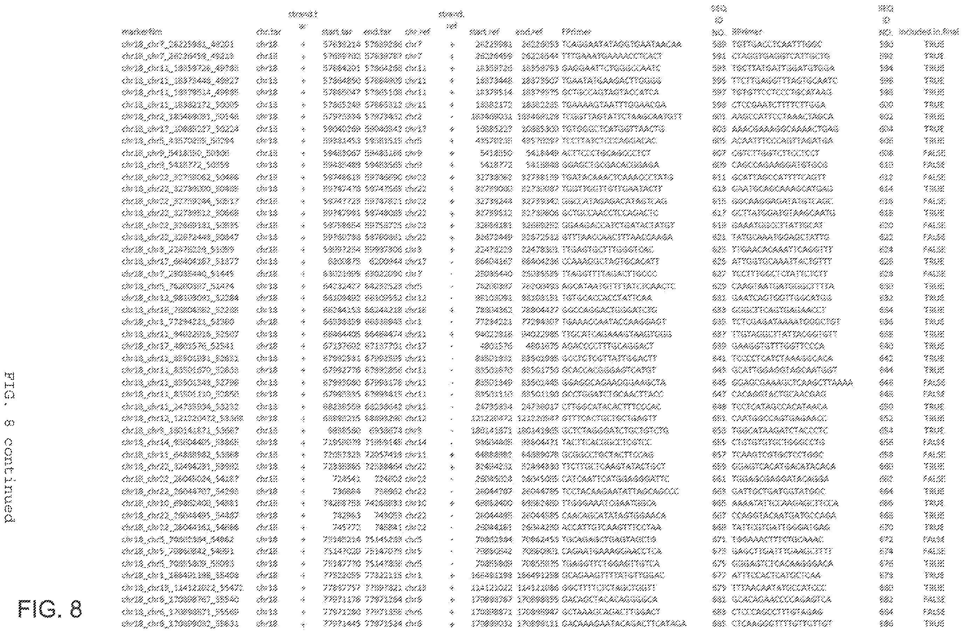

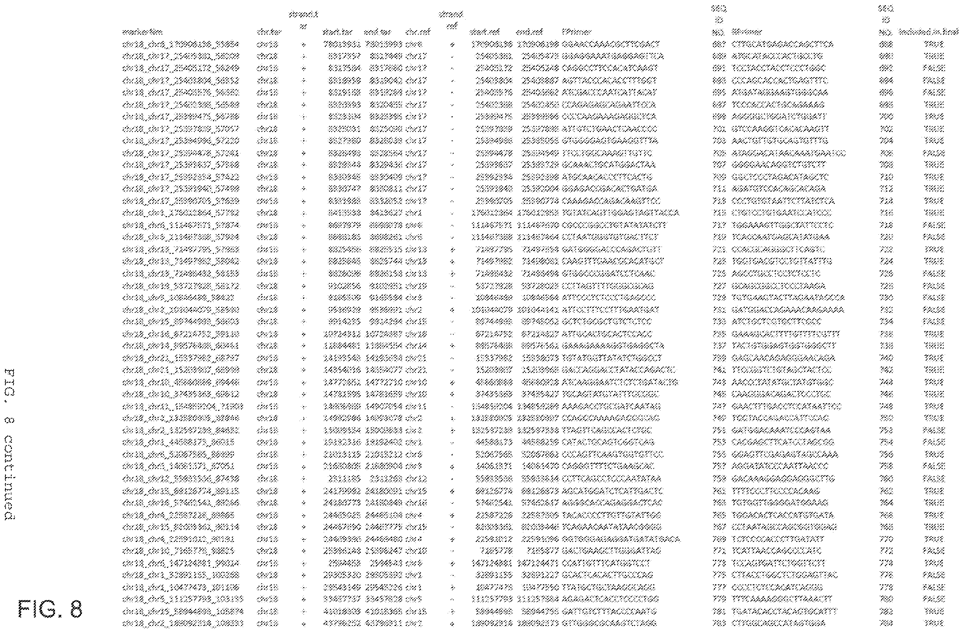

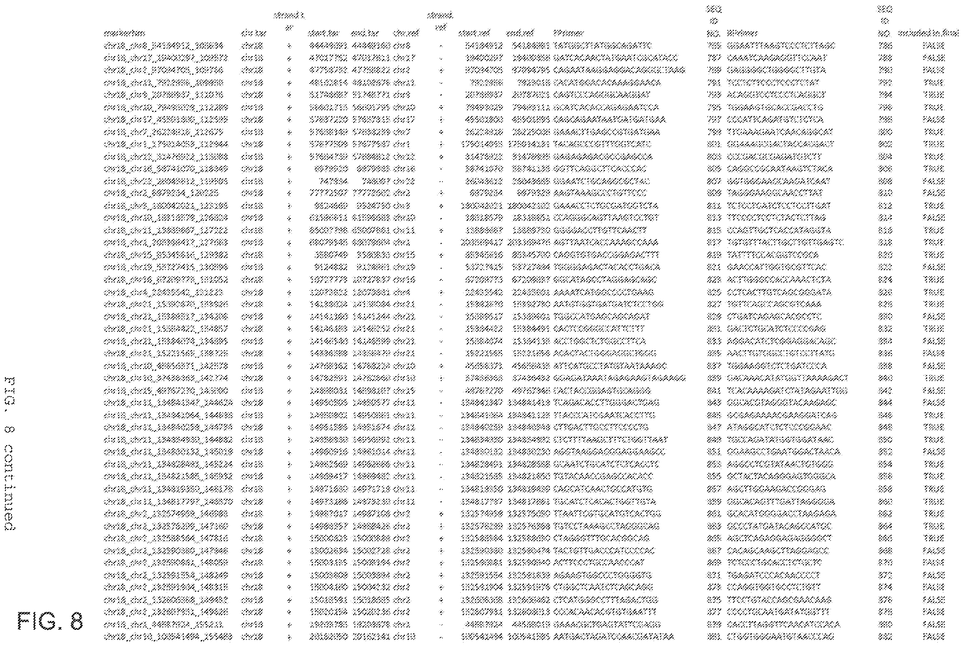

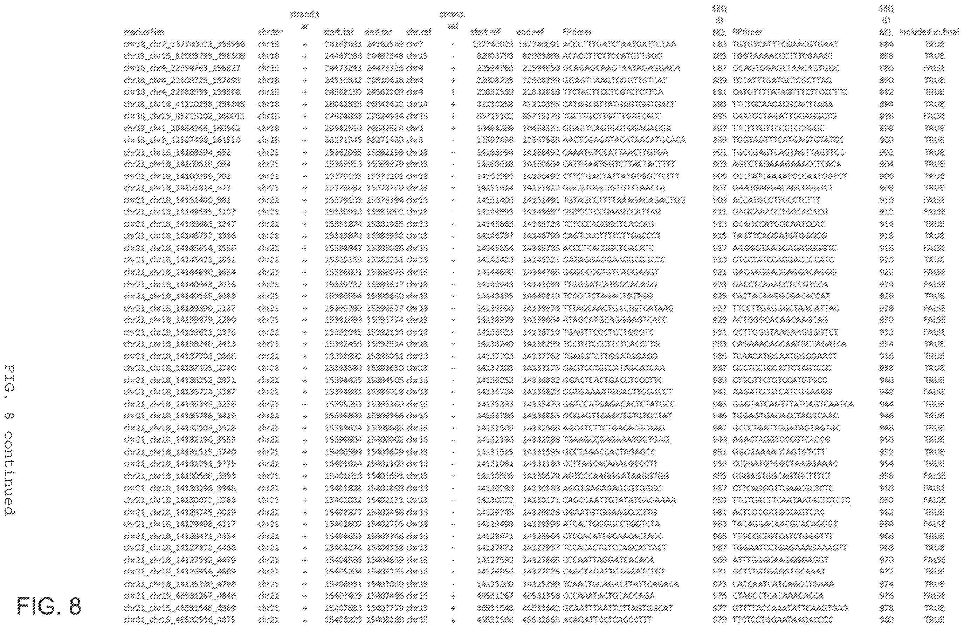

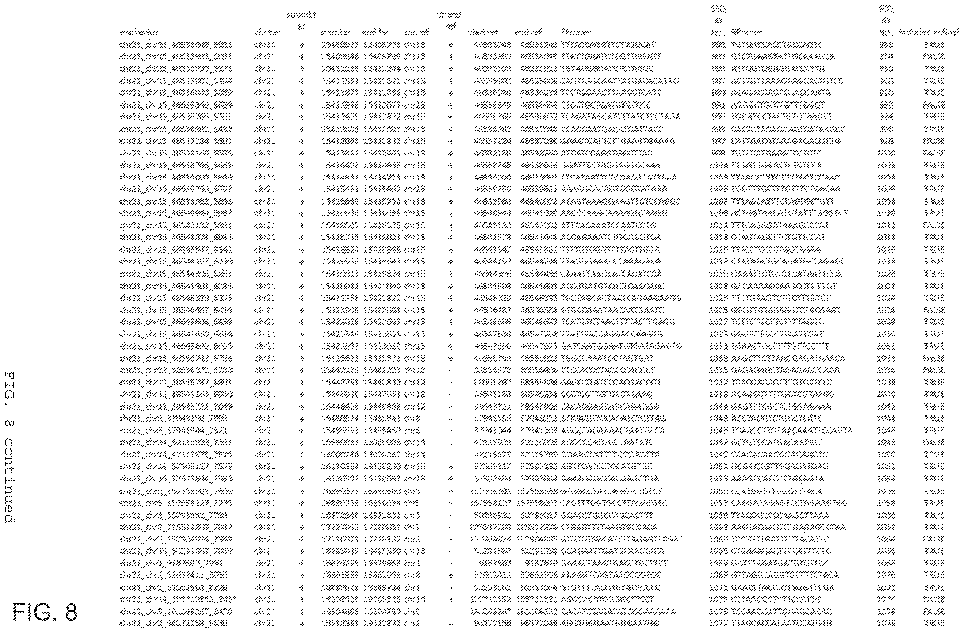

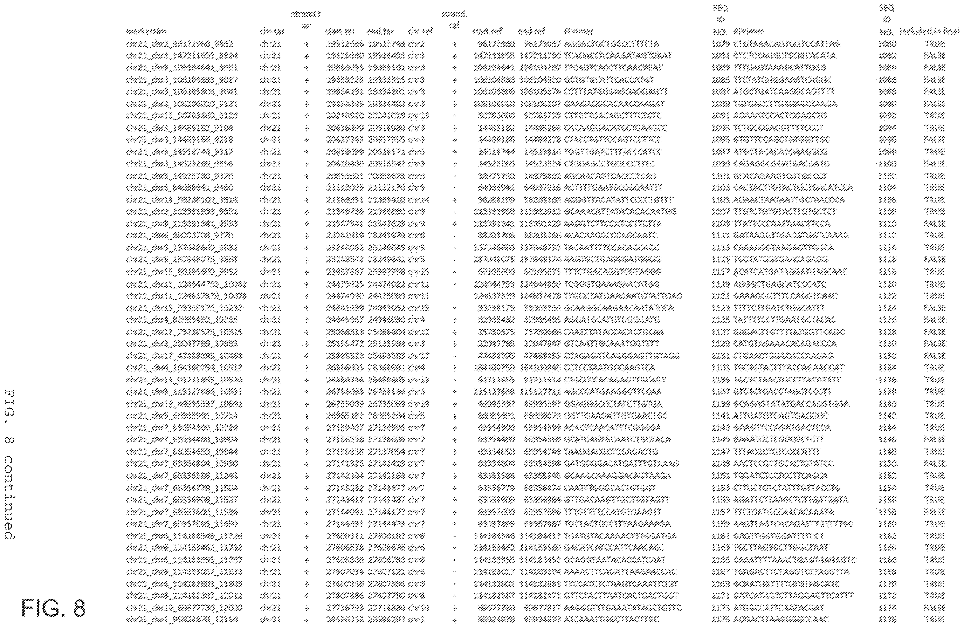

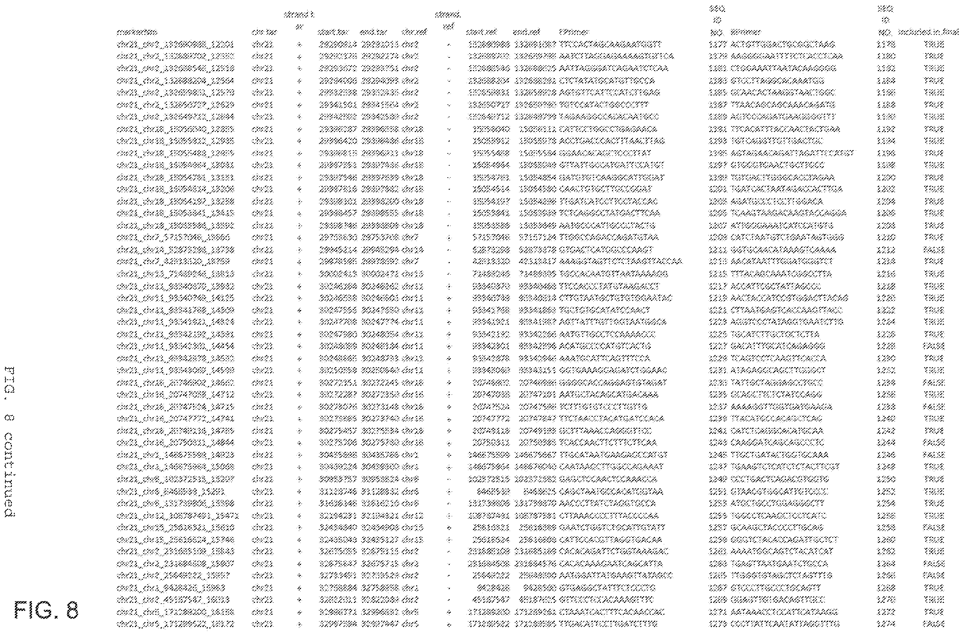

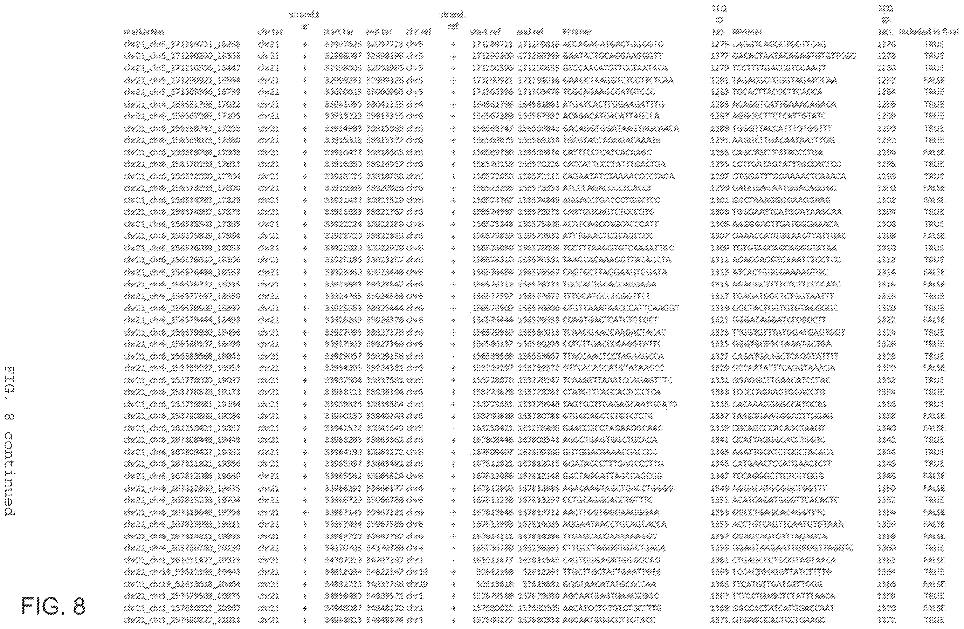

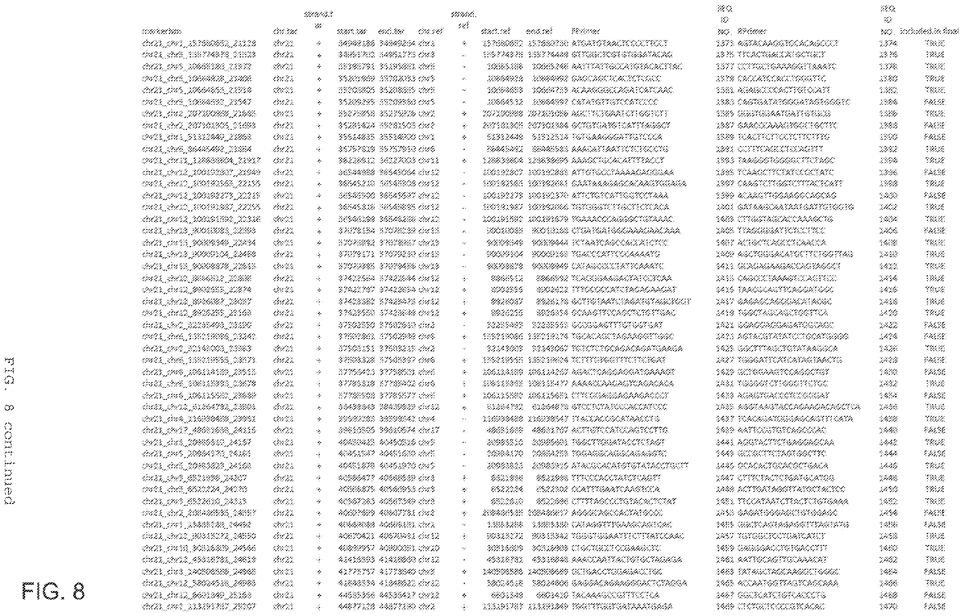

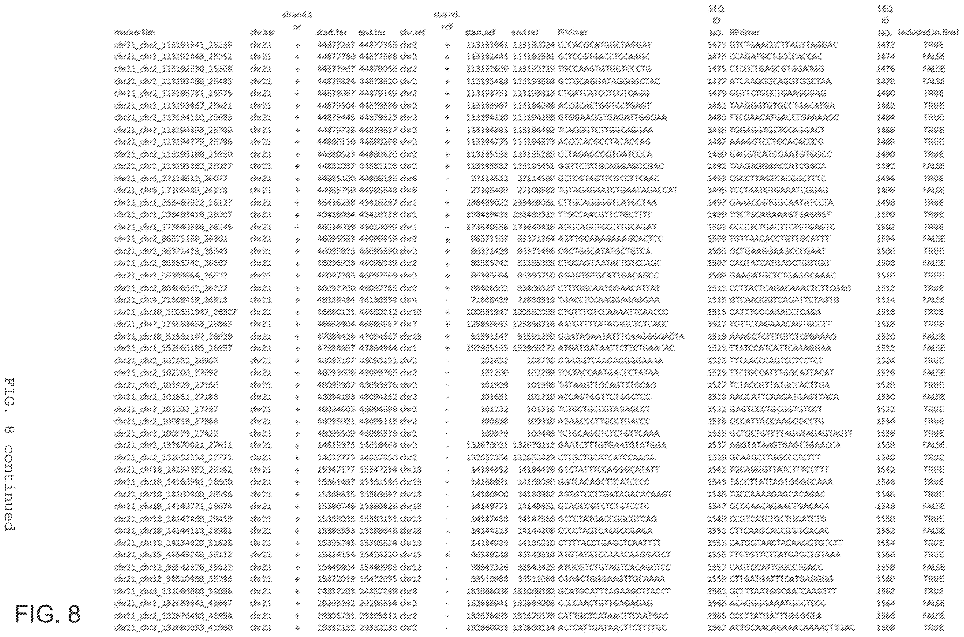

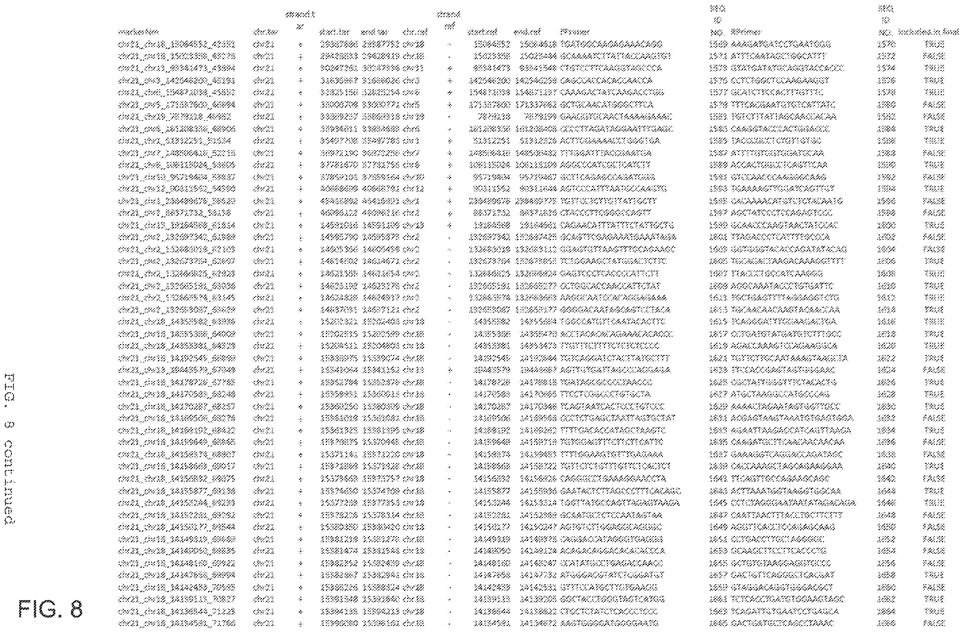

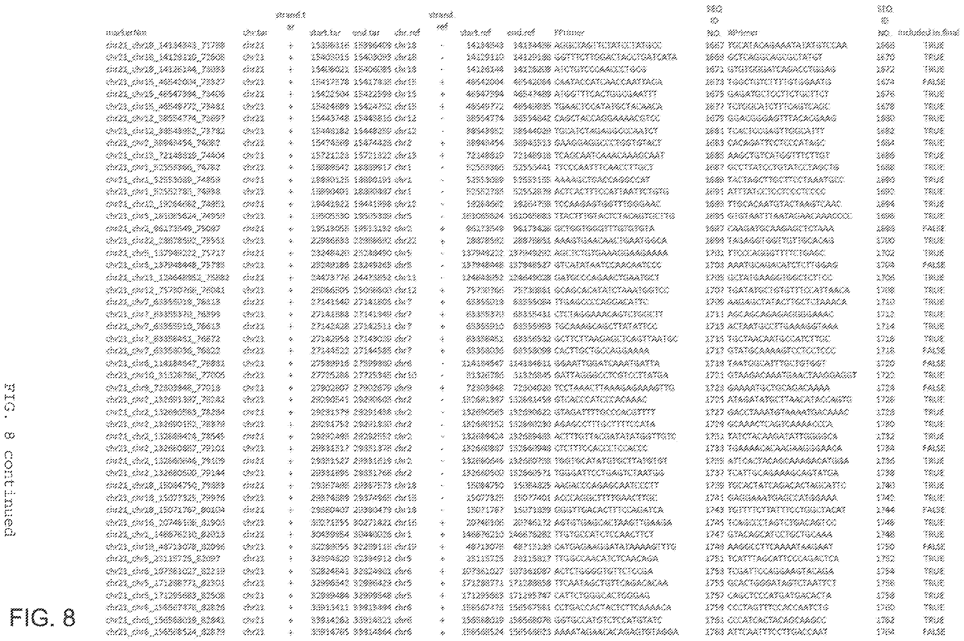

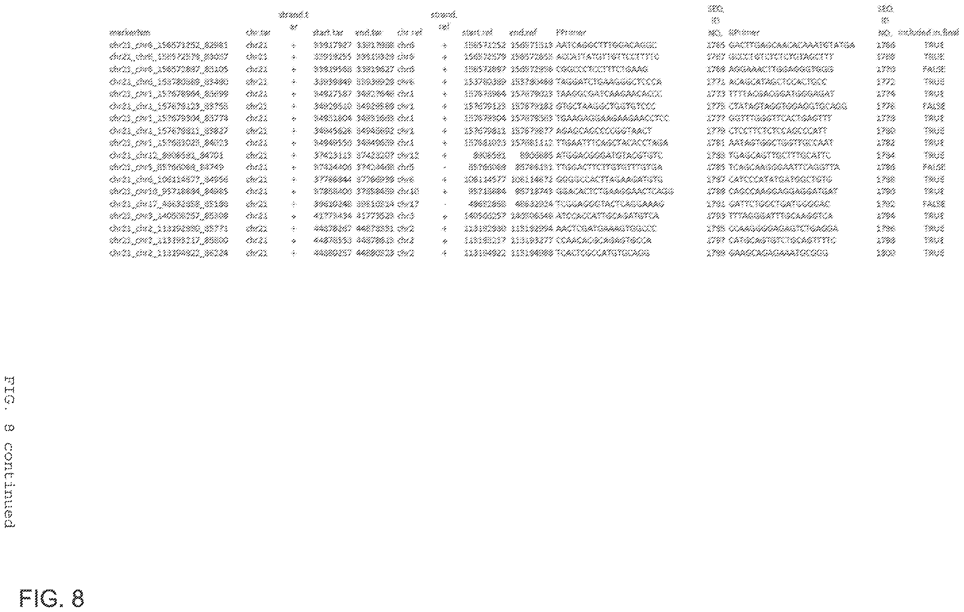

Fig. 8 lists paralogous polynucleotide species sets (assays) for chromosome 21 and chromosome 18. For each assay the target chromosome (21 or 18) position, reference chromosome position, forward primer, reverse primer and whether the particular assay was utilized in the testing of the method for aneuploidy detection is shown.

### DETAILED DESCRIPTION

Provided herein are methods useful for detecting genetic variations. In some cases, the technology relates to the non-invasive determination of the presence or absence of a chromosome abnormality.

For example, in some cases provided is a method for determining the presence or absence of a genetic variation, comprising:
a. amplifying in a single reaction, a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;
b. determining the amount of each amplified paralogous polynucleotide species in each of the sets;
c. determining a paralog ratio for each of the sets between the amount of each amplified paralogous polynucleotide species in each of the sets, thereby generating a plurality of paralog ratios; and
d. determining the presence or absence of the genetic variation based on the plurality of paralog ratios.

In some cases the genetic variant is a chromosomal abnormality. The chromosomal abnormality may be inherited. Or, the chromosomal abnormality may be a somatic mutation. In some cases the chromosomal abnormality is associated with cancer. In other cases, the chromosomal abnormality is associated with other genetic diseases. In some cases, the chromosomal abnormality is detected in fetal DNA. Or the chromosomal abnormality may be detected in the DNA of any subject (e.g., non-fetal male or female).

For example, methods described herein may be used to determine presence or absence of a fetal aneuploidy (e.g., full chromosome aneuploidy, partial chromosome aneuploidy or segmental chromosomal aberration (e.g., mosaicism, deletion and/or insertion)) for a test sample from a pregnant female bearing a fetus. Methods described herein sometimes detect trisomy for one or more chromosomes (e.g., chromosome 18, chromosome 21 or combination thereof) or segment thereof. Or, other aneuploidies may be detected. Provided herein are methods for non-invasive prenatal testing (NIPT) incorporating elements of targeted amplicon sequencing, but with targeting specifically designed to co-amplify exactly two paralogous targets using a single primer pair to facilitate self-normalization of each target through the determination of a locus-specific paralog ratio. One target paralog is mapped to a chromosome of interest (e.g., chromosomes 18 or 21) and a paired paralog sequence is mapped to an alternative autosome.

For example, provided in certain cases are methods for determining presence or absence of a chromosomal aneuploidy, that include: (a) amplifying in a single reaction at least 100 sets of paralogous polynucleotide species from nucleic acid in a sample of circulating cell free nucleic acid of a pregnant female comprising fetally derived and maternally derived nucleic acid, comprising contacting the circulating cell free nucleic acid with amplification primers under amplification conditions, wherein the paralogous polynucleotide species of each of the sets are amplified by a single pair of amplification primers and each primer of the pair of amplification primers is complementary to less than 20 positions in a human genome; (b) determining the amount of each amplified paralogous polynucleotide species in each of the sets; (c) determining a paralog ratio for each of the sets between the amount of each amplified paralogous polynucleotide species in each of the sets, thereby generating a plurality of paralog ratios; and (d) determining the presence or absence of a chromosomal aneuploidy based on the plurality of paralog ratios. Various cases are described in more detail herein.

### Sets of paralogous polynucleotide species

The term "sets of paralogous polynucleotide species" refers to nucleotide sequence species located on different chromosomes at different loci that share a significant level of sequence identity. One paralogous polynucleotide species in a set is located in one chromosome and the other paralogous polynucleotide species of a set is located in another chromosome.

Paralogous polynucleotide species of a set share about 50%, 60%, 70%, 80%, 85%, 86%, 87%, 88%, 89%, 90%, 91%, 92%, 93% or 94%, and all intermediate values thereof, identity to one another in some cases. Paralogous polynucleotide species of a set are "substantially identical" to one another to one another in some cases, which refers to species that share 95%, 96%, 97%, 98% or 99% identity, or greater than 99% identity, with one another. In certain cases, paralogous polynucleotide species of a set may be identical to one another with the exception of one or two base pair mismatches. For example, paralogous polynucleotide species in a set may be identical to one another with the exception of one or two base pair mismatch for a nucleotide sequence species length of about 100 base pairs (e.g., about 60, 62, 64, 66, 68, 70, 72, 74, 76, 78, 80, 82, 84, 86, 88, 90, 92, 94, 96, 98, 100, 102, 104, 106, 108, 110, 112, 114, 116, 118 or 120 base pair sequence length). Paralogous polynucleotide species sometimes have a common evolutionary origin and sometimes are duplicated over time in a genome of interest. Paralogous polynucleotide species sometimes conserve sequence and gene structure (e.g., number and relative position of introns and exons and often transcript length).

A "set" includes paralogous polynucleotide species located on a target chromosome and one or more reference chromosomes. The term "reference chromosome" refers to a chromosome that includes a paralogous polynucleotide species as a subsequence, and is a chromosome not associated with a particular chromosome abnormality being screened. For example, in a prenatal screening method for Down syndrome (i.e., trisomy 21), chromosome 21 is the target chromosome and another chromosome (e.g., chromosome 5) is the reference chromosome. In certain cases, a reference chromosome can be associated with a chromosome abnormality. For example, chromosome 21 can be the target chromosome and chromosome 18 can be the reference chromosome when screening for Down syndrome, and chromosome 18 can the target chromosome and chromosome 21 can be the reference chromosome when screening for Edward syndrome. A set of paralogous polynucleotide species is sometimes referred to as an assay. Or, other sets of chromosomes may be assayed.

Base pair mismatches between paralogous polynucleotide species in a set are not significantly polymorphic in certain cases, and the nucleotides that give rise to the mismatches are present at a rate of over 95% of subjects and chromosomes in a given population (e.g., the same nucleotides that give rise to the mismatches are present in about 98%, 99% or over 99% of subjects and chromosomes in a population). Each paralogous polynucleotide species of a set, in its entirety, often is present in a significant portion of a population without modification (e.g., present without modification in about 97%, 98%, 99%, or over 99% of subjects and chromosomes in a population).

Paralogous polynucleotide species in a set may be of any convenient length. For example, paralogous polynucleotide species of a set can be about 50 to about 200 base pairs in length, or about 10, 15, 20, 25, 30, 35, 40, 45, 50, 55, 60, 65, 70, 75, 80, 85, 90, 95, 100, 200, base pairs in length. In some cases, a paralogous polynucleotide species in a set is about 100 base pairs in length (e.g., about 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118 or 120 base pairs in length). In certain cases, paralogous polynucleotide sequence species of a set are of identical length, and sometimes the paralogous polynucleotide sequence species of a set are of a different length (e.g., one species is longer by about 1 to about 20 nucleotides (e.g., about 2, 3, 4, 5, 6, 7, 8, 9, 10, 11, 12, 13, 14, 15, 16, 17, 18, 19, 20 nucleotides longer).

Paralogous polynucleotide species of a set are non-exonic in some cases, and sometimes the paralogous polynucleotide species of a set are intronic, partially intronic, partially exonic or partially non-exonic. In certain cases, the paralogous polynucleotide species of a set comprises an exonic nucleotide sequence.

Alignment techniques and sequence identity assessment methodology that are known can be used to locate potential paralogous polynucleotide species on either chromosome 18 or chromosome 21 (target chromosomes) and autosomes (reference chromosomes). Such analyses can be performed *in silico.* For example, a target chromosome (e.g., either chromosome 21 or chromosome 18) is sampled, for example, at 10 base pair intervals in 100 base pair segments and each segment aligned to a human reference genome. Segments which map to exactly two regions, one on the target chromosome and one on a reference chromosome, are extracted and merged to obtain paralogous polynucleotide species. Each set of paralogous polynucleotide species is aligned in order to locate nucleotide differences distinguishing target and reference species and to obtain the consensus sequence for primer design.

In some embodiments, the paralogous polynucleotide species sets (assays) comprise one or more of the assays in set forth in Fig. 8 for the analysis of chromosomes 21 and/or 18. Figure 8 provides the paralogous sequences and associated forward and reverse primer pairs for analyzing these particular sequences. Analogous sets of paralogous sequences and associated primer pairs may be derived for other chromosomes.

### Amplification Primers

Because paralogous polynucleotide species of a set have a high degree of sequence similarity, single pairs of amplification primers can be designed to specifically amplify the two paralogous polynucleotide species of a set at a substantially reproducible level. The term "substantially reproducible level" as used herein refers to consistency of amplification levels for the paralogous polynucleotide species of a set. In some cases, a substantially reproducible level varies by 10%, 5%, 4%, 3%, 2%, 1.5%, 1%, 0.5%, 0.1%, 0.05%, 0.01%, 0.005% or 0.001%.

Amplification primers can be designed and synthesized using suitable processes. Amplification primer design software is available and primer design methodology is known. Amplification primer design generally consists of evaluating paralogous polynucleotide species on either side of the one or two base pair mismatch of the species and then choosing the suitable pair of sequences that best meet defined primer criteria. Certain relevant criteria for amplification primer design include: primer GC content, primer size and primer melting temperature. In some cases, the primer design parameters are: primer GC content = 30-70%, primer size = 18-24 base pairs and primer melting temperature = 52-64°C. In certain cases, the primer size can be 20 base pairs and the primer melting temperature can be 60°C. In certain cases, the amplification primers for a set of paralogous polynucleotide species are chosen to produce two equal-sized amplicons (target and reference). In some cases, the size of the amplicons is between 60-100 base pairs.

Designed amplification primers can be filtered to ensure desired sequence properties and to reduce possible sources of variation in the context of multiplexed PCR. In some cases, criteria for filtering amplification primers is based on one or more of the following properties: (1) each primer pair is predicted to produce exactly two equal-sized amplicons between 60-100bp, (2) each primer pair is predicted to produce zero off-target amplification events allowing maximal 3-base mis-priming, (3) each individual primer sequence is complementary to less than 20 positions in a reference human genome, (4) no primer overlaps with any annotated single nucleotide variant with >1% minor allele frequency in dbSNP build137 [39], (5) primers flank at least one position that distinguishes target from reference paralogous polynucleotide species, and (6) all independent amplicons (i.e., sets of paralogous polynucleotide species) are at least 100-bp apart. In certain cases, each individual primer of a pair of amplification primers for a set of paralogous polynucleotide species is complementary to less than 20 positions in a reference human genome. In certain cases, amplification primers satisfy all of the above criterion.

In some cases, to minimize undesired primer-primer interactions in the multiplexed PCR reaction, *in silico* multiplexing optimization is performed. The reverse complement of each primer sequence is locally aligned to all the other primers using known alignment programs such as the Biostrings function 'pairwiseAlignment' [35] in R version 3.0 [36] and a primer-specific alignment score can be calculated. Amplification primers with the largest total alignment score are identified and removed from the primer pool.

In some embodiments, the amplification primers comprise one or more of the amplification primer pairs shown in Fig. 8.

### Multiplex targeted amplification reactions

Because of technical variance a single marker often is not sufficient for classification of a chromosomal abnormality, multiple markers may be used to reduce the variance and improve the accuracy. Thus, the methods herein provide multiplexed assays for the detection of genetic variants. The methods may be used, in some cases, for the analysis of maternal samples comprising fetal nucleic acid. In some cases, the sample is - procured through non-invasive means. For example, in some cases cell free DNA is used.

A typical maternal plasma sample from a pregnant female has between 4-32% (+-2%) cell-free fetal nucleic acid. In order to reliably and accurately detect a fetal chromosomal abnormality, with sufficient specificity and/or sensitivity suitable for a high degree of clinical utility, in a background of maternal nucleic acid, sensitive quantitative methods are needed that can take advantage of the increased power provided by using multiple markers (e.g., multiple sets of paralogous polynucleotide species). By increasing the number of sets of paralogous polynucleotide species, the specificity and sensitivity of the method can be high enough for robust clinical utility as a screening test for chromosome aneuploidy - even in a sample that comprises a mixture of fetal and maternal nucleic acid and a small fetal fraction.

In some cases, sets of paralogous polynucleotide species in the nucleic acid in a sample of circulating cell free nucleic acid of a pregnant female are enriched in multiplex PCR targeted enrichment. In some embodiments, a plurality of sets of paralogous polynucleotide species from the nucleic acid in a sample are amplified in a single reaction (multiplex reaction, multiplex amplification reaction). As used herein "multiplex amplification" refers to simultaneous amplification of many sets of paralogous polynucleotide species in one reaction by using multiple pairs of amplification primers (e.g., a different pair of amplification primers for each set of paralogous polynucleotide species). In some cases, multiplex amplification of a plurality paralogous polynucleotide sequences may be performed in a single reaction vessel or well.

In certain cases, the number of sets of paralogous polynucleotide species (assays) multiplexed include, without limitation, at least 100; at least 150, at least 200, at least 250, at least 300, at least 350, at least 400, at least 450, at least 500, at least 550, at least 600, at least 650, at least 700, at least 750, at least 800, at least 850, at least 900, at least 950 and at least 1000 sets of paralogous polynucleotide species or more per target region. The exact number is dictated by the number of independent measures (assays) required to estimate the predicted response using the multiplexed paralogs, e.g., the ability to obtain statistically significant results using the multiplexed paralogs. For example, a sample where the target abnormality is present in a minority fraction of DNA (discussed in detail herein) may require the use of more assays, i.e., higher number of sets of paralogous polynucleotide species, than a sample where the target abnormality is present in about 50% of the alleles (e.g., a haplotype analysis).

In certain cases, for example for the analysis of fetal DNA from a maternal sample, or cancer cells present at a low amount (<5%) there are at least 250 sets of paralogous polynucleotide species or at least 500 sets of paralogous polynucleotide species (chromosome 21 targets or chromosome 18 targets).

In certain cases, the number of sets of paralogous polynucleotide species (assays) multiplexed include, without limitation, about 50 to about 1,000 sets of paralogous polynucleotide species, and sometimes about 49-51, 51-53, 53-55, 55-57, 57-59, 59-61, 61-63, 63-65, 65-67, 67-69, 69-71, 71-73, 73-75, 75-77, 77-79, 79-81, 81-83, 83-85, 85-87, 87-89, 89-91, 91-93, 93-95, 95-97, 97-101, 101-103, 103-105, 105-107, 107-109, 109-111, 111-113, 113-115, 115-117, 117-119, 121-123, 123-125, 125-127, 127-129, 129-131, 131-133, 133-135, 135-137, 137-139, 139-141, 141-143, 143-145, 145-147, 147-149, 149-151, 151-153, 153-155, 155-157, 157-159, 159-161, 161-163, 163-165, 165-167, 167-169, 169-171, 171-173, 173-175, 175-177, 177-179, 179-181, 181-183, 183-185, 185-187, 187-189, 189-191, 191-193, 193-195, 195-197, 197-199, 199-201, 201-203, 203-205, 205-207, 207-209, 209-211, 211-213, 213-215, 215-217, 217-219, 219-221, 221-223, 223-225, 225-227, 227-229, 229-231, 231-233, 233-235, 235-237, 237-239, 239-241, 241-243, 243-245, 245-247, 247-249, 249-251, 251-253, 253-255, 255-257, 257-259, 259-261, 261-263, 263-265, 265-267, 267-269, 269-271, 271-273, 273-275, 275-277, 277-279, 279-281, 281-283, 283-285, 285-287, 287-289, 289-291, 291-293, 293-295, 295-297, 297-299, 299-301, 301- 303, 303- 305, 305- 307, 307- 309, 309- 311, 311- 313, 313- 315, 315- 317, 317- 319, 319-321, 321-323, 323-325, 325-327, 327-329, 329-331, 331-333, 333- 335, 335-337, 337-339, 339-341, 341-343, 343-345, 345-347, 347-349, 349-351, 351-353, 353-355, 355-357, 357-359, 359-361, 361-363, 363-365, 365-367, 367-369, 369-371, 371-373, 373-375, 375-377, 377-379, 379-381, 381-383, 383-385, 385-387, 387-389, 389-391, 391-393, 393-395, 395-397, 397-401, 401- 403, 403- 405, 405- 407, 407- 409, 409- 411, 411- 413, 413- 415, 415-417, 417- 419, 419-421, 421-423, 423-425, 425-427, 427-429, 429-431, 431-433, 433- 435, 435-437, 437-439, 439-441, 441-443, 443-445, 445-447, 447-449, 449-451, 451-453, 453-455, 455-457, 457-459, 459-461, 461-463, 463-465, 465-467, 467-469, 469-471, 471-473, 473-475, 475-477, 477-479, 479-481, 481-483, 483-485, 485-487, 487-489, 489-491, 491-493, 493-495, 495-497, 497-501 sets of paralogous polynucleotide species or more.

Nucleic acids amplified in a single multiplex amplification reaction can include, but are not limited to the following illustrative examples. In some embodiments, the sets of paralogous polynucleotide species have the target chromosome as chromosome 21 and the reference chromosome as an autosome other than chromosome 21. In some embodiments, the sets of paralogous polynucleotide species have the target chromosome as chromosome 18 and the reference chromosome as an autosome other than chromosome 18. In some embodiments, the sets of paralogous polynucleotide species comprise sets with chromosome 21 as the target chromosome together with sets with chromosome 18 as the target chromosome. In certain embodiments, there are at least 250 sets of paralogous polynucleotide species for target chromosome 21 and there are at least 250 sets of paralogous polynucleotide species for target chromosome 18. In certain embodiments, there are at least 500 sets of paralogous polynucleotide species for target chromosome 21 and there are at least 500 sets of paralogous polynucleotide species for target chromosome 18. In certain embodiments, sets with chromosome 21 as the target chromosome together with sets with chromosome 18 as the target chromosome also include Y-chromosome polynucleotides and polynucleotides comprising single nucleotide polymorphic (SNP) loci. In certain embodiments, there are at least 10 Y-chromosome polynucleotides and at least 150 polynucleotides comprising single nucleotide polymorphic (SNP) loci.

Nucleic acid can be extracted from a biological sample obtained from a subject, as described herein. A subject sometimes is female or male (e.g., human female or human male), and a female can be a pregnant female at any suitable stage of pregnancy (e.g., first, second or third trimester). Extracted nucleic acid from the test subject sometimes is circulating cell free nucleic acid, and circulating cell free nucleic acid sometimes is from a blood plasma, blood serum or urine sample from a test subject. Extracted circulating cell free nucleic acid sometimes is not manipulated prior to performing amplification (e.g., the cell free nucleic acid often is not cleaved by an exonuclease or endonuclease). Any suitable method of amplification of nucleic acid may be utilized.

### Universal amplification reactions

In some embodiments, each of the amplified sets of paralogous polynucleotide species is further amplified by universal PCR to prepare a library of enriched paralogous polynucleotide species for further analysis, such as by sequencing. In certain cases, universal PCR utilizes as primer sites, partial sequencing adaptor motifs that are introduced during the previous multiplex PCR. In some cases, universal PCR incorporates a sample specific DNA sequence that can be used to identify the sample. Sometimes identification of a sample specific DNA sequence is by sequencing.

### Detection and quantitation of amplified paralogous polynucleotide species

Amplified paralogous polynucleotide species can be detected and quantified by any suitable detection process. Detection of amplified paralogous polynucleotide species is performed by any method that can detect single nucleotide differences (differences between the paralogous polynucleotide species on a target chromosome and the paired paralogous polynucleotide species on a reference chromosome). Methods include, but are not limited to, digital PCR, quantitative PCR, allele-specific hybridization-based assays, RFLP analysis, single nucleotide primer extension-based assays and sequencing-based assays. In some cases, detection and determining the amount of (quantitation) of amplified paralogous polynucleotide species is by a sequencing process. A sequencing process utilized often is a whole-genome sequencing process, and in some cases is a targeted sequencing process (e.g., a process that sequences a subset of all nucleic acid in a sample, the amplified paralogous polynucleotide species). A sequencing process utilized sometimes includes sequencing by synthesis. The sequence reads resulting from the sequencing process are mapped to a reference genome. Reads identified as representing paralogous polynucleotide species are quantified and typically expressed as counts.

In some cases, sequence reads are filtered prior to being quantified. In some cases, the filtering requires that sequence reads: (1) are of a minimum read length, (2) have a mapped position of the 5'-end of the read within 5 base pairs of the expected 5'-end of a designated target region, and (3) are not ambiguously aligned. In some cases, the minimum read length is greater than or equal to about 20 base pairs, greater than or equal to about 30 base pairs, greater than or equal to about 40 base pairs, greater than or equal to about 50 base pairs or greater than or equal to about 60 base pairs. In certain cases, the minimum read length is greater than or equal to about 45 base pairs.

### Paralog ratio

In some cases, a paralog ratio is determined for the amount of each amplified paralogous polynucleotide species in a set in the sample. In some cases, a paralog ratio is calculated as the ratio of the counts of the paralogous polynucleotide species on a target chromosome to the counts of the paralogous polynucleotide species on a reference chromosome (i.e., target paralog read depth to reference paralog read depth).

### Fetal fraction and sex determination

In some embodiments, the amount of fetal nucleic acid in a sample (e.g., fetal fraction) is quantified and used in conjunction with paralog ratios in the determination of the presence or absence of a chromosome aneuploidy. In certain cases, determination of the presence or absence of aneuploidy is made only for samples having a certain threshold amount of fetal nucleic acid. A suitable process for quantifying fetal fraction can be utilized, non-limiting examples of which include a mass spectrometric process, sequencing process or combination thereof. In certain cases, fetal fraction can be determined based on allelic ratios of polymorphic sequences (e.g., single nucleotide polymorphisms (SNPs)). In such a method for determining fetal fraction, for example, nucleotide sequence reads are obtained for a maternal sample and fetal fraction is determined by comparing the total number of nucleotide sequence reads that map to a first allele (inferred to be fetal) and the total number of nucleotide sequence reads that map to a second allele (inferred to be maternal) at an informative polymorphic site (e.g., SNP) in a reference genome (e.g., maternal homozygous SNP loci are distinguished from fetal heterozygous SNP loci). In some embodiments, multiple SNP loci are amplified and analyzed. In certain cases 50 to 200 SNP loci are amplified and analyzed. In some cases, 150 SNP loci are amplified and analyzed.

In some cases, fetal sex can be determined by amplifying selected Y chromosome regions and determining the proportion of sequence reads that align to chromosome Y. In some cases, multiple Y chromosome regions are amplified and analyzed. In certain cases, 5 to 50 Y chromosome regions are amplified and analyzed. In some cases, 10 Y chromosome regions are amplified and analyzed.

In certain cases, PCR primers designed to selectively amplify informative single nucleotide polymorphisms and PCR primers designed to selectively amplifying Y chromosome regions are included in the multiplex paralogous polynucleotide species amplification reaction.

### Data processing

In some cases, data (e.g., counts, counts expressed as a ratio) is processed further (e.g., mathematically and/or statistically manipulated) and/or displayed to facilitate providing an outcome. In some cases, paralog ratios are transformed by a suitable method operation or mathematical operation known in the art. In certain cases, paralog ratios are logarithm-transformed to zero-center values.

In some cases, paralog ratios or logarithm-transformed paralog ratios are further processed. A processing step can comprise one or more mathematical and/or statistical manipulations. Any suitable mathematical and/or statistical manipulation (statistic), alone or in combination, may be used to analyze and/or manipulate paralog ratios or logarithm-transformed paralog ratios described herein. Any suitable number of mathematical and/or statistical manipulations can be used.

In certain embodiments, the statistic is a z-score. A z-score is a quotient of (a) subtraction product of (i) the logarithm-transformed paralog ratio, less (ii) a median of the logarithm-transformed paralog ratio of reference euploid samples, divided by (b) a MAD value derived from reference euploid samples. For each set of paralogous polynucleotide species (an assay), a median absolute deviation (MAD) is calculated. The median absolute deviation is the median absolute deviation of logarithm-transformed assay ratios in euploid samples.

In certain embodiments, the statistic is a weighted z-score. A weighted z-score is calculated by weighting individual non-outlier z-scores paralog ratios according to assay MAD or discriminatory power.

In some embodiments, a sample statistic is determined according to the statistic for each of the sets of paralogous polynucleotide species. A sample statistic can be any suitable mathematical and/or statistical manipulation. In certain embodiments, a sample statistic comprises summing statistics for each of sets of paralogous polynucleotide species. In some embodiments, a sample statistic comprises summing z-scores or weighted z-scores of logarithm-transformed paralog ratios.

A sample z-score can be calculated by z-scores of a plurality paralogous polynucleotide species sets, with the exclusion of outliers of z greater than 4 MAD or less than -4 MAD. A sample z-score can be determined according to $Z = \frac{{\sum }_{i = 1}^{N} {z}_{i}}{\sqrt{N}} ,$ where *zᵢ* is the z-score of a non-outlier paralog ratio and N is the number of non-outlier paralogs.

A weighted sample z-score can be calculated by weighted z-scores of a plurality paralogous polynucleotide species sets according to ${Z}_{w} = \frac{{\sum }_{i = 1}^{N} {w}_{i} {z}_{i}}{\sqrt{{\sum }_{i = 1}^{N} {w}_{i}^{2}}} ,$ where *wᵢ* is the weight for each paralog ratio.

### Aneuploidy classification

Methods described herein can provide a determination of the presence or absence of a chromosome aneuploidy based on paralog ratios, thereby providing an outcome. Methods herein sometimes provide a classification of a sample as fetal euploid, fetal trisomy 21 or fetal trisomy 18. Any classification method can be utilized, including but not limited to z-score, PCA, K-nearest neighbors, support vector machine and neural network.

In some cases, classification of the presence or absence of a chromosome aneuploidy is based on z-score. Thresholds for z-score based trisomy 21 and trisomy 18 classification can be determined based on training set samples (where the training set includes samples known to have trisomy 21 or trisomy 18 and samples known to not have trisomy 21 or trisomy 18).

In some cases, optimum performing paralogous polynucleotide species sets (assays or assay sets) are determined independently for fetal trisomy 21 and trisomy 18 classification. In certain cases, assays for aneuploidy classification can be assigned a relative weight based on a number of factors including, but not limited to: sufficient depth of sequencing coverage, small variance expressed as assay MAD, and measurable signal differentiating aneuploid and euploid populations as measured by the p-value from the Wilcoxon rank sum test on log-transformed assay ratios, for example.

Classification performance can be assessed in any suitable manner. In some cases, classification performance is assessed by determining a sensitivity and/or specificity for classification of multiple samples with known chromosome aneuploidy. In certain cases, a classification processes described herein is characterized by a sensitivity of about 90% or greater (e.g., sensitivity of 85, 86, 87, 88, 89, 90, 91, 92, 93, 94, 95, 96, 97, 98, 99, 99.5, 99.9% or greater) and/or independently by a specificity of about 90% or greater (e.g., specificity of 85, 86, 87, 88, 89, 90, 91, 92, 93, 94, 95, 96, 97, 98, 99, 99.5, 99.9% or greater). In certain cases, sensitivity is greater than 96% and specificity is greater than 99% for fetal aneuploidy detection.

In some cases, variations of z-score based classification methods can be utilized, including but not limited to weighting z-scores by assay variance or discriminatory power, merging assays based on physical location and assay correlations prior to z-score calculation.

Reporting of the classified results can ultimately provide useful information. Reporting of the results sometimes is based on an empirically or statistically derived threshold. In some cases, this threshold is used to produce to a result that is determined to be positive or negative for fetal aneuploidy. In some cases, a result is reported as a likelihood of fetal aneuploidy. In certain cases, this likelihood can be reported as a risk score. In some cases, the risk score is reported based solely on the results from the methods described herein. In some cases, the result is reported based upon the results from the method described herein and additional information about a patient. In certain cases, for noninvasive prenatal testing, this information can be, but is not limited to, maternal age and fetal fraction.

### Samples

Provided herein are systems, methods and products for analyzing nucleic acids. In some cases, nucleic acid fragments in a mixture of nucleic acid fragments are analyzed. A mixture of nucleic acids can comprise two or more nucleic acid fragment species having different nucleotide sequences, different fragment lengths, different origins (e.g., genomic origins, fetal vs. maternal origins, cell or tissue origins, sample origins, subject origins, and the like), or combinations thereof.

Nucleic acid or a nucleic acid mixture utilized in systems, methods and products described herein often is isolated from a sample obtained from a subject. A subject can be any living or non-living organism, including but not limited to a human, a non-human animal, a plant, a bacterium, a fungus, a protest or a pathogen. Any human or non-human animal can be selected, and may include, for example, mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female (e.g., woman, a pregnant woman). A subject may be any age (e.g., an embryo, a fetus, an infant, a child, an adult).

Nucleic acid may be isolated from any type of suitable biological specimen or sample (e.g., a test sample). A sample or test sample can be any specimen that is isolated or obtained from a subject or part thereof (e.g., a human subject, a pregnant female, a fetus). A sample sometimes is from a pregnant female subject bearing a fetus at any stage of gestation (e.g., first, second or third trimester for a human subject), and sometimes is from a post-natal subject. A sample sometimes is from a pregnant subject bearing a fetus that is euploid for all chromosomes, and sometimes is from a pregnant subject bearing a fetus having a chromosome aneuploidy (e.g., one, three (i.e., trisomy (e.g., T21, T18, T13)), or four copies of a chromosome) or other genetic variation. Non-limiting examples of specimens include fluid or tissue from a subject, including, without limitation, blood or a blood product (e.g., serum, plasma, or the like), umbilical cord blood, chorionic villi, amniotic fluid, cerebrospinal fluid, spinal fluid, lavage fluid (e.g., bronchoalveolar, gastric, peritoneal, ductal, ear, arthroscopic), biopsy sample (e.g., from pre-implantation embryo; cancer biopsy), celocentesis sample, cells (blood cells, placental cells, embryo or fetal cells, fetal nucleated cells or fetal cellular remnants, normal cells, abnormal cells (e.g., cancer cells)) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, the like or combinations thereof. In some cases, a biological sample is a cervical swab from a subject. A fluid or tissue sample from which nucleic acid is extracted may be acellular (e.g., cell-free). In some cases, a fluid or tissue sample may contain cellular elements or cellular remnants. In some cases, fetal cells may be included in the sample.

A sample can be a liquid sample. A liquid sample can comprise extracellular nucleic acid (e.g., circulating cell-free DNA). Non-limiting examples of liquid samples, include, blood or a blood product (e.g., serum, plasma, or the like), urine, a liquid sample described above, the like or combinations thereof. In certain cases, a sample is a liquid biopsy, which generally refers to an assessment of a liquid sample from a subject for the presence, absence, progression or remission of a disease. In certain instances, extracellular nucleic acid is analyzed in a liquid biopsy.

In some cases, a biological sample may be blood, plasma or serum. The term "blood" encompasses whole blood, blood product or any fraction of blood, such as serum, plasma, buffy coat, or the like as conventionally defined. Blood or fractions thereof often comprise nucleosomes. Nucleosomes comprise nucleic acids and are sometimes cell-free or intracellular. Blood also comprises buffy coats. Buffy coats are sometimes isolated by utilizing a ficoll gradient. Buffy coats can comprise white blood cells (e.g., leukocytes, T-cells, B-cells, platelets, and the like). Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow. For blood, an appropriate amount of peripheral blood (e.g., between 3 to 40 milliliters, between 5 to 50 milliliters) often is collected and can be stored according to standard procedures prior to or after preparation.

An analysis of nucleic acid found in a subjects blood may be performed using, e.g., whole blood, serum, or plasma. An analysis of fetal DNA found in maternal blood, for example, may be performed using, e.g., whole blood, serum, or plasma. Methods for preparing serum or plasma from blood obtained from a subject (e.g., a maternal subject) are known. For example, a subject's blood (e.g., a pregnant woman's blood) can be placed in a tube containing EDTA or a specialized commercial product such as Vacutainer SST (Becton Dickinson, Franklin Lakes, N.J.) to prevent blood clotting, and plasma can then be obtained from whole blood through centrifugation. Serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for nucleic acid extraction. In addition to the acellular portion of the whole blood, nucleic acid may also be recovered from the cellular fraction, enriched in the buffy coat portion, which can be obtained following centrifugation of a whole blood sample from the subject and removal of the plasma.

A sample may be heterogeneous. For example, a sample may include more than one cell type and/or one or more nucleic acid species. In some instances, a sample may include fetal cells and maternal cells. In some instances, a sample may include (i) fetal derived and maternal derived nucleic acid, and/or more generally (ii) mutated and wild-type nucleic acid. In some instances, a sample may include a minority nucleic acid species and a majority nucleic acid species, as described in further detail below. In some instances, a sample may include cells and/or nucleic acid from a single subject or may include cells and/or nucleic acid from multiple subj ects.

For prenatal applications of technology described herein, fluid or tissue sample may be collected from a female at a gestational age suitable for testing, or from a female who is being tested for possible pregnancy. Suitable gestational age may vary depending on the prenatal test being performed. In certain cases, a pregnant female subject sometimes is in the first trimester of pregnancy, at times in the second trimester of pregnancy, or sometimes in the third trimester of pregnancy. In certain cases, a fluid or tissue is collected from a pregnant female between about 1 to about 45 weeks of fetal gestation (e.g., at 1-4, 4-8, 8-12, 12-16, 16-20, 20-24, 24-28, 28-32, 32-36, 36-40 or 40-44 weeks of fetal gestation), and sometimes between about 5 to about 28 weeks of fetal gestation (e.g., at 6, 7, 8, 9,10, 11, 12, 13, 14, 15, 16, 17, 18, 19, 20, 21, 22, 23, 24, 25, 26 or 27 weeks of fetal gestation). In certain cases a fluid or tissue sample is collected from a pregnant female during or just after (e.g., 0 to 72 hours after) giving birth (e.g., vaginal or non-vaginal birth (e.g., surgical delivery)).

### Cell types

As used herein, a "cell type" refers to a type of cell that can be distinguished from another type of cell. Extracellular nucleic acid can include nucleic acid from several different cell types. Non-limiting examples of cell types that can contribute nucleic acid to circulating cell-free nucleic acid include liver cells (e.g., hepatocytes), lung cells, spleen cells, pancreas cells, colon cells, skin cells, bladder cells, eye cells, brain cells, esophagus cells, cells of the head, cells of the neck, cells of the ovary, cells of the testes, prostate cells, placenta cells, epithelial cells, endothelial cells, adipocyte cells, kidney/renal cells, heart cells, muscle cells, blood cells (e.g., white blood cells), central nervous system (CNS) cells, the like and combinations of the foregoing. In some cases, cell types that contribute nucleic acid to circulating cell-free nucleic acid analyzed include white blood cells, endothelial cells and hepatocyte liver cells. Different cell types can be screened as part of identifying and selecting nucleic acid loci for which a marker state is the same or substantially the same for a cell type in subjects having a medical condition and for the cell type in subjects not having the medical condition, as described in further detail herein.

A particular cell type sometimes remains the same or substantially the same in subjects having a medical condition and in subjects not having a medical condition. In a non-limiting example, the number of living or viable cells of a particular cell type may be reduced in a cell degenerative condition, and the living, viable cells are not modified, or are not modified significantly, in subjects having the medical condition.

A particular cell type sometimes is modified as part of a medical condition and has one or more different properties than in its original state. In a non-limiting example, a particular cell type may proliferate at a higher than normal rate, may transform into a cell having a different morphology, may transform into a cell that expresses one or more different cell surface markers and/or may become part of a tumor, as part of a cancer condition. In cases for which a particular cell type (i.e., a progenitor cell) is modified as part of a medical condition, the marker state for each of the one or more markers assayed often is the same or substantially the same for the particular cell type in subjects having the medical condition and for the particular cell type in subjects not having the medical condition. Thus, the term "cell type" sometimes pertains to a type of cell in subjects not having a medical condition, and to a modified version of the cell in subjects having the medical condition. In some cases, a "cell type" is a progenitor cell only and not a modified version arising from the progenitor cell. A "cell type" sometimes pertains to a progenitor cell and a modified cell arising from the progenitor cell. In such cases, a marker state for a marker analyzed often is the same or substantially the same for a cell type in subjects having a medical condition and for the cell type in subjects not having the medical condition.

Different cell types can be distinguished by any suitable characteristic, including without limitation, one or more different cell surface markers, one or more different morphological features, one or more different functions, one or more different protein (e.g., histone) modifications and one or more different nucleic acid markers. Non-limiting examples of nucleic acid markers include single-nucleotide polymorphisms (SNPs), methylation state of a nucleic acid locus, short tandem repeats, insertions (e.g., microinsertions), deletions (microdeletions) the like and combinations thereof. Non-limiting examples of protein (e.g., histone) modifications include acetylation, methylation, ubiquitylation, phosphorylation, sumoylation, the like and combinations thereof.

As used herein, the term a "related cell type" refers to a cell type having multiple characteristics in common with another cell type. In related cell types, 75% or more cell surface markers sometimes are common to the cell types (e.g., about 80%, 85%, 90% or 95% or more of cell surface markers are common to the related cell types).

### Nucleic acid

Provided herein are methods for analyzing nucleic acid. The terms "nucleic acid" and "nucleic acid molecule" may be used interchangeably throughout the disclosure. The terms refer to nucleic acids of any composition from, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, RNA highly expressed by a fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single- or double-stranded form, and unless otherwise limited, can encompass known analogs of natural nucleotides that can function in a similar manner as naturally occurring nucleotides. A nucleic acid may be, or may be from, a plasmid, phage, virus, bacterium, autonomously replicating sequence (ARS), mitochondria, centromere, artificial chromosome, chromosome, or other nucleic acid able to replicate or be replicated in vitro or in a host cell, a cell, a cell nucleus or cytoplasm of a cell in certain cases. A template nucleic acid in some cases can be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense," "plus" strand or "minus" strand, "forward" reading frame or "reverse" reading frame) and double-stranded polynucleotides. The term "gene" refers to a section of DNA involved in producing a polypeptide chain; and generally includes regions preceding and following the coding region (leader and trailer) involved in the transcription/translation of the gene product and the regulation of the transcription/translation, as well as intervening sequences (introns) between individual coding regions (exons). A nucleotide or base generally refers to the purine and pyrimidine molecular units of nucleic acid (e.g., adenine (A), thymine (T), guanine (G), and cytosine (C)). For RNA, the base thymine is replaced with uracil. Nucleic acid length or size may be expressed as a number of bases.

Nucleic acid may be single or double stranded. Single stranded DNA, for example, can be generated by denaturing double stranded DNA by heating or by treatment with alkali, for example. In certain cases, nucleic acid is in a D-loop structure, formed by strand invasion of a duplex DNA molecule by an oligonucleotide or a DNA-like molecule such as peptide nucleic acid (PNA). D loop formation can be facilitated by addition of E. Coli RecA protein and/or by alteration of salt concentration, for example, using methods known in the art.

Nucleic acid provided for processes described herein may contain nucleic acid from one sample or from two or more samples (e.g., from 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more samples).

Nucleic acid may be derived from one or more sources (e.g., biological sample, blood, cells, serum, plasma, buffy coat, urine, lymphatic fluid, skin, soil, and the like) by methods known in the art. Any suitable method can be used for isolating, extracting and/or purifying DNA from a biological sample (e.g., from blood or a blood product), non-limiting examples of which include methods of DNA preparation (e.g., described by Sambrook and Russell, Molecular Cloning: A Laboratory Manual 3d ed., 2001), various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep^{™} Blood DNA Isolation Kit (Promega, Madison, Wis.), and GFX^{™} Genomic Blood DNA Purification Kit (Amersham, Piscataway, N.J.), the like or combinations thereof.

In some cases, nucleic acid is extracted from cells using a cell lysis procedure. Cell lysis procedures and reagents are known in the art and may generally be performed by chemical (e.g., detergent, hypotonic solutions, enzymatic procedures, and the like, or combination thereof), physical (e.g., French press, sonication, and the like), or electrolytic lysis methods. Any suitable lysis procedure can be utilized. For example, chemical methods generally employ lysing agents to disrupt cells and extract the nucleic acids from the cells, followed by treatment with chaotropic salts. Physical methods such as freeze/thaw followed by grinding, the use of cell presses and the like also are useful. In some instances, a high salt and/or an alkaline lysis procedure may be utilized.

Nucleic acids can include extracellular nucleic acid in certain cases. The term "extracellular nucleic acid" as used herein can refer to nucleic acid isolated from a source having substantially no cells and also is referred to as "cell-free" nucleic acid, "circulating cell-free nucleic acid" (e.g., CCF fragments, ccf DNA) and/or "cell-free circulating nucleic acid." Extracellular nucleic acid can be present in and obtained from blood (e.g., from the blood of a human subject). Extracellular nucleic acid often includes no detectable cells and may contain cellular elements or cellular remnants. Non-limiting examples of acellular sources for extracellular nucleic acid are blood, blood plasma, blood serum and urine. As used herein, the term "obtain cell-free circulating sample nucleic acid" includes obtaining a sample directly (e.g., collecting a sample, e.g., a test sample) or obtaining a sample from another who has collected a sample. Without being limited by theory, extracellular nucleic acid may be a product of cell apoptosis and cell breakdown, which provides basis for extracellular nucleic acid often having a series of lengths across a spectrum (e.g., a "ladder").

Extracellular nucleic acid can include different nucleic acid species, and therefore is referred to herein as "heterogeneous" in certain cases. For example, blood serum or plasma from a pregnant female can include maternal nucleic acid and fetal nucleic acid. In some instances, fetal nucleic acid sometimes is about 5% to about 50% of the overall nucleic acid (e.g., about 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49% of the total nucleic acid is cancer or fetal nucleic acid).

At least two different nucleic acid species can exist in different amounts in extracellular nucleic acid and sometimes are referred to as minority species and majority species. For example, in some cases (e.g., fetal DNA or cancer DNA) the DNA of interest is a minority species. In certain cases, a genetic variation (e.g., copy number alteration, single nucleotide variation, chromosome alteration, translocation) is determined for a minority nucleic acid species. In certain cases, a genetic variation is determined for a majority nucleic acid species. Generally it is not intended that the terms "minority" or "majority" be rigidly defined in any respect. In one aspect, a nucleic acid that is considered "minority," for example, can have an abundance of at least about 0.1% of the total nucleic acid in a sample to less than 50% of the total nucleic acid in a sample. In some cases, a minority nucleic acid can have an abundance of at least about 1% of the total nucleic acid in a sample to about 40% of the total nucleic acid in a sample. In some cases, a minority nucleic acid can have an abundance of at least about 2% of the total nucleic acid in a sample to about 30% of the total nucleic acid in a sample. In some cases, a minority nucleic acid can have an abundance of at least about 3% of the total nucleic acid in a sample to about 25% of the total nucleic acid in a sample. For example, a minority nucleic acid can have an abundance of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% of the total nucleic acid in a sample. In some instances, a minority species of extracellular nucleic acid sometimes is about 1% to about 40% of the overall nucleic acid (e.g., about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40% of the nucleic acid is minority species nucleic acid). In some cases, the minority nucleic acid is extracellular DNA. In some cases, the minority nucleic acid is extracellular DNA from apoptotic tissue. In some cases, the minority nucleic acid is extracellular fetal DNA.

In another aspect, a nucleic acid that is considered "majority," for example, can have an abundance greater than 50% of the total nucleic acid in a sample to about 99.9% of the total nucleic acid in a sample. In some cases, a majority nucleic acid can have an abundance of at least about 60% of the total nucleic acid in a sample to about 99% of the total nucleic acid in a sample. In some cases, a majority nucleic acid can have an abundance of at least about 70% of the total nucleic acid in a sample to about 98% of the total nucleic acid in a sample. In some cases, a majority nucleic acid can have an abundance of at least about 75% of the total nucleic acid in a sample to about 97% of the total nucleic acid in a sample. For example, a majority nucleic acid can have an abundance of at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% of the total nucleic acid in a sample. In some cases, the majority nucleic acid is extracellular DNA. In some cases, the majority nucleic acid is extracellular maternal DNA.

In some cases, a minority species of extracellular nucleic acid is of a length of about 500 base pairs or less (e.g., about 80, 85, 90, 91, 92, 93, 94, 95, 96, 97, 98, 99 or 100% of minority species nucleic acid is of a length of about 500 base pairs or less). In some cases, a minority species of extracellular nucleic acid is of a length of about 300 base pairs or less (e.g., about 80, 85, 90, 91, 92, 93, 94, 95, 96, 97, 98, 99 or 100% of minority species nucleic acid is of a length of about 300 base pairs or less). In some cases, a minority species of extracellular nucleic acid is of a length of about 250 base pairs or less (e.g., about 80, 85, 90, 91, 92, 93, 94, 95, 96, 97, 98, 99 or 100% of minority species nucleic acid is of a length of about 250 base pairs or less). In some cases, a minority species of extracellular nucleic acid is of a length of about 200 base pairs or less (e.g., about 80, 85, 90, 91, 92, 93, 94, 95, 96, 97, 98, 99 or 100% of minority species nucleic acid is of a length of about 200 base pairs or less). In some cases, a minority species of extracellular nucleic acid is of a length of about 150 base pairs or less (e.g., about 80, 85, 90, 91, 92, 93, 94, 95, 96, 97, 98, 99 or 100% of minority species nucleic acid is of a length of about 150 base pairs or less). In some cases, a minority species of extracellular nucleic acid is of a length of about 100 base pairs or less (e.g., about 80, 85, 90, 91, 92, 93, 94, 95, 96, 97, 98, 99 or 100% of minority species nucleic acid is of a length of about 100 base pairs or less). In some cases, a minority species of extracellular nucleic acid is of a length of about 50 base pairs or less (e.g., about 80, 85, 90, 91, 92, 93, 94, 95, 96, 97, 98, 99 or 100% of minority species nucleic acid is of a length of about 50 base pairs or less).

Nucleic acid may be provided for conducting methods described herein with or without processing of the sample(s) containing the nucleic acid. In some cases, nucleic acid is provided for conducting methods described herein after processing of the sample(s) containing the nucleic acid. For example, a nucleic acid can be extracted, isolated, purified, partially purified or amplified from the sample(s). The term "isolated" as used herein refers to nucleic acid removed from its original environment (e.g., the natural environment if it is naturally occurring, or a host cell if expressed exogenously), and thus is altered by human intervention (e.g., "by the hand of man") from its original environment. The term "isolated nucleic acid" as used herein can refer to a nucleic acid removed from a subject (e.g., a human subject). An isolated nucleic acid can be provided with fewer non-nucleic acid components (e.g., protein, lipid) than the amount of components present in a source sample. A composition comprising isolated nucleic acid can be about 50% to greater than 99% free of non-nucleic acid components. A composition comprising isolated nucleic acid can be about 90%, 91%, 92%, 93%, 94%, 95%, 96%, 97%, 98%, 99% or greater than 99% free of non-nucleic acid components. The term "purified" as used herein can refer to a nucleic acid provided that contains fewer non-nucleic acid components (e.g., protein, lipid, carbohydrate) than the amount of non-nucleic acid components present prior to subjecting the nucleic acid to a purification procedure. A composition comprising purified nucleic acid may be about 80%, 81%, 82%, 83%, 84%, 85%, 86%, 87%, 88%, 89%, 90%, 91%, 92%, 93%, 94%, 95%, 96%, 97%, 98%, 99% or greater than 99% free of other non-nucleic acid components. The term "purified" as used herein can refer to a nucleic acid provided that contains fewer nucleic acid species than in the sample source from which the nucleic acid is derived. A composition comprising purified nucleic acid may be about 90%, 91%, 92%, 93%, 94%, 95%, 96%, 97%, 98%, 99% or greater than 99% free of other nucleic acid species. For example, fetal nucleic acid can be purified from a mixture comprising maternal and fetal nucleic acid. In certain examples, small fragments of fetal nucleic acid (e.g., 30 to 500 bp fragments) can be purified, or partially purified, from a mixture comprising both fetal and maternal nucleic acid fragments. In certain examples, nucleosomes comprising smaller fragments of fetal nucleic acid can be purified from a mixture of larger nucleosome complexes comprising larger fragments of maternal nucleic acid. In some cases, nucleic acid is provided for conducting methods described herein without prior processing of the sample(s) containing the nucleic acid. For example, nucleic acid may be analyzed directly from a sample without prior extraction, purification, partial purification, and/or amplification.

In some cases nucleic acids are sheared or cleaved prior to, during or after a method described herein. The term "shearing" or "cleavage" generally refers to a procedure or conditions in which a nucleic acid molecule, such as a nucleic acid template gene molecule or amplified product thereof, may be severed into two (or more) smaller nucleic acid molecules. Such shearing or cleavage can be sequence specific, base specific, or nonspecific, and can be accomplished by any of a variety of methods, reagents or conditions, including, for example, chemical, enzymatic, physical shearing (e.g., physical fragmentation). Sheared or cleaved nucleic acids may have a nominal, average or mean length of about 5 to about 10,000 base pairs, about 100 to about 1,000 base pairs, about 100 to about 500 base pairs, or about 10, 15, 20, 25, 30, 35, 40, 45, 50, 55, 60, 65, 70, 75, 80, 85, 90, 95, 100, 200, 300, 400, 500, 600, 700, 800, 900, 1000, 2000, 3000, 4000, 5000, 6000, 7000, 8000 or 9000 base pairs.

Sheared or cleaved nucleic acids can be generated by a suitable method, non-limiting examples of which include physical methods (e.g., shearing, e.g., sonication, French press, heat, UV irradiation, the like), enzymatic processes (e.g., enzymatic cleavage agents (e.g., a suitable nuclease, a suitable restriction enzyme, a suitable methylation sensitive restriction enzyme)), chemical methods (e.g., alkylation, DMS, piperidine, acid hydrolysis, base hydrolysis, heat, the like, or combinations thereof), processes described in U.S. Patent Application Publication No. 2005/0112590, the like or combinations thereof. The average, mean or nominal length of the resulting nucleic acid fragments can be controlled by selecting an appropriate fragment-generating method.

The term "amplified" as used herein refers to subjecting a target nucleic acid in a sample to a process that linearly or exponentially generates amplicon nucleic acids having the same or substantially the same nucleotide sequence as the target nucleic acid, or part thereof. In certain cases the term "amplified" refers to a method that comprises a polymerase chain reaction (PCR). In certain instances, an amplified product can contain one or more nucleotides more than the amplified nucleotide region of a nucleic acid template sequence (e.g., a primer can contain "extra" nucleotides such as a transcriptional initiation sequence, in addition to nucleotides complementary to a nucleic acid template gene molecule, resulting in an amplified product containing "extra" nucleotides or nucleotides not corresponding to the amplified nucleotide region of the nucleic acid template gene molecule).

Nucleic acid also may be exposed to a process that modifies certain nucleotides in the nucleic acid before providing nucleic acid for a method described herein. A process that selectively modifies nucleic acid based upon the methylation state of nucleotides therein can be applied to nucleic acid, for example. In addition, conditions such as high temperature, ultraviolet radiation, x-radiation, can induce changes in the sequence of a nucleic acid molecule. Nucleic acid may be provided in any suitable form useful for conducting a sequence analysis.

### Enriching nucleic acids

In some cases, nucleic acid (e.g., extracellular nucleic acid) is enriched or relatively enriched for a subpopulation or species of nucleic acid. Nucleic acid subpopulations can include, for example, fetal nucleic acid, maternal nucleic acid, nucleic acid comprising fragments of a particular length or range of lengths, or nucleic acid from a particular genome region (e.g., single chromosome, set of chromosomes, and/or certain chromosome regions). Such enriched samples can be used in conjunction with a method provided herein. Thus, in certain cases, methods of the technology comprise an additional step of enriching for a subpopulation of nucleic acid in a sample, such as, for example, fetal nucleic acid. In certain cases, a method for determining fetal fraction also can be used to enrich for fetal nucleic acid. In certain cases, maternal nucleic acid is selectively removed (partially, substantially, almost completely or completely) from the sample. In certain cases, enriching for a particular low copy number species nucleic acid (e.g., fetal nucleic acid) may improve quantitative sensitivity. Methods for enriching a sample for a particular species of nucleic acid are described, for example, in U.S. Patent No. 6,927,028, International Patent Application Publication No. WO2007/140417, International Patent Application Publication No. WO2007/147063, International Patent Application Publication No. WO2009/032779, International Patent Application Publication No. WO2009/032781, International Patent Application Publication No. WO2010/033639, International Patent Application Publication No. WO2011/034631, International Patent Application Publication No. WO2006/056480, and International Patent Application Publication No. WO2011/1436559.

In some cases, nucleic acid is enriched for certain target fragment species and/or reference fragment species. In certain cases, nucleic acid is enriched for a specific nucleic acid fragment length or range of fragment lengths using one or more length-based separation methods described below. In certain cases, nucleic acid is enriched for fragments from a select genomic region (e.g., chromosome) using one or more sequence-based separation methods described herein and/or known in the art. Non-limiting examples of methods for enriching for a nucleic acid subpopulation in a sample include methods that exploit epigenetic differences between nucleic acid species (e.g., methylation-based fetal nucleic acid enrichment methods described in U.S. Patent Application Publication No. 2010/0105049); restriction endonuclease enhanced polymorphic sequence approaches (e.g., such as a method described in U.S. Patent Application Publication No. 2009/0317818); selective enzymatic degradation approaches; massively parallel signature sequencing (MPSS) approaches; amplification (e.g., PCR)-based approaches (e.g., loci-specific amplification methods, multiplex SNP allele PCR approaches; universal amplification methods); pull-down approaches (e.g., biotinylated ultramer pull-down methods); extension and ligation-based methods (e.g., molecular inversion probe (MIP) extension and ligation); and combinations thereof.

In some cases, nucleic acid is enriched for fragments from a select genomic region (e.g., chromosome) using one or more sequence-based separation methods described herein. Sequence-based separation generally is based on nucleotide sequences present in the fragments of interest (e.g., target and/or reference fragments) and substantially not present in other fragments of the sample or present in an insubstantial amount of the other fragments (e.g., 5% or less). In some cases, sequence-based separation can generate separated target fragments and/or separated reference fragments. Separated target fragments and/or separated reference fragments often are isolated away from the remaining fragments in the nucleic acid sample. In certain cases, the separated target fragments and the separated reference fragments also are isolated away from each other (e.g., isolated in separate assay compartments). In certain cases, the separated target fragments and the separated reference fragments are isolated together (e.g., isolated in the same assay compartment). In some cases, unbound fragments can be differentially removed or degraded or digested.

In some cases, a selective nucleic acid capture process is used to separate target and/or reference fragments away from a nucleic acid sample. Commercially available nucleic acid capture systems include, for example, Nimblegen sequence capture system (Roche NimbleGen, Madison, WI); Illumina BEADARRAY platform (Illumina, San Diego, CA); Affymetrix GENECHIP platform (Affymetrix, Santa Clara, CA); Agilent SureSelect Target Enrichment System (Agilent Technologies, Santa Clara, CA); and related platforms. Such methods typically involve hybridization of a capture oligonucleotide to a part or all of the nucleotide sequence of a target or reference fragment and can include use of a solid phase (e.g., solid phase array) and/or a solution based platform. Capture oligonucleotides (sometimes referred to as "bait") can be selected or designed such that they preferentially hybridize to nucleic acid fragments from selected genomic regions or loci (e.g., one of chromosomes 21, 18, 13, X or Y, or a reference chromosome). In certain cases, a hybridization-based method (e.g., using oligonucleotide arrays) can be used to enrich for nucleic acid sequences from certain chromosomes (e.g., a potentially aneuploid chromosome, reference chromosome or other chromosome of interest) or regions of interest thereof.

In some cases, nucleic acid is enriched for a particular nucleic acid fragment length, range of lengths, or lengths under or over a particular threshold or cutoff using one or more length-based separation methods. Nucleic acid fragment length typically refers to the number of nucleotides in the fragment. Nucleic acid fragment length also is sometimes referred to as nucleic acid fragment size. In some cases, a length-based separation method is performed without measuring lengths of individual fragments. In some cases, a length based separation method is performed in conjunction with a method for determining length of individual fragments. In some cases, length-based separation refers to a size fractionation procedure where all or part of the fractionated pool can be isolated (e.g., retained) and/or analyzed. Size fractionation procedures are known in the art (e.g., separation on an array, separation by a molecular sieve, separation by gel electrophoresis, separation by column chromatography (e.g., size-exclusion columns), and microfluidics-based approaches). In certain instances, length-based separation approaches can include selective sequence tagging approaches, fragment circularization, chemical treatment (e.g., formaldehyde, polyethylene glycol (PEG) precipitation), mass spectrometry and/or size-specific nucleic acid amplification, for example.

### Nucleic acid quantification

The amount of nucleic acid (e.g., concentration, relative amount, absolute amount, copy number, and the like) in a sample may be determined. The amount of a minority nucleic acid (e.g., concentration, relative amount, absolute amount, copy number, and the like) in nucleic acid is determined in some cases. In certain cases, the amount of a minority nucleic acid species in a sample is referred to as "minority species fraction." In some cases "minority species fraction" refers to the fraction of a minority nucleic acid species in circulating cell-free nucleic acid in a sample (e.g., a blood sample, a serum sample, a plasma sample, a urine sample) obtained from a subj ect.

The amount of a minority nucleic acid in extracellular nucleic acid can be quantified and used in conjunction with a method provided herein. Thus, in certain cases, methods described herein comprise an additional step of determining the amount of a minority nucleic acid. The amount of a minority nucleic acid can be determined in a sample from a subject before or after processing to prepare sample nucleic acid. In certain cases, the amount of a minority nucleic acid is determined in a sample after sample nucleic acid is processed and prepared, which amount is utilized for further assessment. In some cases, an outcome comprises factoring the minority species fraction in the sample nucleic acid (e.g., adjusting counts, removing samples, making a call or not making a call).

A determination of minority species fraction can be performed before, during, or at any one point in a method described herein, or after certain methods described herein (e.g., detection of a genetic variation). For example, to conduct a genetic variation determination method with a certain sensitivity or specificity, a minority nucleic acid quantification method may be implemented prior to, during or after genetic variation determination to identify those samples with greater than about 2%, 3%, 4%, 5%, 6%, 7%, 8%, 9%, 10%, 11%, 12%, 13%, 14%,15%,16%, 17%, 18%, 19%, 20%, 21%, 22%, 23%, 24%, 25% or more minority nucleic acid. In some cases, samples determined as having a certain threshold amount of minority nucleic acid (e.g., about 15% or more minority nucleic acid; about 4% or more minority nucleic acid) are further analyzed for a genetic variation, or the presence or absence of a genetic variation, for example. In certain cases, determinations of, for example, a genetic variation are selected (e.g., selected and communicated to a patient) only for samples having a certain threshold amount of a minority nucleic acid (e.g., about 15% or more minority nucleic acid; about 4% or more minority nucleic acid).

The amount of fetal nucleic acid (e.g., concentration, relative amount, absolute amount, copy number, and the like) in nucleic acid is determined in some cases. In certain cases, the amount of fetal nucleic acid in a sample is referred to as "fetal fraction." In some cases "fetal fraction" refers to the fraction of fetal nucleic acid in circulating cell-free nucleic acid in a sample (e.g., a blood sample, a serum sample, a plasma sample, a urine sample) obtained from a pregnant female. Certain methods described herein or known in the art for determining fetal fraction can be used for determining a minority species fraction.

In certain instances, fetal fraction may be determined according to markers specific to a male fetus (e.g., Y-chromosome STR markers (e.g., DYS 19, DYS 385, DYS 392 markers); RhD marker in RhD-negative females), allelic ratios of polymorphic sequences, or according to one or more markers specific to fetal nucleic acid and not maternal nucleic acid (e.g., differential epigenetic biomarkers (e.g., methylation) between mother and fetus, or fetal RNA markers in maternal blood plasma (see e.g., Lo, 2005, Journal of Histochemistry and Cytochemistry 53 (3): 293-296)). Determination of fetal fraction sometimes is performed using a fetal quantifier assay (FQA) as described, for example, in U.S. Patent Application Publication No. 2010/0105049. This type of assay allows for the detection and quantification of fetal nucleic acid in a maternal sample based on the methylation status of the nucleic acid in the sample.

In certain cases, a minority species fraction can be determined based on allelic ratios of polymorphic sequences (e.g., single nucleotide polymorphisms (SNPs)), such as, for example, using a method described in U.S. Patent Application Publication No. 2011/0224087. In such a method for determining fetal fraction, for example, nucleotide sequence reads are obtained for a maternal sample and fetal fraction is determined by comparing the total number of nucleotide sequence reads that map to a first allele and the total number of nucleotide sequence reads that map to a second allele at an informative polymorphic site (e.g., SNP) in a reference genome.

A minority species fraction can be determined, in some cases, using methods that incorporate information derived from chromosomal aberrations as described, for example, in International Patent Application Publication No. WO2014/055774. A minority species fraction can be determined, in some cases, using methods that incorporate information derived from sex chromosomes as described, for example, in U.S. Patent Application Publication No. 2013/0288244 and U.S. Patent Application Publication No. 2013/0338933.

A minority species fraction can be determined in some cases using methods that incorporate fragment length information (e.g., fragment length ratio (FLR) analysis, fetal ratio statistic (FRS) analysis as described in International Patent Application Publication No. WO2013/177086). Cell-free fetal nucleic acid fragments generally are shorter than maternally-derived nucleic acid fragments (see e.g., Chan et al. (2004) Clin. Chem. 50:88-92; Lo et al. (2010) Sci. Transl. Med. 2:61ra91). Thus, fetal fraction can be determined, in some cases, by counting fragments under a particular length threshold and comparing the counts, for example, to counts from fragments over a particular length threshold and/or to the amount of total nucleic acid in the sample. Methods for counting nucleic acid fragments of a particular length are described in further detail in International Patent Application Publication No. WO2013/177086.

A minority species fraction can be determined, in some cases, according to portion-specific fraction estimates (e.g., as described in International Patent Application Publication No. WO 2014/205401). Without being limited to theory, the amount of reads from fetal CCF fragments (e.g., fragments of a particular length, or range of lengths) often map with ranging frequencies to portions (e.g., within the same sample, e.g., within the same sequencing run). Also, without being limited to theory, certain portions, when compared among multiple samples, tend to have a similar representation of reads from fetal CCF fragments (e.g., fragments of a particular length, or range of lengths), and that the representation correlates with portion-specific fetal fractions (e.g., the relative amount, percentage or ratio of CCF fragments originating from a fetus). Portion-specific fetal fraction estimates generally are determined according to portion-specific parameters and their relation to fetal fraction.

In some cases, the determination of minority species fraction (e.g, fetal fraction) is not required or necessary for identifying the presence or absence of a genetic variation. In some cases, identifying the presence or absence of a genetic variation does not require a sequence differentiation of a minority nucleic acid versus a majority nucleic acid. In certain cases, this is because the summed contribution of both minority and majority sequences in a particular chromosome, chromosome portion or part thereof is analyzed. In some cases, identifying the presence or absence of a genetic variation does not rely on a priori sequence information that would distinguish minority nucleic acid from majority nucleic acid.

### Nucleic acid library

In some cases a nucleic acid library is a plurality of polynucleotide molecules (e.g., a sample of nucleic acids) that are prepared, assembled and/or modified for a specific process, non-limiting examples of which include immobilization on a solid phase (e.g., a solid support, a flow cell, a bead), enrichment, amplification, cloning, detection and/or for nucleic acid sequencing. In certain cases, a nucleic acid library is prepared prior to or during a sequencing process. A nucleic acid library (e.g., sequencing library) can be prepared by a suitable method as known in the art. A nucleic acid library can be prepared by a targeted or a non-targeted preparation process.

In some cases a library of nucleic acids is modified to comprise a chemical moiety (e.g., a functional group) configured for immobilization of nucleic acids to a solid support. In some cases a library of nucleic acids is modified to comprise a biomolecule (e.g., a functional group) and/or member of a binding pair configured for immobilization of the library to a solid support, non-limiting examples of which include thyroxin-binding globulin, steroid-binding proteins, antibodies, antigens, haptens, enzymes, lectins, nucleic acids, repressors, protein A, protein G, avidin, streptavidin, biotin, complement component Clq, nucleic acid-binding proteins, receptors, carbohydrates, oligonucleotides, polynucleotides, complementary nucleic acid sequences, the like and combinations thereof. Some examples of specific binding pairs include, without limitation: an avidin moiety and a biotin moiety; an antigenic epitope and an antibody or immunologically reactive fragment thereof; an antibody and a hapten; a digoxigen moiety and an anti-digoxigen antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; an oligonucleotide or polynucleotide and its corresponding complement; the like or combinations thereof.

In some cases, a library of nucleic acids is modified to comprise one or more polynucleotides of known composition, non-limiting examples of which include an identifier (e.g., a tag, an indexing tag), a capture sequence, a label, an adapter, a restriction enzyme site, a promoter, an enhancer, an origin of replication, a stem loop, a complimentary sequence (e.g., a primer binding site, an annealing site), a suitable integration site (e.g., a transposon, a viral integration site), a modified nucleotide, the like or combinations thereof. Polynucleotides of known sequence can be added at a suitable position, for example on the 5' end, 3' end or within a nucleic acid sequence. Polynucleotides of known sequence can be the same or different sequences. In some cases a polynucleotide of known sequence is configured to hybridize to one or more oligonucleotides immobilized on a surface (e.g., a surface in flow cell). For example, a nucleic acid molecule comprising a 5' known sequence may hybridize to a first plurality of oligonucleotides while the 3' known sequence may hybridize to a second plurality of oligonucleotides. In some cases a library of nucleic acid can comprise chromosome-specific tags, capture sequences, labels and/or adaptors. In some cases, a library of nucleic acids comprises one or more detectable labels. In some cases one or more detectable labels may be incorporated into a nucleic acid library at a 5' end, at a 3' end, and/or at any nucleotide position within a nucleic acid in the library. In some cases a library of nucleic acids comprises hybridized oligonucleotides. In certain cases hybridized oligonucleotides are labeled probes. In some cases a library of nucleic acids comprises hybridized oligonucleotide probes prior to immobilization on a solid phase.

In some cases, a polynucleotide of known sequence comprises a universal sequence. A universal sequence is a specific nucleotide sequence that is integrated into two or more nucleic acid molecules or two or more subsets of nucleic acid molecules where the universal sequence is the same for all molecules or subsets of molecules that it is integrated into. A universal sequence is often designed to hybridize to and/or amplify a plurality of different sequences using a single universal primer that is complementary to a universal sequence. In some cases two (e.g., a pair) or more universal sequences and/or universal primers are used. A universal primer often comprises a universal sequence. In some cases adapters (e.g., universal adapters) comprise universal sequences. In some cases one or more universal sequences are used to capture, identify and/or detect multiple species or subsets of nucleic acids.

In certain cases of preparing a nucleic acid library, (e.g., in certain sequencing by synthesis procedures), nucleic acids are size selected and/or fragmented into lengths of several hundred base pairs, or less (e.g., in preparation for library generation). In some cases, library preparation is performed without fragmentation (e.g., when using cell-free DNA).

In certain cases, a ligation-based library preparation method is used (e.g., ILLUMINA TRUSEQ, Illumina, San Diego CA). Ligation-based library preparation methods often make use of an adaptor (e.g., a methylated adaptor) design which can incorporate an index sequence at the initial ligation step and often can be used to prepare samples for single-read sequencing, paired-end sequencing and multiplexed sequencing. For example, nucleic acids (e.g., fragmented nucleic acids or cell-free DNA) may be end repaired by a fill-in reaction, an exonuclease reaction or a combination thereof. In some cases the resulting blunt-end repaired nucleic acid can then be extended by a single nucleotide, which is complementary to a single nucleotide overhang on the 3' end of an adapter/primer. Any nucleotide can be used for the extension/overhang nucleotides. In some cases nucleic acid library preparation comprises ligating an adapter oligonucleotide. Adapter oligonucleotides are often complementary to flow-cell anchors, and sometimes are utilized to immobilize a nucleic acid library to a solid support, such as the inside surface of a flow cell, for example. In some cases, an adapter oligonucleotide comprises an identifier, one or more sequencing primer hybridization sites (e.g., sequences complementary to universal sequencing primers, single end sequencing primers, paired end sequencing primers, multiplexed sequencing primers, and the like), or combinations thereof (e.g., adapter/sequencing, adapter/identifier, adapter/identifier/sequencing).

An identifier can be a suitable detectable label incorporated into or attached to a nucleic acid (e.g., a polynucleotide) that allows detection and/or identification of nucleic acids that comprise the identifier. In some cases an identifier is incorporated into or attached to a nucleic acid during a sequencing method (e.g., by a polymerase). Non-limiting examples of identifiers include nucleic acid tags, nucleic acid indexes or barcodes, a radiolabel (e.g., an isotope), metallic label, a fluorescent label, a chemiluminescent label, a phosphorescent label, a fluorophore quencher, a dye, a protein (e.g., an enzyme, an antibody or part thereof, a linker, a member of a binding pair), the like or combinations thereof. In some cases an identifier (e.g., a nucleic acid index or barcode) is a unique, known and/or identifiable sequence of nucleotides or nucleotide analogues. In some cases identifiers are six or more contiguous nucleotides. A multitude of fluorophores are available with a variety of different excitation and emission spectra. Any suitable type and/or number of fluorophores can be used as an identifier. In some cases 1 or more, 2 or more, 3 or more, 4 or more, 5 or more, 6 or more, 7 or more, 8 or more, 9 or more, 10 or more, 20 or more, 30 or more or 50 or more different identifiers are utilized in a method described herein (e.g., a nucleic acid detection and/or sequencing method). In some cases, one or two types of identifiers (e.g., fluorescent labels) are linked to each nucleic acid in a library. Detection and/or quantification of an identifier can be performed by a suitable method, apparatus or machine, non-limiting examples of which include flow cytometry, quantitative polymerase chain reaction (qPCR), gel electrophoresis, a luminometer, a fluorometer, a spectrophotometer, a suitable gene-chip or microarray analysis, Western blot, mass spectrometry, chromatography, cytofluorimetric analysis, fluorescence microscopy, a suitable fluorescence or digital imaging method, confocal laser scanning microscopy, laser scanning cytometry, affinity chromatography, manual batch mode separation, electric field suspension, a suitable nucleic acid sequencing method and/or nucleic acid sequencing apparatus, the like and combinations thereof.

In some cases, a transposon-based library preparation method is used (e.g., EPICENTRE NEXTERA, Epicentre, Madison, WI). Transposon-based methods typically use in vitro transposition to simultaneously fragment and tag DNA in a single-tube reaction (often allowing incorporation of platform-specific tags and optional barcodes), and prepare sequencer-ready libraries.

In some cases a nucleic acid library or parts thereof are amplified (e.g., amplified by a PCR-based method). In some cases a sequencing method comprises amplification of a nucleic acid library. A nucleic acid library can be amplified prior to or after immobilization on a solid support (e.g., a solid support in a flow cell). Nucleic acid amplification includes the process of amplifying or increasing the numbers of a nucleic acid template and/or of a complement thereof that are present (e.g., in a nucleic acid library), by producing one or more copies of the template and/or its complement. Amplification can be carried out by a suitable method. A nucleic acid library can be amplified by a thermocycling method or by an isothermal amplification method. In some cases a rolling circle amplification method is used. In some cases amplification takes place on a solid support (e.g., within a flow cell) where a nucleic acid library or portion thereof is immobilized. In certain sequencing methods, a nucleic acid library is added to a flow cell and immobilized by hybridization to anchors under suitable conditions. This type of nucleic acid amplification is often referred to as solid phase amplification. In some cases of solid phase amplification, all or a portion of the amplified products are synthesized by an extension initiating from an immobilized primer. Solid phase amplification reactions are analogous to standard solution phase amplifications except that at least one of the amplification oligonucleotides (e.g., primers) is immobilized on a solid support.

In some cases solid phase amplification comprises a nucleic acid amplification reaction comprising only one species of oligonucleotide primer immobilized to a surface. In certain cases solid phase amplification comprises a plurality of different immobilized oligonucleotide primer species. In some cases solid phase amplification may comprise a nucleic acid amplification reaction comprising one species of oligonucleotide primer immobilized on a solid surface and a second different oligonucleotide primer species in solution. Multiple different species of immobilized or solution based primers can be used. Non-limiting examples of solid phase nucleic acid amplification reactions include interfacial amplification, bridge amplification, emulsion PCR, WildFire amplification (e.g., U.S. Patent Application Publication No. 2013/0012399), the like or combinations thereof.

### Nucleic acid sequencing and processing

Methods provided herein generally include nucleic acid sequencing and analysis. In some cases, nucleic acid is sequenced and the sequencing product (e.g., a collection of sequence reads) is processed prior to, or in conjunction with, an analysis of the sequenced nucleic acid. For example, sequence reads may be processed according to one or more of the following: aligning, mapping, filtering portions, selecting portions, counting, normalizing, weighting, generating a profile, and the like, and combinations thereof. Certain processing steps may be performed in any order and certain processing steps may be repeated. For example, portions may be filtered followed by sequence read count normalization, and, in certain cases, sequence read counts may be normalized followed by portion filtering. In some cases, a portion filtering step is followed by sequence read count normalization followed by a further portion filtering step. Certain sequencing methods and processing steps are described in further detail below.

### Sequencing

In some cases, nucleic acid (e.g., nucleic acid fragments, sample nucleic acid, cell-free nucleic acid) is sequenced. In certain instances, a full or substantially full sequence is obtained and sometimes a partial sequence is obtained. Nucleic acid sequencing generally produces a collection of sequence reads. As used herein, "reads" (e.g., "a read," "a sequence read") are short nucleotide sequences produced by any sequencing process described herein or known in the art. Reads can be generated from one end of nucleic acid fragments ("single-end reads"), and sometimes are generated from both ends of nucleic acid fragments (e.g., paired-end reads, double-end reads).

The length of a sequence read is often associated with the particular sequencing technology. High-throughput methods, for example, provide sequence reads that can vary in size from tens to hundreds of base pairs (bp). Nanopore sequencing, for example, can provide sequence reads that can vary in size from tens to hundreds to thousands of base pairs. In some cases, sequence reads are of a mean, median, average or absolute length of about 15 bp to about 900 bp long. In certain cases sequence reads are of a mean, median, average or absolute length of about 1000 bp or more.

In some cases the nominal, average, mean or absolute length of single-end reads sometimes is about 15 contiguous nucleotides to about 50 or more contiguous nucleotides, about 15 contiguous nucleotides to about 40 or more contiguous nucleotides, and sometimes about 15 contiguous nucleotides or about 36 or more contiguous nucleotides. In certain cases the nominal, average, mean or absolute length of single-end reads is about 20 to about 30 bases, or about 24 to about 28 bases in length. In certain cases the nominal, average, mean or absolute length of single-end reads is about 1, 2, 3, 4, 5, 6, 7, 8, 9, 10, 11, 12, 13, 14, 15, 16, 17, 18, 19, 21, 22, 23, 24, 25, 26, 27, 28 or about 29 bases or more in length. In certain cases, the nominal, average, mean or absolute length of paired-end reads sometimes is about 10 contiguous nucleotides to about 25 contiguous nucleotides or more (e.g., about 10, 11, 12, 13, 14, 15, 16, 17, 18, 19, 20, 21, 22, 23, 24 or 25 nucleotides in length or more), about 15 contiguous nucleotides to about 20 contiguous nucleotides or more, and sometimes is about 17 contiguous nucleotides or about 18 contiguous nucleotides.

In some cases, nucleotide sequence reads obtained from a sample are partial nucleotide sequence reads. As used herein, "partial nucleotide sequence reads" refers to sequence reads of any length with incomplete sequence information, also referred to as sequence ambiguity. Partial nucleotide sequence reads may lack information regarding nucleobase identity and/or nucleobase position or order. Partial nucleotide sequence reads generally do not include sequence reads in which the only incomplete sequence information (or in which less than all of the bases are sequenced or determined) is from inadvertent or unintentional sequencing errors. Such sequencing errors can be inherent to certain sequencing processes and include, for example, incorrect calls for nucleobase identity, and missing or extra nucleobases. Thus, for partial nucleotide sequence reads herein, certain information about the sequence is often deliberately excluded. That is, one deliberately obtains sequence information with respect to less than all of the nucleobases or which might otherwise be characterized as or be a sequencing error. In some cases, a partial nucleotide sequence read can span a portion of a nucleic acid fragment. In some cases, a partial nucleotide sequence read can span the entire length of a nucleic acid fragment. Partial nucleotide sequence reads are described, for example, in International Patent Application Publication No. WO2013/052907.

Reads generally are representations of nucleotide sequences in a physical nucleic acid. For example, in a read containing an ATGC depiction of a sequence, "A" represents an adenine nucleotide, "T" represents a thymine nucleotide, "G" represents a guanine nucleotide and "C" represents a cytosine nucleotide, in a physical nucleic acid. Sequence reads obtained from a sample from a subject can be reads from a mixture of a minority nucleic acid and a majority nucleic acid. For example, sequence reads obtained from the blood of a pregnant female can be reads from a mixture of fetal nucleic acid and maternal nucleic acid. A mixture of relatively short reads can be transformed by processes described herein into a representation of genomic nucleic acid present in the subject, and/or a representation of genomic nucleic acid present in a fetus. In certain instances, a mixture of relatively short reads can be transformed into a representation of a copy number alteration, a genetic variation or an aneuploidy, for example. In one example, reads of a mixture of maternal and fetal nucleic acid can be transformed into a representation of a composite chromosome or a part thereof comprising features of one or both maternal and fetal chromosomes.

In some instances, circulating cell free nucleic acid fragments (CCF fragments) obtained from a pregnant female comprise nucleic acid fragments originating from fetal cells (i.e., fetal fragments) and nucleic acid fragments originating from maternal cells (i.e., maternal fragments). Sequence reads derived from CCF fragments originating from a fetus are referred to herein as "fetal reads." Sequence reads derived from CCF fragments originating from the genome of a pregnant female (e.g., a mother) bearing a fetus are referred to herein as "maternal reads." CCF fragments from which fetal reads are obtained are referred to herein as fetal templates and CCF fragments from which maternal reads are obtained are referred herein to as maternal templates.

In certain cases, "obtaining" nucleic acid sequence reads of a sample from a subject and/or "obtaining" nucleic acid sequence reads of a biological specimen from one or more reference persons can involve directly sequencing nucleic acid to obtain the sequence information. In some cases, "obtaining" can involve receiving sequence information obtained directly from a nucleic acid by another.

In some cases, some or all nucleic acids in a sample are enriched and/or amplified (e.g., non-specifically, e.g., by a PCR based method) prior to or during sequencing. In certain cases specific nucleic acid species or subsets in a sample are enriched and/or amplified prior to or during sequencing. In some cases, a species or subset of a pre-selected pool of nucleic acids is sequenced randomly. In some cases, nucleic acids in a sample are not enriched and/or amplified prior to or during sequencing.

In some cases, a representative fraction of a genome is sequenced and is sometimes referred to as "coverage" or "fold coverage." For example, a 1-fold coverage indicates that roughly 100% of the nucleotide sequences of the genome are represented by reads. In some instances, fold coverage is referred to as (and is directly proportional to) "sequencing depth." In some cases, "fold coverage" is a relative term referring to a prior sequencing run as a reference. For example, a second sequencing run may have 2-fold less coverage than a first sequencing run. In some cases a genome is sequenced with redundancy, where a given region of the genome can be covered by two or more reads or overlapping reads (e.g., a "fold coverage" greater than 1, e.g., a 2-fold coverage). In some cases, a genome (e.g., a whole genome) is sequenced with about 0.1-fold to about 100-fold coverage, about 0.2-fold to 20-fold coverage, or about 0.2-fold to about 1-fold coverage (e.g., about 0.2-, 0.3-, 0.4-, 0.5-, 0.6-, 0.7-, 0.8-, 0.9-, 1-, 2-, 3-, 4-, 5-, 6-, 7-, 8-, 9-, 10-, 15-, 20-, 30-, 40-, 50-, 60-, 70-, 80-, 90-fold coverage). In some cases, specific parts of a genome (e.g., genomic parts from targeted and/or probe-based methods) are sequenced and fold coverage values generally refer to the fraction of the specific genomic parts sequenced (i.e., fold coverage values do not refer to the whole genome). In some instances, specific genomic parts are sequenced at 1000-fold coverage or more. For example, specific genomic parts may be sequenced at 2000-fold, 5,000-fold, 10,000-fold, 20,000-fold, 30,000-fold, 40,000-fold or 50,000-fold coverage.

In some cases, one nucleic acid sample from one individual is sequenced. In certain cases, nucleic acids from each of two or more samples are sequenced, where samples are from one individual or from different individuals. In certain cases, nucleic acid samples from two or more biological samples are pooled, where each biological sample is from one individual or two or more individuals, and the pool is sequenced. In the latter cases, a nucleic acid sample from each biological sample often is identified by one or more unique identifiers.

In some cases, a sequencing method utilizes identifiers that allow multiplexing of sequence reactions in a sequencing process. The greater the number of unique identifiers, the greater the number of samples and/or chromosomes for detection, for example, that can be multiplexed in a sequencing process. A sequencing process can be performed using any suitable number of unique identifiers (e.g., 4, 8, 12, 24, 48, 96, or more).

A sequencing process sometimes makes use of a solid phase, and sometimes the solid phase comprises a flow cell on which nucleic acid from a library can be attached and reagents can be flowed and contacted with the attached nucleic acid. A flow cell sometimes includes flow cell lanes, and use of identifiers can facilitate analyzing a number of samples in each lane. A flow cell often is a solid support that can be configured to retain and/or allow the orderly passage of reagent solutions over bound analytes. Flow cells frequently are planar in shape, optically transparent, generally in the millimeter or sub-millimeter scale, and often have channels or lanes in which the analyte/reagent interaction occurs. In some cases the number of samples analyzed in a given flow cell lane is dependent on the number of unique identifiers utilized during library preparation and/or probe design. Multiplexing using 12 identifiers, for example, allows simultaneous analysis of 96 samples (e.g., equal to the number of wells in a 96 well microwell plate) in an 8 lane flow cell. Similarly, multiplexing using 48 identifiers, for example, allows simultaneous analysis of 384 samples (e.g., equal to the number of wells in a 384 well microwell plate) in an 8 lane flow cell. Non-limiting examples of commercially available multiplex sequencing kits include Illumina's multiplexing sample preparation oligonucleotide kit and multiplexing sequencing primers and PhiX control kit (e.g., Illumina's catalog numbers PE-400-1001 and PE-400-1002, respectively).

Any suitable method of sequencing nucleic acids can be used, non-limiting examples of which include Maxim & Gilbert, chain-termination methods, sequencing by synthesis, sequencing by ligation, sequencing by mass spectrometry, microscopy-based techniques, the like or combinations thereof. In some cases, a first generation technology, such as, for example, Sanger sequencing methods including automated Sanger sequencing methods, including microfluidic Sanger sequencing, can be used in a method provided herein. In some cases, sequencing technologies that include the use of nucleic acid imaging technologies (e.g., transmission electron microscopy (TEM) and atomic force microscopy (AFM)), can be used. In some cases, a high-throughput sequencing method is used. High-throughput sequencing methods generally involve clonally amplified DNA templates or single DNA molecules that are sequenced in a massively parallel fashion, sometimes within a flow cell. Next generation (e.g., 2nd and 3rd generation) sequencing techniques capable of sequencing DNA in a massively parallel fashion can be used for methods described herein and are collectively referred to herein as "massively parallel sequencing" (MPS). In some cases, MPS sequencing methods utilize a targeted approach, where specific chromosomes, genes or regions of interest are sequenced. In certain cases, a non-targeted approach is used where most or all nucleic acids in a sample are sequenced, amplified and/or captured randomly.

In some cases a targeted enrichment, amplification and/or sequencing approach is used. A targeted approach often isolates, selects and/or enriches a subset of nucleic acids in a sample for further processing by use of sequence-specific oligonucleotides. In some cases a library of sequence-specific oligonucleotides are utilized to target (e.g., hybridize to) one or more sets of nucleic acids in a sample. Sequence-specific oligonucleotides and/or primers are often selective for particular sequences (e.g., unique nucleic acid sequences) present in one or more chromosomes, genes, exons, introns, and/or regulatory regions of interest. Any suitable method or combination of methods can be used for enrichment, amplification and/or sequencing of one or more subsets of targeted nucleic acids. In some cases targeted sequences are isolated and/or enriched by capture to a solid phase (e.g., a flow cell, a bead) using one or more sequence-specific anchors. In some cases targeted sequences are enriched and/or amplified by a polymerase-based method (e.g., a PCR-based method, by any suitable polymerase based extension) using sequence-specific primers and/or primer sets. Sequence specific anchors often can be used as sequence-specific primers.

MPS sequencing sometimes makes use of sequencing by synthesis and certain imaging processes. A nucleic acid sequencing technology that may be used in a method described herein is sequencing-by-synthesis and reversible terminator-based sequencing (e.g., Illumina's Genome Analyzer; Genome Analyzer II; HISEQ 2000; HISEQ 2500 (Illumina, San Diego CA)). With this technology, millions of nucleic acid (e.g., DNA) fragments can be sequenced in parallel. In one example of this type of sequencing technology, a flow cell is used which contains an optically transparent slide with 8 individual lanes on the surfaces of which are bound oligonucleotide anchors (e.g., adaptor primers).

Sequencing by synthesis generally is performed by iteratively adding (e.g., by covalent addition) a nucleotide to a primer or preexisting nucleic acid strand in a template directed manner. Each iterative addition of a nucleotide is detected and the process is repeated multiple times until a sequence of a nucleic acid strand is obtained. The length of a sequence obtained depends, in part, on the number of addition and detection steps that are performed. In some cases of sequencing by synthesis, one, two, three or more nucleotides of the same type (e.g., A, G, C or T) are added and detected in a round of nucleotide addition. Nucleotides can be added by any suitable method (e.g., enzymatically or chemically). For example, in some cases a polymerase or a ligase adds a nucleotide to a primer or to a preexisting nucleic acid strand in a template directed manner. In some cases of sequencing by synthesis, different types of nucleotides, nucleotide analogues and/or identifiers are used. In some cases reversible terminators and/or removable (e.g., cleavable) identifiers are used. In some cases fluorescent labeled nucleotides and/or nucleotide analogues are used. In certain cases sequencing by synthesis comprises a cleavage (e.g., cleavage and removal of an identifier) and/or a washing step. In some cases the addition of one or more nucleotides is detected by a suitable method described herein or known in the art, non-limiting examples of which include any suitable imaging apparatus, a suitable camera, a digital camera, a CCD (Charge Couple Device) based imaging apparatus (e.g., a CCD camera), a CMOS (Complementary Metal Oxide Silicon) based imaging apparatus (e.g., a CMOS camera), a photo diode (e.g., a photomultiplier tube), electron microscopy, a field-effect transistor (e.g., a DNA field-effect transistor), an ISFET ion sensor (e.g., a CHEMFET sensor), the like or combinations thereof.

Any suitable MPS method, system or technology platform for conducting methods described herein can be used to obtain nucleic acid sequence reads. Non-limiting examples of MPS platforms include Illumina/Solex/HiSeq (e.g., Illumina's Genome Analyzer; Genome Analyzer II; HISEQ 2000; HISEQ), SOLiD, Roche/454, PACBIO and/or SMRT, Helicos True Single Molecule Sequencing, Ion Torrent and Ion semiconductor-based sequencing (e.g., as developed by Life Technologies), WildFire, 5500, 5500xl W and/or 5500xl W Genetic Analyzer based technologies (e.g., as developed and sold by Life Technologies, U.S. Patent Application Publication No. 2013/0012399); Polony sequencing, Pyrosequencing, Massively Parallel Signature Sequencing (MPSS), RNA polymerase (RNAP) sequencing, LaserGen systems and methods, Nanopore-based platforms, chemical-sensitive field effect transistor (CHEMFET) array, electron microscopy-based sequencing (e.g., as developed by ZS Genetics, Halcyon Molecular), nanoball sequencing, the like or combinations thereof. Other sequencing methods that may be used to conduct methods herein include digital PCR, sequencing by hybridization, nanopore sequencing, chromosome-specific sequencing (e.g., using DANSR (digital analysis of selected regions) technology.

In some cases, sequence reads are generated, obtained, gathered, assembled, manipulated, transformed, processed, and/or provided by a sequence module. A machine comprising a sequence module can be a suitable machine and/or apparatus that determines the sequence of a nucleic acid utilizing a sequencing technology known in the art. In some cases a sequence module can align, assemble, fragment, complement, reverse complement, and/or error check (e.g., error correct sequence reads).

### Mapping reads

Sequence reads can be mapped and the number of reads mapping to a specified nucleic acid region (e.g., a chromosome or portion thereof) are referred to as counts. Any suitable mapping method (e.g., process, algorithm, program, software, module, the like or combination thereof) can be used. Certain aspects of mapping processes are described hereafter.

Mapping nucleotide sequence reads (i.e., sequence information from a fragment whose physical genomic position is unknown) can be performed in a number of ways, and often comprises alignment of the obtained sequence reads with a matching sequence in a reference genome. In such alignments, sequence reads generally are aligned to a reference sequence and those that align are designated as being "mapped," as "a mapped sequence read" or as "a mapped read." In certain cases, a mapped sequence read is referred to as a "hit" or "count." In some cases, mapped sequence reads are grouped together according to various parameters and assigned to particular genomic portions, which are discussed in further detail below.

The terms "aligned," "alignment," or "aligning" generally refer to two or more nucleic acid sequences that can be identified as a match (e.g., 100% identity) or partial match. Alignments can be done manually or by a computer (e.g., a software, program, module, or algorithm), non-limiting examples of which include the Efficient Local Alignment of Nucleotide Data (ELAND) computer program distributed as part of the Illumina Genomics Analysis pipeline. Alignment of a sequence read can be a 100% sequence match. In some cases, an alignment is less than a 100% sequence match (i.e., non-perfect match, partial match, partial alignment). In some cases an alignment is about a 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76% or 75% match. In some cases, an alignment comprises a mismatch. In some cases, an alignment comprises 1, 2, 3, 4 or 5 mismatches. Two or more sequences can be aligned using either strand (e.g., sense or antisense strand). In certain cases a nucleic acid sequence is aligned with the reverse complement of another nucleic acid sequence.

Various computational methods can be used to map each sequence read to a portion. Non-limiting examples of computer algorithms that can be used to align sequences include, without limitation, BLAST, BLITZ, FASTA, BOWTIE 1, BOWTIE 2, ELAND, MAQ, PROBEMATCH, SOAP, BWA or SEQMAP, or variations thereof or combinations thereof. In some cases, sequence reads can be aligned with sequences in a reference genome. In some cases, sequence reads can be found and/or aligned with sequences in nucleic acid databases known in the art including, for example, GenBank, dbEST, dbSTS, EMBL (European Molecular Biology Laboratory) and DDBJ (DNA Databank of Japan). BLAST or similar tools can be used to search identified sequences against a sequence database. Search hits can then be used to sort the identified sequences into appropriate portions (described hereafter), for example.

In some cases, a read may uniquely or non-uniquely map to portions in a reference genome. A read is considered as "uniquely mapped" if it aligns with a single sequence in the reference genome. A read is considered as "non-uniquely mapped" if it aligns with two or more sequences in the reference genome. In some cases, non-uniquely mapped reads are eliminated from further analysis (e.g. quantification). A certain, small degree of mismatch (0-1) may be allowed to account for single nucleotide polymorphisms that may exist between the reference genome and the reads from individual samples being mapped, in certain cases. In some cases, no degree of mismatch is allowed for a read mapped to a reference sequence.

As used herein, the term "reference genome" can refer to any particular known, sequenced or characterized genome, whether partial or complete, of any organism or virus which may be used to reference identified sequences from a subject. For example, a reference genome used for human subjects as well as many other organisms can be found at the National Center for Biotechnology Information at World Wide Web URL ncbi.nlm.nih.gov. A "genome" refers to the complete genetic information of an organism or virus, expressed in nucleic acid sequences. As used herein, a reference sequence or reference genome often is an assembled or partially assembled genomic sequence from an individual or multiple individuals. In some cases, a reference genome is an assembled or partially assembled genomic sequence from one or more human individuals. In some cases, a reference genome comprises sequences assigned to chromosomes.

In certain cases, mappability is assessed for a genomic region (e.g., portion, genomic portion). Mappability is the ability to unambiguously align a nucleotide sequence read to a portion of a reference genome, typically up to a specified number of mismatches, including, for example, 0, 1, 2 or more mismatches. For a given genomic region, the expected mappability can be estimated using a sliding-window approach of a preset read length and averaging the resulting read-level mappability values. Genomic regions comprising stretches of unique nucleotide sequence sometimes have a high mappability value.

### Portions

In some cases, mapped sequence reads are grouped together according to various parameters and assigned to particular genomic portions (e.g., portions of a reference genome). A "portion" also may be referred to herein as a "genomic section," "bin," "partition," "portion of a reference genome," "portion of a chromosome" or "genomic portion."

A portion often is defined by partitioning of a genome according to one or more features. Non-limiting examples of certain partitioning features include length (e.g., fixed length, non-fixed length) and other structural features. Genomic portions sometimes include one or more of the following features: fixed length, non-fixed length, random length, non-random length, equal length, unequal length (e.g., at least two of the genomic portions are of unequal length), do not overlap (e.g., the 3' ends of the genomic portions sometimes abut the 5' ends of adjacent genomic portions), overlap (e.g., at least two of the genomic portions overlap), contiguous, consecutive, not contiguous, and not consecutive. Genomic portions sometimes are about 1 to about 1,000 kilobases in length (e.g., about 2, 3, 4, 5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, 900 kilobases in length), about 5 to about 500 kilobases in length, about 10 to about 100 kilobases in length, or about 40 to about 60 kilobases in length.

Partitioning sometimes is based on, or is based in part on certain informational features, such as, information content and information gain, for example. Non-limiting examples of certain informational features include speed and/or convenience of alignment, sequencing coverage variability, GC content (e.g., stratified GC content, particular GC contents, high or low GC content), uniformity of GC content, other measures of sequence content (e.g., fraction of individual nucleotides, fraction of pyrimidines or purines, fraction of natural vs. non-natural nucleic acids, fraction of methylated nucleotides, and CpG content), methylation state, duplex melting temperature, amenability to sequencing or PCR, uncertainty value assigned to individual portions of a reference genome, and/or a targeted search for particular features. In some cases, information content may be quantified using a p-value profile measuring the significance of particular genomic locations for distinguishing between groups of confirmed normal and abnormal subjects (e.g. euploid and trisomy subjects, respectively).

In some cases, partitioning a genome may eliminate similar regions (e.g., identical or homologous regions or sequences) across a genome and only keep unique regions. Regions removed during partitioning may be within a single chromosome, may be one or more chromosomes, or may span multiple chromosomes. In some cases, a partitioned genome is reduced and optimized for faster alignment, often focusing on uniquely identifiable sequences.

In some cases, genomic portions result from a partitioning based on non-overlapping fixed size, which results in consecutive, non-overlapping portions of fixed length. Such portions often are shorter than a chromosome and often are shorter than a copy number variation region (e.g., a region that is duplicated or is deleted), the latter of which can be referred to as a segment. A "segment" or "genomic segment" often includes two or more fixed-length genomic portions, and often includes two or more consecutive fixed-length portions (e.g., about 2 to about 100 such portions (e.g., 2, 3, 4, 5, 6, 7, 8, 9, 10, 11, 12, 13, 14, 15, 16, 17, 18, 19, 20, 25, 30, 35, 40, 45, 50, 60, 70, 80, 90 such portions)).

Multiple portions sometimes are analyzed in groups, and sometimes reads mapped to portions are quantified according to a particular group of genomic portions. Where portions are partitioned by structural features and correspond to regions in a genome, portions sometimes are grouped into one or more segments and/or one or more regions. Non-limiting examples of regions include sub-chromosome (i.e., shorter than a chromosome), chromosome, autosome, sex chromosome and combinations thereof. One or more sub-chromosome regions sometimes are genes, gene fragments, regulatory sequences, introns, exons, segments (e.g., a segment spanning a copy number alteration region), microduplications, microdeletions and the like. A region sometimes is smaller than a chromosome of interest or is the same size of a chromosome of interest, and sometimes is smaller than a reference chromosome or is the same size as a reference chromosome.

### Filtering and/or selecting portions

In some cases, one or more processing steps can comprise one or more portion filtering steps and/or portion selection steps. The term "filtering" as used herein refers to removing portions or portions of a reference genome from consideration. In certain cases one or more portions are filtered (e.g., subjected to a filtering process) thereby providing filtered portions. In some cases a filtering process removes certain portions and retains portions (e.g., a subset of portions). Following a filtering process, retained portions are often referred to herein as filtered portions.

Portions of a reference genome can be selected for removal based on any suitable criteria, including but not limited to redundant data (e.g., redundant or overlapping mapped reads), non-informative data (e.g., portions of a reference genome with zero median counts), portions of a reference genome with over represented or under represented sequences, noisy data, the like, or combinations of the foregoing. A filtering process often involves removing one or more portions of a reference genome from consideration and subtracting the counts in the one or more portions of a reference genome selected for removal from the counted or summed counts for the portions of a reference genome, chromosome or chromosomes, or genome under consideration. In some cases, portions of a reference genome can be removed successively (e.g., one at a time to allow evaluation of the effect of removal of each individual portion), and in certain cases all portions of a reference genome marked for removal can be removed at the same time. In some cases, portions of a reference genome characterized by a variance above or below a certain level are removed, which sometimes is referred to herein as filtering "noisy" portions of a reference genome. In certain cases, a filtering process comprises obtaining data points from a data set that deviate from the mean profile level of a portion, a chromosome, or part of a chromosome by a predetermined multiple of the profile variance, and in certain cases, a filtering process comprises removing data points from a data set that do not deviate from the mean profile level of a portion, a chromosome or part of a chromosome by a predetermined multiple of the profile variance. In some cases, a filtering process is utilized to reduce the number of candidate portions of a reference genome analyzed for the presence or absence of a genetic variation and/or copy number alteration (e.g., aneuploidy, microdeletion, microduplication). Reducing the number of candidate portions of a reference genome analyzed for the presence or absence of a genetic variation and/or copy number alteration often reduces the complexity and/or dimensionality of a data set, and sometimes increases the speed of searching for and/or identifying genetic variations and/or copy number alterations by two or more orders of magnitude.

Portions may be processed (e.g., filtered and/or selected) by any suitable method and according to any suitable parameter. Non-limiting examples of features and/or parameters that can be used to filter and/or select portions include redundant data (e.g., redundant or overlapping mapped reads), non-informative data (e.g., portions of a reference genome with zero mapped counts), portions of a reference genome with over represented or under represented sequences, noisy data, counts, count variability, coverage, mappability, variability, a repeatability measure, read density, variability of read density, a level of uncertainty, guanine-cytosine (GC) content, CCF fragment length and/or read length (e.g., a fragment length ratio (FLR), a fetal ratio statistic (FRS)), DNaseI-sensitivity, methylation state, acetylation, histone distribution, chromatin structure, percent repeats, the like or combinations thereof. Portions can be filtered and/or selected according to any suitable feature or parameter that correlates with a feature or parameter listed or described herein. Portions can be filtered and/or selected according to features or parameters that are specific to a portion (e.g., as determined for a single portion according to multiple samples) and/or features or parameters that are specific to a sample (e.g., as determined for multiple portions within a sample). In some cases portions are filtered and/or removed according to relatively low mappability, relatively high variability, a high level of uncertainty, relatively long CCF fragment lengths (e.g., low FRS, low FLR), relatively large fraction of repetitive sequences, high GC content, low GC content, low counts, zero counts, high counts, the like, or combinations thereof. In some cases portions (e.g., a subset of portions) are selected according to suitable level of mappability, variability, level of uncertainty, fraction of repetitive sequences, count, GC content, the like, or combinations thereof. In some cases portions (e.g., a subset of portions) are selected according to relatively short CCF fragment lengths (e.g., high FRS, high FLR). Counts and/or reads mapped to portions are sometimes processed (e.g., normalized) prior to and/or after filtering or selecting portions (e.g., a subset of portions). In some cases counts and/or reads mapped to portions are not processed prior to and/or after filtering or selecting portions (e.g., a subset of portions).

In some cases, portions may be filtered according to a measure of error (e.g., standard deviation, standard error, calculated variance, p-value, mean absolute error (MAE), average absolute deviation and/or mean absolute deviation (MAD)). In certain instances, a measure of error may refer to count variability. In some cases portions are filtered according to count variability. In certain cases count variability is a measure of error determined for counts mapped to a portion (i.e., portion) of a reference genome for multiple samples (e.g., multiple sample obtained from multiple subjects, e.g., 50 or more, 100 or more, 500 or more 1000 or more, 5000 or more or 10,000 or more subjects). In some cases, portions with a count variability above a pre-determined upper range are filtered (e.g., excluded from consideration). In some cases portions with a count variability below a pre-determined lower range are filtered (e.g., excluded from consideration). In some cases, portions with a count variability outside a pre-determined range are filtered (e.g., excluded from consideration). In some cases portions with a count variability within a pre-determined range are selected (e.g., used for determining the presence or absence of a copy number alteration). In some cases, count variability of portions represents a distribution (e.g., a normal distribution). In some cases portions are selected within a quantile of the distribution. In some cases portions within a 99% quantile of the distribution of count variability are selected.

Sequence reads from any suitable number of samples can be utilized to identify a subset of portions that meet one or more criteria, parameters and/or features described herein. Sequence reads from a group of samples from multiple subjects sometimes are utilized. In some cases, the multiple subjects include pregnant females. In some cases, the multiple subjects include healthy subjects. One or more samples from each of the multiple subjects can be addressed (e.g., 1 to about 20 samples from each subject (e.g., about 2, 3, 4, 5, 6, 7, 8, 9, 10, 11, 12, 13, 14, 15, 16, 17, 18 or 19 samples)), and a suitable number of subjects may be addressed (e.g., about 2 to about 10,000 subjects (e.g., about 10, 20, 30, 40, 50, 60, 70, 80, 90, 100, 150, 200, 250, 300, 350, 400, 500, 600, 700, 800, 900, 1000, 2000, 3000, 4000, 5000, 6000, 7000, 8000, 9000 subjects)). In some cases, sequence reads from the same test sample(s) from the same subject are mapped to portions in the reference genome and are used to generate the subset of portions.

Portions can be selected and/or filtered by any suitable method. In some cases portions are selected according to visual inspection of data, graphs, plots and/or charts. In certain cases portions are selected and/or filtered (e.g., in part) by a system or a machine comprising one or more microprocessors and memory. In some cases portions are selected and/or filtered (e.g., in part) by a non-transitory computer-readable storage medium with an executable program stored thereon, where the program instructs a microprocessor to perform the selecting and/or filtering.

In some cases, sequence reads derived from a sample are mapped to all or most portions of a reference genome and a pre-selected subset of portions are thereafter selected. For example, a subset of portions to which reads from fragments under a particular length threshold preferentially map may be selected. Certain methods for pre-selecting a subset of portions are described in U.S. Patent Application Publication No. 2014/0180594. Reads from a selected subset of portions often are utilized in further steps of a determination of the presence or absence of a genetic variation, for example. Often, reads from portions not selected are not utilized in further steps of a determination of the presence or absence of a genetic variation (e.g., reads in the non-selected portions are removed or filtered).

In some cases portions associated with read densities (e.g., where a read density is for a portion) are removed by a filtering process and read densities associated with removed portions are not included in a determination of the presence or absence of a copy number alteration (e.g., a chromosome aneuploidy, microduplication, microdeletion). In some cases a read density profile comprises and/or consists of read densities of filtered portions. Portions are sometimes filtered according to a distribution of counts and/or a distribution of read densities. In some cases portions are filtered according to a distribution of counts and/or read densities where the counts and/or read densities are obtained from one or more reference samples. One or more reference samples may be referred to herein as a training set. In some cases portions are filtered according to a distribution of counts and/or read densities where the counts and/or read densities are obtained from one or more test samples. In some cases portions are filtered according to a measure of uncertainty for a read density distribution. In certain cases, portions that demonstrate a large deviation in read densities are removed by a filtering process. For example, a distribution of read densities (e.g., a distribution of average mean, or median read densities) can be determined, where each read density in the distribution maps to the same portion. A measure of uncertainty (e.g., a MAD) can be determined by comparing a distribution of read densities for multiple samples where each portion of a genome is associated with measure of uncertainty. According to the foregoing example, portions can be filtered according to a measure of uncertainty (e.g., a standard deviation (SD), a MAD) associated with each portion and a predetermined threshold. In certain instances, portions comprising MAD values within the acceptable range are retained and portions comprising MAD values outside of the acceptable range are removed from consideration by a filtering process. In some cases, according to the foregoing example, portions comprising read densities values (e.g., median, average or mean read densities) outside a pre-determined measure of uncertainty are often removed from consideration by a filtering process. In some cases portions comprising read densities values (e.g., median, average or mean read densities) outside an inter-quartile range of a distribution are removed from consideration by a filtering process. In some cases portions comprising read densities values outside more than 2 times, 3 times, 4 times or 5 times an inter-quartile range of a distribution are removed from consideration by a filtering process. In some cases portions comprising read densities values outside more than 2 sigma, 3 sigma, 4 sigma, 5 sigma, 6 sigma, 7 sigma or 8 sigma (e.g., where sigma is a range defined by a standard deviation) are removed from consideration by a filtering process.

### Sequence read quantification

Sequence reads that are mapped or partitioned based on a selected feature or variable can be quantified to determine the amount or number of reads that are mapped to one or more portions (e.g., portion of a reference genome), in some cases. In certain cases the quantity of sequence reads that are mapped to a portion or segment is referred to as a count or read density.

A count often is associated with a genomic portion. In some cases a count is determined from some or all of the sequence reads mapped to (i.e., associated with) a portion. In certain cases, a count is determined from some or all of the sequence reads mapped to a group of portions (e.g., portions in a segment or region (described herein)).

A count can be determined by a suitable method, operation or mathematical process. A count sometimes is the direct sum of all sequence reads mapped to a genomic portion or a group of genomic portions corresponding to a segment, a group of portions corresponding to a subregion of a genome (e.g., copy number variation region, copy number duplication region, copy number deletion region, microduplication region, microdeletion region, chromosome region, autosome region, sex chromosome region) and/or sometimes is a group of portions corresponding to a genome. A read quantification sometimes is a ratio, and sometimes is a ratio of a quantification for portion(s) in region *a* to a quantification for portion(s) in region *b.* Region *a* sometimes is one portion, segment region, copy number variation region, copy number duplication region, copy number deletion region, microduplication region, microdeletion region, chromosome region, autosome region and/or sex chromosome region. Region b independently sometimes is one portion, segment region, copy number variation region, copy number duplication region, copy number deletion region, microduplication region, microdeletion region, chromosome region, autosome region, sex chromosome region, a region including all autosomes, a region including sex chromosomes and/or a region including all chromosomes.

In some cases, a count is derived from raw sequence reads and/or filtered sequence reads. In certain cases a count is an average, mean or sum of sequence reads mapped to a genomic portion or group of genomic portions (e.g., genomic portions in a region). In some cases, a count is associated with an uncertainty value. A count sometimes is adjusted. A count may be adjusted according to sequence reads associated with a genomic portion or group of portions that have been weighted, removed, filtered, normalized, adjusted, averaged, derived as a mean, derived as a median, added, or combination thereof.

A sequence read quantification sometimes is a read density. A read density may be determined and/or generated for one or more segments of a genome. In certain instances, a read density may be determined and/or generated for one or more chromosomes. In some cases a read density comprises a quantitative measure of counts of sequence reads mapped to a segment or portion of a reference genome. A read density can be determined by a suitable process. In some cases a read density is determined by a suitable distribution and/or a suitable distribution function. Non-limiting examples of a distribution function include a probability function, probability distribution function, probability density function (PDF), a kernel density function (kernel density estimation), a cumulative distribution function, probability mass function, discrete probability distribution, an absolutely continuous univariate distribution, the like, any suitable distribution, or combinations thereof. A read density may be a density estimation derived from a suitable probability density function. A density estimation is the construction of an estimate, based on observed data, of an underlying probability density function. In some cases a read density comprises a density estimation (e.g., a probability density estimation, a kernel density estimation). A read density may be generated according to a process comprising generating a density estimation for each of the one or more portions of a genome where each portion comprises counts of sequence reads. A read density may be generated for normalized and/or weighted counts mapped to a portion or segment. In some instances, each read mapped to a portion or segment may contribute to a read density, a value (e.g., a count) equal to its weight obtained from a normalization process described herein. In some cases read densities for one or more portions or segments are adjusted. Read densities can be adjusted by a suitable method. For example, read densities for one or more portions can be weighted and/or normalized.

Reads quantified for a given portion or segment can be from one source or different sources. In one example, reads may be obtained from a nucleic acid sample from a pregnant female bearing a fetus. In such circumstances, reads mapped to one or more portions often are reads representative of both the fetus and the mother of the fetus (e.g., a pregnant female subject). In certain cases some of the reads mapped to a portion are from a fetal genome and some of the reads mapped to the same portion are from a maternal genome.

### Levels

In some cases, a value (e.g., a number, a quantitative value) is ascribed to a level. A level can be determined by a suitable method, operation or mathematical process (e.g., a processed level). A level often is, or is derived from, counts (e.g., normalized counts) for a set of portions. In some cases a level of a portion is substantially equal to the total number of counts mapped to a portion (e.g., counts, normalized counts). Often a level is determined from counts that are processed, transformed or manipulated by a suitable method, operation or mathematical process known in the art. In some cases a level is derived from counts that are processed and non-limiting examples of processed counts include weighted, removed, filtered, normalized, adjusted, averaged, derived as a mean (e.g., mean level), added, subtracted, transformed counts or combination thereof. In some cases a level comprises counts that are normalized (e.g., normalized counts of portions). A level can be for counts normalized by a suitable process, non-limiting examples of which are described herein. A level can comprise normalized counts or relative amounts of counts. In some cases a level is for counts or normalized counts of two or more portions that are averaged and the level is referred to as an average level. In some cases a level is for a set of portions having a mean count or mean of normalized counts which is referred to as a mean level. In some cases a level is derived for portions that comprise raw and/or filtered counts. In some cases, a level is based on counts that are raw. In some cases a level is associated with an uncertainty value (e.g., a standard deviation, a MAD). In some cases a level is represented by a Z-score or p-value.

A level for one or more portions is synonymous with a "genomic section level" herein. The term "level" as used herein is sometimes synonymous with the term "elevation." A determination of the meaning of the term "level" can be determined from the context in which it is used. For example, the term "level," when used in the context of portions, profiles, reads and/or counts often means an elevation. The term "level," when used in the context of a substance or composition (e.g., level of RNA, plexing level) often refers to an amount. The term "level," when used in the context of uncertainty (e.g., level of error, level of confidence, level of deviation, level of uncertainty) often refers to an amount.

Normalized or non-normalized counts for two or more levels (e.g., two or more levels in a profile) can sometimes be mathematically manipulated (e.g., added, multiplied, averaged, normalized, the like or combination thereof) according to levels. For example, normalized or non-normalized counts for two or more levels can be normalized according to one, some or all of the levels in a profile. In some cases normalized or non-normalized counts of all levels in a profile are normalized according to one level in the profile. In some cases normalized or non-normalized counts of a fist level in a profile are normalized according to normalized or non-normalized counts of a second level in the profile.

Non-limiting examples of a level (e.g., a first level, a second level) are a level for a set of portions comprising processed counts, a level for a set of portions comprising a mean, median or average of counts, a level for a set of portions comprising normalized counts, the like or any combination thereof. In some cases, a first level and a second level in a profile are derived from counts of portions mapped to the same chromosome. In some cases, a first level and a second level in a profile are derived from counts of portions mapped to different chromosomes.

In some cases a level is determined from normalized or non-normalized counts mapped to one or more portions. In some cases, a level is determined from normalized or non-normalized counts mapped to two or more portions, where the normalized counts for each portion often are about the same. There can be variation in counts (e.g., normalized counts) in a set of portions for a level. In a set of portions for a level there can be one or more portions having counts that are significantly different than in other portions of the set (e.g., peaks and/or dips). Any suitable number of normalized or non-normalized counts associated with any suitable number of portions can define a level.

In some cases one or more levels can be determined from normalized or non-normalized counts of all or some of the portions of a genome. Often a level can be determined from all or some of the normalized or non-normalized counts of a chromosome, or part thereof. In some cases, two or more counts derived from two or more portions (e.g., a set of portions) determine a level. In some cases two or more counts (e.g., counts from two or more portions) determine a level. In some cases, counts from 2 to about 100,000 portions determine a level. In some cases, counts from 2 to about 50,000, 2 to about 40,000, 2 to about 30,000, 2 to about 20,000, 2 to about 10,000, 2 to about 5000, 2 to about 2500, 2 to about 1250, 2 to about 1000, 2 to about 500, 2 to about 250, 2 to about 100 or 2 to about 60 portions determine a level. In some cases counts from about 10 to about 50 portions determine a level. In some cases counts from about 20 to about 40 or more portions determine a level. In some cases, a level comprises counts from about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60 or more portions. In some cases, a level corresponds to a set of portions (e.g., a set of portions of a reference genome, a set of portions of a chromosome or a set of portions of a part of a chromosome).

In some cases, a level is determined for normalized or non-normalized counts of portions that are contiguous. In some cases portions (e.g., a set of portions) that are contiguous represent neighboring regions of a genome or neighboring regions of a chromosome or gene. For example, two or more contiguous portions, when aligned by merging the portions end to end, can represent a sequence assembly of a DNA sequence longer than each portion. For example two or more contiguous portions can represent of an intact genome, chromosome, gene, intron, exon or part thereof. In some cases a level is determined from a collection (e.g., a set) of contiguous portions and/or non-contiguous portions.

### Data processing and normalization

Mapped sequence reads that have been counted are referred to herein as raw data, since the data represents unmanipulated counts (e.g., raw counts). In some cases, sequence read data in a data set can be processed further (e.g., mathematically and/or statistically manipulated) and/or displayed to facilitate providing an outcome. In certain cases, data sets, including larger data sets, may benefit from pre-processing to facilitate further analysis. Pre-processing of data sets sometimes involves removal of redundant and/or uninformative portions or portions of a reference genome (e.g., portions of a reference genome with uninformative data, redundant mapped reads, portions with zero median counts, over represented or under represented sequences). Without being limited by theory, data processing and/or preprocessing may (i) remove noisy data, (ii) remove uninformative data, (iii) remove redundant data, (iv) reduce the complexity of larger data sets, and/or (v) facilitate transformation of the data from one form into one or more other forms. The terms "pre-processing" and "processing" when utilized with respect to data or data sets are collectively referred to herein as "processing." Processing can render data more amenable to further analysis, and can generate an outcome in some cases. In some cases one or more or all processing methods (e.g., normalization methods, portion filtering, mapping, validation, the like or combinations thereof) are performed by a processor, a microprocessor, a computer, in conjunction with memory and/or by a microprocessor controlled apparatus.

The term "noisy data" as used herein refers to (a) data that has a significant variance between data points when analyzed or plotted, (b) data that has a significant standard deviation (e.g., greater than 3 standard deviations), (c) data that has a significant standard error of the mean, the like, and combinations of the foregoing. Noisy data sometimes occurs due to the quantity and/or quality of starting material (e.g., nucleic acid sample), and sometimes occurs as part of processes for preparing or replicating DNA used to generate sequence reads. In certain cases, noise results from certain sequences being overrepresented when prepared using PCR-based methods. Methods described herein can reduce or eliminate the contribution of noisy data, and therefore reduce the effect of noisy data on the provided outcome.

The terms "uninformative data," "uninformative portions of a reference genome," and "uninformative portions" as used herein refer to portions, or data derived therefrom, having a numerical value that is significantly different from a predetermined threshold value or falls outside a predetermined cutoff range of values. The terms "threshold" and "threshold value" herein refer to any number that is calculated using a qualifying data set and serves as a limit of diagnosis of a genetic variation (e.g., a copy number alteration, an aneuploidy, a microduplication, a microdeletion, a chromosomal aberration, and the like). In certain cases, a threshold is exceeded by results obtained by methods described herein and a subject is diagnosed with a copy number alteration. A threshold value or range of values often is calculated by mathematically and/or statistically manipulating sequence read data (e.g., from a reference and/or subject), in some cases, and in certain cases, sequence read data manipulated to generate a threshold value or range of values is sequence read data (e.g., from a reference and/or subject). In some cases, an uncertainty value is determined. An uncertainty value generally is a measure of variance or error and can be any suitable measure of variance or error. In some cases an uncertainty value is a standard deviation, standard error, calculated variance, p-value, or mean absolute deviation (MAD). In some cases an uncertainty value can be calculated according to a formula described herein.

Any suitable procedure can be utilized for processing data sets described herein. Non-limiting examples of procedures suitable for use for processing data sets include filtering, normalizing, weighting, monitoring peak heights, monitoring peak areas, monitoring peak edges, peak level analysis, peak width analysis, peak edge location analysis, peak lateral tolerances, determining area ratios, mathematical processing of data, statistical processing of data, application of statistical algorithms, analysis with fixed variables, analysis with optimized variables, plotting data to identify patterns or trends for additional processing, the like and combinations of the foregoing. In some cases, data sets are processed based on various features (e.g., GC content, redundant mapped reads, centromere regions, telomere regions, the like and combinations thereof) and/or variables (e.g., subject gender, subject age, subject ploidy, fetal gender, maternal age, maternal ploidy, percent contribution of fetal nucleic acid, the like or combinations thereof). In certain cases, processing data sets as described herein can reduce the complexity and/or dimensionality of large and/or complex data sets. A non-limiting example of a complex data set includes sequence read data generated from one or more test subjects and a plurality of reference subjects of different ages and ethnic backgrounds. In some cases, data sets can include from thousands to millions of sequence reads for each test and/or reference subject.

Data processing can be performed in any number of steps, in certain cases. For example, data may be processed using only a single processing procedure in some cases, and in certain cases data may be processed using 1 or more, 5 or more, 10 or more or 20 or more processing steps (e.g., 1 or more processing steps, 2 or more processing steps, 3 or more processing steps, 4 or more processing steps, 5 or more processing steps, 6 or more processing steps, 7 or more processing steps, 8 or more processing steps, 9 or more processing steps, 10 or more processing steps, 11 or more processing steps, 12 or more processing steps, 13 or more processing steps, 14 or more processing steps, 15 or more processing steps, 16 or more processing steps, 17 or more processing steps, 18 or more processing steps, 19 or more processing steps, or 20 or more processing steps). In some cases, processing steps may be the same step repeated two or more times (e.g., filtering two or more times, normalizing two or more times), and in certain cases, processing steps may be two or more different processing steps (e.g., filtering, normalizing; normalizing, monitoring peak heights and edges; filtering, normalizing, normalizing to a reference, statistical manipulation to determine p-values, and the like), carried out simultaneously or sequentially. In some cases, any suitable number and/or combination of the same or different processing steps can be utilized to process sequence read data to facilitate providing an outcome. In certain cases, processing data sets by the criteria described herein may reduce the complexity and/or dimensionality of a data set.

In some cases one or more processing steps can comprise one or more normalization steps. Normalization can be performed by a suitable method described herein or known in the art. In certain cases, normalization comprises adjusting values measured on different scales to a notionally common scale. In certain cases, normalization comprises a sophisticated mathematical adjustment to bring probability distributions of adjusted values into alignment. In some cases normalization comprises aligning distributions to a normal distribution. In certain cases normalization comprises mathematical adjustments that allow comparison of corresponding normalized values for different datasets in a way that eliminates the effects of certain gross influences (e.g., error and anomalies). In certain cases normalization comprises scaling. Normalization sometimes comprises division of one or more data sets by a predetermined variable or formula. Normalization sometimes comprises subtraction of one or more data sets by a predetermined variable or formula. Non-limiting examples of normalization methods include portion-wise normalization, normalization by GC content, median count (median bin count, median portion count) normalization, linear and nonlinear least squares regression, LOESS, GC LOESS, LOWESS (locally weighted scatterplot smoothing), principal component normalization, repeat masking (RM), GC-normalization and repeat masking (GCRM), cQn and/or combinations thereof. In some cases, the determination of a presence or absence of a copy number alteration (e.g., an aneuploidy, a microduplication, a microdeletion) utilizes a normalization method (e.g., portion-wise normalization, normalization by GC content, median count (median bin count, median portion count) normalization, linear and nonlinear least squares regression, LOESS, GC LOESS, LOWESS (locally weighted scatterplot smoothing), principal component normalization, repeat masking (RM), GC-normalization and repeat masking (GCRM), cQn, a normalization method known in the art and/or a combination thereof). Described in greater detail hereafter are certain examples of normalization processes that can be utilized, such as LOESS normalization, principal component normalization, and hybrid normalization methods, for example. Aspects of certain normalization processes also are described, for example, in International Patent Application Publication No. WO2013/052913 and International Patent Application Publication No. WO2015/051163.

Any suitable number of normalizations can be used. In some cases, data sets can be normalized 1 or more, 5 or more, 10 or more or even 20 or more times. Data sets can be normalized to values (e.g., normalizing value) representative of any suitable feature or variable (e.g., sample data, reference data, or both). Non-limiting examples of types of data normalizations that can be used include normalizing raw count data for one or more selected test or reference portions to the total number of counts mapped to the chromosome or the entire genome on which the selected portion or sections are mapped; normalizing raw count data for one or more selected portions to a median reference count for one or more portions or the chromosome on which a selected portion is mapped; normalizing raw count data to previously normalized data or derivatives thereof; and normalizing previously normalized data to one or more other predetermined normalization variables. Normalizing a data set sometimes has the effect of isolating statistical error, depending on the feature or property selected as the predetermined normalization variable. Normalizing a data set sometimes also allows comparison of data characteristics of data having different scales, by bringing the data to a common scale (e.g., predetermined normalization variable). In some cases, one or more normalizations to a statistically derived value can be utilized to minimize data differences and diminish the importance of outlying data. Normalizing portions, or portions of a reference genome, with respect to a normalizing value sometimes is referred to as "portion-wise normalization."

In certain cases, a processing step can comprise one or more mathematical and/or statistical manipulations. Any suitable mathematical and/or statistical manipulation, alone or in combination, may be used to analyze and/or manipulate a data set described herein. Any suitable number of mathematical and/or statistical manipulations can be used. In some cases, a data set can be mathematically and/or statistically manipulated 1 or more, 5 or more, 10 or more or 20 or more times. Non-limiting examples of mathematical and statistical manipulations that can be used include addition, subtraction, multiplication, division, algebraic functions, least squares estimators, curve fitting, differential equations, rational polynomials, double polynomials, orthogonal polynomials, z-scores, p-values, chi values, phi values, analysis of peak levels, determination of peak edge locations, calculation of peak area ratios, analysis of median chromosomal level, calculation of mean absolute deviation, sum of squared residuals, mean, standard deviation, standard error, the like or combinations thereof. A mathematical and/or statistical manipulation can be performed on all or a portion of sequence read data, or processed products thereof. Non-limiting examples of data set variables or features that can be statistically manipulated include raw counts, filtered counts, normalized counts, peak heights, peak widths, peak areas, peak edges, lateral tolerances, P-values, median levels, mean levels, count distribution within a genomic region, relative representation of nucleic acid species, the like or combinations thereof.

In some cases, a processing step can comprise the use of one or more statistical algorithms. Any suitable statistical algorithm, alone or in combination, may be used to analyze and/or manipulate a data set described herein. Any suitable number of statistical algorithms can be used. In some cases, a data set can be analyzed using 1 or more, 5 or more, 10 or more or 20 or more statistical algorithms. Non-limiting examples of statistical algorithms suitable for use with methods described herein include principal component analysis, decision trees, counternulls, multiple comparisons, omnibus test, Behrens-Fisher problem, bootstrapping, Fisher's method for combining independent tests of significance, null hypothesis, type I error, type II error, exact test, one-sample Z test, two-sample Z test, one-sample t-test, paired t-test, two-sample pooled t-test having equal variances, two-sample unpooled t-test having unequal variances, one-proportion z-test, two-proportion z-test pooled, two-proportion z-test unpooled, one-sample chi-square test, two-sample F test for equality of variances, confidence interval, credible interval, significance, meta analysis, simple linear regression, robust linear regression, the like or combinations of the foregoing. Non-limiting examples of data set variables or features that can be analyzed using statistical algorithms include raw counts, filtered counts, normalized counts, peak heights, peak widths, peak edges, lateral tolerances, P-values, median levels, mean levels, count distribution within a genomic region, relative representation of nucleic acid species, the like or combinations thereof.

In certain cases, a data set can be analyzed by utilizing multiple (e.g., 2 or more) statistical algorithms (e.g., least squares regression, principal component analysis, linear discriminant analysis, quadratic discriminant analysis, bagging, neural networks, support vector machine models, random forests, classification tree models, K-nearest neighbors, logistic regression and/or smoothing) and/or mathematical and/or statistical manipulations (e.g., referred to herein as manipulations). The use of multiple manipulations can generate an N-dimensional space that can be used to provide an outcome, in some cases. In certain cases, analysis of a data set by utilizing multiple manipulations can reduce the complexity and/or dimensionality of the data set. For example, the use of multiple manipulations on a reference data set can generate an N-dimensional space (e.g., probability plot) that can be used to represent the presence or absence of a genetic variation and/or copy number alteration, depending on the status of the reference samples (e.g., positive or negative for a selected copy number alteration). Analysis of test samples using a substantially similar set of manipulations can be used to generate an N-dimensional point for each of the test samples. The complexity and/or dimensionality of a test subject data set sometimes is reduced to a single value or N-dimensional point that can be readily compared to the N-dimensional space generated from the reference data. Test sample data that fall within the N-dimensional space populated by the reference subject data are indicative of a genetic status substantially similar to that of the reference subjects. Test sample data that fall outside of the N-dimensional space populated by the reference subject data are indicative of a genetic status substantially dissimilar to that of the reference subjects. In some cases, references are euploid or do not otherwise have a genetic variation and/or copy number alteration and/or medical condition.

After data sets have been counted, optionally filtered, normalized, and optionally weighted the processed data sets can be further manipulated by one or more filtering and/or normalizing and/or weighting procedures, in some cases. A data set that has been further manipulated by one or more filtering and/or normalizing and/or weighting procedures can be used to generate a profile, in certain cases. The one or more filtering and/or normalizing and/or weighting procedures sometimes can reduce data set complexity and/or dimensionality, in some cases. An outcome can be provided based on a data set of reduced complexity and/or dimensionality. In some cases, a profile plot of processed data further manipulated by weighting, for example, is generated to facilitate classification and/or providing an outcome. An outcome can be provided based on a profile plot of weighted data, for example.

Filtering or weighting of portions can be performed at one or more suitable points in an analysis. For example, portions may be filtered or weighted before or after sequence reads are mapped to portions of a reference genome. Portions may be filtered or weighted before or after an experimental bias for individual genome portions is determined in some cases. In certain cases, portions may be filtered or weighted before or after levels are calculated.

After data sets have been counted, optionally filtered, normalized, and optionally weighted, the processed data sets can be manipulated by one or more mathematical and/or statistical (e.g., statistical functions or statistical algorithm) manipulations, in some cases. In certain cases, processed data sets can be further manipulated by calculating Z-scores for one or more selected portions, chromosomes, or portions of chromosomes. In some cases, processed data sets can be further manipulated by calculating P-values. In certain cases, mathematical and/or statistical manipulations include one or more assumptions pertaining to ploidy and/or fraction of a minority species (e.g., fetal fraction). In some cases, a profile plot of processed data further manipulated by one or more statistical and/or mathematical manipulations is generated to facilitate classification and/or providing an outcome. An outcome can be provided based on a profile plot of statistically and/or mathematically manipulated data. An outcome provided based on a profile plot of statistically and/or mathematically manipulated data often includes one or more assumptions pertaining to ploidy and/or fraction of a minority species (e.g., fetal fraction). In some cases, analysis and processing of data can include the use of one or more assumptions. A suitable number or type of assumptions can be utilized to analyze or process a data set. Non-limiting examples of assumptions that can be used for data processing and/or analysis include subject ploidy, cancer cell contribution, maternal ploidy, fetal contribution, prevalence of certain sequences in a reference population, ethnic background, prevalence of a selected medical condition in related family members, parallelism between raw count profiles from different patients and/or runs after GC-normalization and repeat masking (e.g., GCRM), identical matches represent PCR artifacts (e.g., identical base position), assumptions inherent in a nucleic acid quantification assay (e.g., fetal quantifier assay (FQA)), assumptions regarding twins (e.g., if 2 twins and only 1 is affected the effective fetal fraction is only 50% of the total measured fetal fraction (similarly for triplets, quadruplets and the like)), cell free DNA (e.g., cfDNA) uniformly covers the entire genome, the like and combinations thereof.

In those instances where the quality and/or depth of mapped sequence reads does not permit an outcome prediction of the presence or absence of a genetic variation and/or copy number alteration at a desired confidence level (e.g., 95% or higher confidence level), based on the normalized count profiles, one or more additional mathematical manipulation algorithms and/or statistical prediction algorithms, can be utilized to generate additional numerical values useful for data analysis and/or providing an outcome. The term "normalized count profile" as used herein refers to a profile generated using normalized counts. Examples of methods that can be used to generate normalized counts and normalized count profiles are described herein. As noted, mapped sequence reads that have been counted can be normalized with respect to test sample counts or reference sample counts. In some cases, a normalized count profile can be presented as a plot.

Described in greater detail hereafter are non-limiting examples of processing steps and normalization methods that can be utilized, such as normalizing to a window (static or sliding), weighting, determining bias relationship, LOESS normalization, principal component normalization, hybrid normalization, generating a profile and performing a comparison.

### Normalizing to a window (static or sliding)

In certain cases, a processing step comprises normalizing to a static window, and in some cases, a processing step comprises normalizing to a moving or sliding window. The term "window" as used herein refers to one or more portions chosen for analysis, and sometimes is used as a reference for comparison (e.g., used for normalization and/or other mathematical or statistical manipulation). The term "normalizing to a static window" as used herein refers to a normalization process using one or more portions selected for comparison between a test subject and reference subject data set. In some cases the selected portions are utilized to generate a profile. A static window generally includes a predetermined set of portions that do not change during manipulations and/or analysis. The terms "normalizing to a moving window" and "normalizing to a sliding window" as used herein refer to normalizations performed to portions localized to the genomic region (e.g., immediate surrounding portions, adjacent portion or sections, and the like) of a selected test portion, where one or more selected test portions are normalized to portions immediately surrounding the selected test portion. In certain cases, the selected portions are utilized to generate a profile. A sliding or moving window normalization often includes repeatedly moving or sliding to an adjacent test portion, and normalizing the newly selected test portion to portions immediately surrounding or adjacent to the newly selected test portion, where adjacent windows have one or more portions in common. In certain cases, a plurality of selected test portions and/or chromosomes can be analyzed by a sliding window process.

In some cases, normalizing to a sliding or moving window can generate one or more values, where each value represents normalization to a different set of reference portions selected from different regions of a genome (e.g., chromosome). In certain cases, the one or more values generated are cumulative sums (e.g., a numerical estimate of the integral of the normalized count profile over the selected portion, domain (e.g., part of chromosome), or chromosome). The values generated by the sliding or moving window process can be used to generate a profile and facilitate arriving at an outcome. In some cases, cumulative sums of one or more portions can be displayed as a function of genomic position. Moving or sliding window analysis sometimes is used to analyze a genome for the presence or absence of microdeletions and/or microduplications. In certain cases, displaying cumulative sums of one or more portions is used to identify the presence or absence of regions of copy number alteration (e.g., microdeletion, microduplication).

### Weighting

In some cases, a processing step comprises a weighting. The terms "weighted," "weighting" or "weight function" or grammatical derivatives or equivalents thereof, as used herein, refer to a mathematical manipulation of a portion or all of a data set sometimes utilized to alter the influence of certain data set features or variables with respect to other data set features or variables (e.g., increase or decrease the significance and/or contribution of data contained in one or more portions or portions of a reference genome, based on the quality or usefulness of the data in the selected portion or portions of a reference genome). A weighting function can be used to increase the influence of data with a relatively small measurement variance, and/or to decrease the influence of data with a relatively large measurement variance, in some cases. For example, portions of a reference genome with under represented or low quality sequence data can be "down weighted" to minimize the influence on a data set, whereas selected portions of a reference genome can be "up weighted" to increase the influence on a data set. A non-limiting example of a weighting function is [1 / (standard deviation)²]. Weighting portions sometimes removes portion dependencies. In some cases one or more portions are weighted by an eigen function (e.g., an eigenfunction). In some cases an eigen function comprises replacing portions with orthogonal eigen-portions. A weighting step sometimes is performed in a manner substantially similar to a normalizing step. In some cases, a data set is adjusted (e.g., divided, multiplied, added, subtracted) by a predetermined variable (e.g., weighting variable). In some cases, a data set is divided by a predetermined variable (e.g., weighting variable). A predetermined variable (e.g., minimized target function, Phi) often is selected to weigh different parts of a data set differently (e.g., increase the influence of certain data types while decreasing the influence of other data types).

### Bias relationships

In some cases, a processing step comprises determining a bias relationship. For example, one or more relationships may be generated between local genome bias estimates and bias frequencies. The term "relationship" as use herein refers to a mathematical and/or a graphical relationship between two or more variables or values. A relationship can be generated by a suitable mathematical and/or graphical process. Non-limiting examples of a relationship include a mathematical and/or graphical representation of a function, a correlation, a distribution, a linear or non-linear equation, a line, a regression, a fitted regression, the like or a combination thereof. Sometimes a relationship comprises a fitted relationship. In some cases a fitted relationship comprises a fitted regression. Sometimes a relationship comprises two or more variables or values that are weighted. In some cases a relationship comprise a fitted regression where one or more variables or values of the relationship a weighted. Sometimes a regression is fitted in a weighted fashion. Sometimes a regression is fitted without weighting. In certain cases, generating a relationship comprises plotting or graphing.

In certain cases, a relationship is generated between GC densities and GC density frequencies. In some cases generating a relationship between (i) GC densities and (ii) GC density frequencies for a sample provides a sample GC density relationship. In some cases generating a relationship between (i) GC densities and (ii) GC density frequencies for a reference provides a reference GC density relationship. In some cases, where local genome bias estimates are GC densities, a sample bias relationship is a sample GC density relationship and a reference bias relationship is a reference GC density relationship. GC densities of a reference GC density relationship and/or a sample GC density relationship are often representations (e.g., mathematical or quantitative representation) of local GC content.

In some cases a relationship between local genome bias estimates and bias frequencies comprises a distribution. In some cases a relationship between local genome bias estimates and bias frequencies comprises a fitted relationship (e.g., a fitted regression). In some cases a relationship between local genome bias estimates and bias frequencies comprises a fitted linear or non-linear regression (e.g., a polynomial regression). In certain cases a relationship between local genome bias estimates and bias frequencies comprises a weighted relationship where local genome bias estimates and/or bias frequencies are weighted by a suitable process. In some cases a weighted fitted relationship (e.g., a weighted fitting) can be obtained by a process comprising a quantile regression, parameterized distributions or an empirical distribution with interpolation. In certain cases a relationship between local genome bias estimates and bias frequencies for a test sample, a reference or part thereof, comprises a polynomial regression where local genome bias estimates are weighted. In some cases a weighed fitted model comprises weighting values of a distribution. Values of a distribution can be weighted by a suitable process. In some cases, values located near tails of a distribution are provided less weight than values closer to the median of the distribution. For example, for a distribution between local genome bias estimates (e.g., GC densities) and bias frequencies (e.g., GC density frequencies), a weight is determined according to the bias frequency for a given local genome bias estimate, where local genome bias estimates comprising bias frequencies closer to the mean of a distribution are provided greater weight than local genome bias estimates comprising bias frequencies further from the mean.

In some cases, a processing step comprises normalizing sequence read counts by comparing local genome bias estimates of sequence reads of a test sample to local genome bias estimates of a reference (e.g., a reference genome, or part thereof). In some cases, counts of sequence reads are normalized by comparing bias frequencies of local genome bias estimates of a test sample to bias frequencies of local genome bias estimates of a reference. In some cases counts of sequence reads are normalized by comparing a sample bias relationship and a reference bias relationship, thereby generating a comparison.

Counts of sequence reads may be normalized according to a comparison of two or more relationships. In certain cases two or more relationships are compared thereby providing a comparison that is used for reducing local bias in sequence reads (e.g., normalizing counts). Two or more relationships can be compared by a suitable method. In some cases a comparison comprises adding, subtracting, multiplying and/or dividing a first relationship from a second relationship. In certain cases comparing two or more relationships comprises a use of a suitable linear regression and/or a non-linear regression. In certain cases comparing two or more relationships comprises a suitable polynomial regression (e.g., a 3^{rd} order polynomial regression). In some cases a comparison comprises adding, subtracting, multiplying and/or dividing a first regression from a second regression. In some cases two or more relationships are compared by a process comprising an inferential framework of multiple regressions. In some cases two or more relationships are compared by a process comprising a suitable multivariate analysis. In some cases two or more relationships are compared by a process comprising a basis function (e.g., a blending function, e.g., polynomial bases, Fourier bases, or the like), splines, a radial basis function and/or wavelets.

In certain cases a distribution of local genome bias estimates comprising bias frequencies for a test sample and a reference is compared by a process comprising a polynomial regression where local genome bias estimates are weighted. In some cases a polynomial regression is generated between (i) ratios, each of which ratios comprises bias frequencies of local genome bias estimates of a reference and bias frequencies of local genome bias estimates of a sample and (ii) local genome bias estimates. In some cases a polynomial regression is generated between (i) a ratio of bias frequencies of local genome bias estimates of a reference to bias frequencies of local genome bias estimates of a sample and (ii) local genome bias estimates. In some cases a comparison of a distribution of local genome bias estimates for reads of a test sample and a reference comprises determining a log ratio (e.g., a log2 ratio) of bias frequencies of local genome bias estimates for the reference and the sample. In some cases a comparison of a distribution of local genome bias estimates comprises dividing a log ratio (e.g., a log2 ratio) of bias frequencies of local genome bias estimates for the reference by a log ratio (e.g., a log2 ratio) of bias frequencies of local genome bias estimates for the sample.

Normalizing counts according to a comparison typically adjusts some counts and not others. Normalizing counts sometimes adjusts all counts and sometimes does not adjust any counts of sequence reads. A count for a sequence read sometimes is normalized by a process that comprises determining a weighting factor and sometimes the process does not include directly generating and utilizing a weighting factor. Normalizing counts according to a comparison sometimes comprises determining a weighting factor for each count of a sequence read. A weighting factor is often specific to a sequence read and is applied to a count of a specific sequence read. A weighting factor is often determined according to a comparison of two or more bias relationships (e.g., a sample bias relationship compared to a reference bias relationship). A normalized count is often determined by adjusting a count value according to a weighting factor. Adjusting a count according to a weighting factor sometimes includes adding, subtracting, multiplying and/or dividing a count for a sequence read by a weighting factor. A weighting factor and/or a normalized count sometimes are determined from a regression (e.g., a regression line). A normalized count is sometimes obtained directly from a regression line (e.g., a fitted regression line) resulting from a comparison between bias frequencies of local genome bias estimates of a reference (e.g., a reference genome) and a test sample. In some cases each count of a read of a sample is provided a normalized count value according to a comparison of (i) bias frequencies of a local genome bias estimates of reads compared to (ii) bias frequencies of a local genome bias estimates of a reference. In certain cases, counts of sequence reads obtained for a sample are normalized and bias in the sequence reads is reduced.

### LOESS normalization

In some cases, a processing step comprises a LOESS normalization. LOESS is a regression modeling method known in the art that combines multiple regression models in a k-nearest-neighbor-based meta-model. LOESS is sometimes referred to as a locally weighted polynomial regression. GC LOESS, in some cases, applies an LOESS model to the relationship between fragment count (e.g., sequence reads, counts) and GC composition for portions of a reference genome. Plotting a smooth curve through a set of data points using LOESS is sometimes called an LOESS curve, particularly when each smoothed value is given by a weighted quadratic least squares regression over the span of values of the y-axis scattergram criterion variable. For each point in a data set, the LOESS method fits a low-degree polynomial to a subset of the data, with explanatory variable values near the point whose response is being estimated. The polynomial is fitted using weighted least squares, giving more weight to points near the point whose response is being estimated and less weight to points further away. The value of the regression function for a point is then obtained by evaluating the local polynomial using the explanatory variable values for that data point. The LOESS fit is sometimes considered complete after regression function values have been computed for each of the data points. Many of the details of this method, such as the degree of the polynomial model and the weights, are flexible.

### Principal component analysis

In some cases, a processing step comprises a principal component analysis (PCA). In some cases, sequence read counts (e.g., sequence read counts of a test sample) is adjusted according to a principal component analysis (PCA). In some cases a read density profile (e.g., a read density profile of a test sample) is adjusted according to a principal component analysis (PCA). A read density profile of one or more reference samples and/or a read density profile of a test subject can be adjusted according to a PCA. Removing bias from a read density profile by a PCA related process is sometimes referred to herein as adjusting a profile. A PCA can be performed by a suitable PCA method, or a variation thereof. Non-limiting examples of a PCA method include a canonical correlation analysis (CCA), a Karhunen-Loève transform (KLT), a Hotelling transform, a proper orthogonal decomposition (POD), a singular value decomposition (SVD) of X, an eigenvalue decomposition (EVD) of XTX, a factor analysis, an Eckart-Young theorem, a Schmidt-Mirsky theorem, empirical orthogonal functions (EOF), an empirical eigenfunction decomposition, an empirical component analysis, quasiharmonic modes, a spectral decomposition, an empirical modal analysis, the like, variations or combinations thereof. A PCA often identifies and/or adjusts for one or more biases in a read density profile. A bias identified and/or adjusted for by a PCA is sometimes referred to herein as a principal component. In some cases one or more biases can be removed by adjusting a read density profile according to one or more principal component using a suitable method. A read density profile can be adjusted by adding, subtracting, multiplying and/or dividing one or more principal components from a read density profile. In some cases, one or more biases can be removed from a read density profile by subtracting one or more principal components from a read density profile. Although bias in a read density profile is often identified and/or quantitated by a PCA of a profile, principal components are often subtracted from a profile at the level of read densities. A PCA often identifies one or more principal components. In some cases a PCA identifies a 1^{st}, 2^{nd}, 3^{rd}, 4^{th}, 5^{th}, 6^{th}, 7^{th}, 8^{th}, 9^{th}, and a 10^{th} or more principal components. In certain cases, 1, 2, 3, 4, 5, 6, 7, 8, 9, 10 or more principal components are used to adjust a profile. In certain cases, 5 principal components are used to adjust a profile. Often, principal components are used to adjust a profile in the order of appearance in a PCA. For example, where three principal components are subtracted from a read density profile, a 1^{st}, 2^{nd} and 3^{rd} principal component are used. Sometimes a bias identified by a principal component comprises a feature of a profile that is not used to adjust a profile. For example, a PCA may identify a copy number alteration (e.g., an aneuploidy, microduplication, microdeletion, deletion, translocation, insertion) and/or a gender difference as a principal component. Thus, in some cases, one or more principal components are not used to adjust a profile. For example, sometimes a 1^{st}, 2^{nd} and 4^{th} principal component are used to adjust a profile where a 3^{rd} principal component is not used to adjust a profile.

A principal component can be obtained from a PCA using any suitable sample or reference. In some cases principal components are obtained from a test sample (e.g., a test subject). In some cases principal components are obtained from one or more references (e.g., reference samples, reference sequences, a reference set). In certain instances, a PCA is performed on a median read density profile obtained from a training set comprising multiple samples resulting in the identification of a 1^{st} principal component and a 2^{nd} principal component. In some cases, principal components are obtained from a set of subjects devoid of a copy number alteration in question. In some cases, principal components are obtained from a set of known euploids. Principal component are often identified according to a PCA performed using one or more read density profiles of a reference (e.g., a training set). One or more principal components obtained from a reference are often subtracted from a read density profile of a test subject thereby providing an adjusted profile.

### Hybrid normalization

In some cases, a processing step comprises a hybrid normalization method. A hybrid normalization method may reduce bias (e.g., GC bias), in certain instances. A hybrid normalization, in some cases, comprises (i) an analysis of a relationship of two variables (e.g., counts and GC content) and (ii) selection and application of a normalization method according to the analysis. A hybrid normalization, in certain cases, comprises (i) a regression (e.g., a regression analysis) and (ii) selection and application of a normalization method according to the regression. In some cases counts obtained for a first sample (e.g., a first set of samples) are normalized by a different method than counts obtained from another sample (e.g., a second set of samples). In some cases counts obtained for a first sample (e.g., a first set of samples) are normalized by a first normalization method and counts obtained from a second sample (e.g., a second set of samples) are normalized by a second normalization method. For example, in certain cases a first normalization method comprises use of a linear regression and a second normalization method comprises use of a non-linear regression (e.g., a LOESS, GC-LOESS, LOWESS regression, LOESS smoothing).

In some cases a hybrid normalization method is used to normalize sequence reads mapped to portions of a genome or chromosome (e.g., counts, mapped counts, mapped reads). In certain cases raw counts are normalized and in some cases adjusted, weighted, filtered or previously normalized counts are normalized by a hybrid normalization method. In certain cases, levels or Z-scores are normalized. In some cases counts mapped to selected portions of a genome or chromosome are normalized by a hybrid normalization approach. Counts can refer to a suitable measure of sequence reads mapped to portions of a genome, non-limiting examples of which include raw counts (e.g., unprocessed counts), normalized counts (e.g., normalized by LOESS, principal component, or a suitable method), portion levels (e.g., average levels, mean levels, median levels, or the like), Z-scores, the like, or combinations thereof. The counts can be raw counts or processed counts from one or more samples (e.g., a test sample, a sample from a pregnant female). In some cases counts are obtained from one or more samples obtained from one or more subjects.

In some cases a normalization method (e.g., the type of normalization method) is selected according to a regression (e.g., a regression analysis) and/or a correlation coefficient. A regression analysis refers to a statistical technique for estimating a relationship among variables (e.g., counts and GC content). In some cases a regression is generated according to counts and a measure of GC content for each portion of multiple portions of a reference genome. A suitable measure of GC content can be used, non-limiting examples of which include a measure of guanine, cytosine, adenine, thymine, purine (GC), or pyrimidine (AT or ATU) content, melting temperature (Tₘ) (e.g., denaturation temperature, annealing temperature, hybridization temperature), a measure of free energy, the like or combinations thereof. A measure of guanine (G), cytosine (C), adenine (A), thymine (T), purine (GC), or pyrimidine (AT or ATU) content can be expressed as a ratio or a percentage. In some cases any suitable ratio or percentage is used, non-limiting examples of which include GC/AT, GC/total nucleotide, GC/A, GC/T, AT/total nucleotide, AT/GC, AT/G, AT/C, G/A, C/A, G/T, G/A, G/AT, C/T, the like or combinations thereof. In some cases a measure of GC content is a ratio or percentage of GC to total nucleotide content. In some cases a measure of GC content is a ratio or percentage of GC to total nucleotide content for sequence reads mapped to a portion of reference genome. In certain cases the GC content is determined according to and/or from sequence reads mapped to each portion of a reference genome and the sequence reads are obtained from a sample. In some cases a measure of GC content is not determined according to and/or from sequence reads. In certain cases, a measure of GC content is determined for one or more samples obtained from one or more subjects.

In some cases generating a regression comprises generating a regression analysis or a correlation analysis. A suitable regression can be used, non-limiting examples of which include a regression analysis, (e.g., a linear regression analysis), a goodness of fit analysis, a Pearson's correlation analysis, a rank correlation, a fraction of variance unexplained, Nash-Sutcliffe model efficiency analysis, regression model validation, proportional reduction in loss, root mean square deviation, the like or a combination thereof. In some cases a regression line is generated. In certain cases generating a regression comprises generating a linear regression. In certain cases generating a regression comprises generating a non-linear regression (e.g., an LOESS regression, an LOWESS regression).

In some cases a regression determines the presence or absence of a correlation (e.g., a linear correlation), for example between counts and a measure of GC content. In some cases a regression (e.g., a linear regression) is generated and a correlation coefficient is determined. In some cases a suitable correlation coefficient is determined, non-limiting examples of which include a coefficient of determination, an R² value, a Pearson's correlation coefficient, or the like.

In some cases goodness of fit is determined for a regression (e.g., a regression analysis, a linear regression). Goodness of fit sometimes is determined by visual or mathematical analysis. An assessment sometimes includes determining whether the goodness of fit is greater for a non-linear regression or for a linear regression. In some cases a correlation coefficient is a measure of a goodness of fit. In some cases an assessment of a goodness of fit for a regression is determined according to a correlation coefficient and/or a correlation coefficient cutoff value. In some cases an assessment of a goodness of fit comprises comparing a correlation coefficient to a correlation coefficient cutoff value. In some cases an assessment of a goodness of fit for a regression is indicative of a linear regression. For example, in certain cases, a goodness of fit is greater for a linear regression than for a non-linear regression and the assessment of the goodness of fit is indicative of a linear regression. In some cases an assessment is indicative of a linear regression and a linear regression is used to normalized the counts. In some cases an assessment of a goodness of fit for a regression is indicative of a non-linear regression. For example, in certain cases, a goodness of fit is greater for a non-linear regression than for a linear regression and the assessment of the goodness of fit is indicative of a non-linear regression. In some cases an assessment is indicative of a non-linear regression and a non-linear regression is used to normalized the counts.

In some cases an assessment of a goodness of fit is indicative of a linear regression when a correlation coefficient is equal to or greater than a correlation coefficient cutoff. In some cases an assessment of a goodness of fit is indicative of a non-linear regression when a correlation coefficient is less than a correlation coefficient cutoff. In some cases a correlation coefficient cutoff is pre-determined. In some cases a correlation coefficient cut-off is about 0.5 or greater, about 0.55 or greater, about 0.6 or greater, about 0.65 or greater, about 0.7 or greater, about 0.75 or greater, about 0.8 or greater or about 0.85 or greater.

In some cases a specific type of regression is selected (e.g., a linear or non-linear regression) and, after the regression is generated, counts are normalized by subtracting the regression from the counts. In some cases subtracting a regression from the counts provides normalized counts with reduced bias (e.g., GC bias). In some cases a linear regression is subtracted from the counts. In some cases a non-linear regression (e.g., a LOESS, GC-LOESS, LOWESS regression) is subtracted from the counts. Any suitable method can be used to subtract a regression line from the counts. For example, if counts x are derived from portion i (e.g., a portion i) comprising a GC content of 0.5 and a regression line determines counts y at a GC content of 0.5, then x-y = normalized counts for portion i. In some cases counts are normalized prior to and/or after subtracting a regression. In some cases, counts normalized by a hybrid normalization approach are used to generate levels, Z-scores, levels and/or profiles of a genome or a part thereof. In certain cases, counts normalized by a hybrid normalization approach are analyzed by methods described herein to determine the presence or absence of a genetic variation (e.g., copy number alteration).

In some cases a hybrid normalization method comprises filtering or weighting one or more portions before or after normalization. A suitable method of filtering portions, including methods of filtering portions (e.g., portions of a reference genome) described herein can be used. In some cases, portions (e.g., portions of a reference genome) are filtered prior to applying a hybrid normalization method. In some cases, only counts of sequencing reads mapped to selected portions (e.g., portions selected according to count variability) are normalized by a hybrid normalization. In some cases counts of sequencing reads mapped to filtered portions of a reference genome (e.g., portions filtered according to count variability) are removed prior to utilizing a hybrid normalization method. In some cases a hybrid normalization method comprises selecting or filtering portions (e.g., portions of a reference genome) according to a suitable method (e.g., a method described herein). In some cases a hybrid normalization method comprises selecting or filtering portions (e.g., portions of a reference genome) according to an uncertainty value for counts mapped to each of the portions for multiple test samples. In some cases a hybrid normalization method comprises selecting or filtering portions (e.g., portions of a reference genome) according to count variability. In some cases a hybrid normalization method comprises selecting or filtering portions (e.g., portions of a reference genome) according to GC content, repetitive elements, repetitive sequences, introns, exons, the like or a combination thereof.

### Profiles

In some cases, a processing step comprises generating one or more profiles (e.g., profile plot) from various aspects of a data set or derivation thereof (e.g., product of one or more mathematical and/or statistical data processing steps known in the art and/or described herein). The term "profile" as used herein refers to a product of a mathematical and/or statistical manipulation of data that can facilitate identification of patterns and/or correlations in large quantities of data. A "profile" often includes values resulting from one or more manipulations of data or data sets, based on one or more criteria. A profile often includes multiple data points. Any suitable number of data points may be included in a profile depending on the nature and/or complexity of a data set. In certain cases, profiles may include 2 or more data points, 3 or more data points, 5 or more data points, 10 or more data points, 24 or more data points, 25 or more data points, 50 or more data points, 100 or more data points, 500 or more data points, 1000 or more data points, 5000 or more data points, 10,000 or more data points, or 100,000 or more data points.

In some cases, a profile is representative of the entirety of a data set, and in certain cases, a profile is representative of a part or subset of a data set. That is, a profile sometimes includes or is generated from data points representative of data that has not been filtered to remove any data, and sometimes a profile includes or is generated from data points representative of data that has been filtered to remove unwanted data. In some cases, a data point in a profile represents the results of data manipulation for a portion. In certain cases, a data point in a profile includes results of data manipulation for groups of portions. In some cases, groups of portions may be adjacent to one another, and in certain cases, groups of portions may be from different parts of a chromosome or genome.

Data points in a profile derived from a data set can be representative of any suitable data categorization. Non-limiting examples of categories into which data can be grouped to generate profile data points include: portions based on size, portions based on sequence features (e.g., GC content, AT content, position on a chromosome (e.g., short arm, long arm, centromere, telomere), and the like), levels of expression, chromosome, the like or combinations thereof. In some cases, a profile may be generated from data points obtained from another profile (e.g., normalized data profile renormalized to a different normalizing value to generate a renormalized data profile). In certain cases, a profile generated from data points obtained from another profile reduces the number of data points and/or complexity of the data set. Reducing the number of data points and/or complexity of a data set often facilitates interpretation of data and/or facilitates providing an outcome.

A profile (e.g., a genomic profile, a chromosome profile, a profile of a part of a chromosome) often is a collection of normalized or non-normalized counts for two or more portions. A profile often includes at least one level, and often comprises two or more levels (e.g., a profile often has multiple levels). A level generally is for a set of portions having about the same counts or normalized counts. Levels are described in greater detail herein. In certain cases, a profile comprises one or more portions, which portions can be weighted, removed, filtered, normalized, adjusted, averaged, derived as a mean, added, subtracted, processed or transformed by any combination thereof. A profile often comprises normalized counts mapped to portions defining two or more levels, where the counts are further normalized according to one of the levels by a suitable method. Often counts of a profile (e.g., a profile level) are associated with an uncertainty value.

A profile comprising one or more levels is sometimes padded (e.g., hole padding). Padding (e.g., hole padding) refers to a process of identifying and adjusting levels in a profile that are due to copy number alterations (e.g., maternal microduplications or microdeletions). In some cases, levels are padded that are due to microduplications or microdeletions in a fetus. Microduplications or microdeletions in a profile can, in some cases, artificially raise or lower the overall level of a profile (e.g., a profile of a chromosome) leading to false positive or false negative determinations of a chromosome aneuploidy (e.g., a trisomy). In some cases, levels in a profile that are due to microduplications and/or deletions are identified and adjusted (e.g., padded and/or removed) by a process sometimes referred to as padding or hole padding.

A profile comprising one or more levels can include a first level and a second level. In some cases a first level is different (e.g., significantly different) than a second level. In some cases a first level comprises a first set of portions, a second level comprises a second set of portions and the first set of portions is not a subset of the second set of portions. In certain cases, a first set of portions is different than a second set of portions from which a first and second level are determined. In some cases a profile can have multiple first levels that are different (e.g., significantly different, e.g., have a significantly different value) than a second level within the profile. In some cases a profile comprises one or more first levels that are significantly different than a second level within the profile and one or more of the first levels are adjusted. In some cases a first level within a profile is removed from the profile or adjusted (e.g., padded). A profile can comprise multiple levels that include one or more first levels significantly different than one or more second levels and often the majority of levels in a profile are second levels, which second levels are about equal to one another. In some cases greater than 50%, greater than 60%, greater than 70%, greater than 80%, greater than 90% or greater than 95% of the levels in a profile are second levels.

A profile sometimes is displayed as a plot. For example, one or more levels representing counts (e.g., normalized counts) of portions can be plotted and visualized. Non-limiting examples of profile plots that can be generated include raw count (e.g., raw count profile or raw profile), normalized count, portion-weighted, z-score, p-value, area ratio versus fitted ploidy, median level versus ratio between fitted and measured minority species fraction, principal components, the like, or combinations thereof. Profile plots allow visualization of the manipulated data, in some cases. In certain cases, a profile plot can be utilized to provide an outcome (e.g., area ratio versus fitted ploidy, median level versus ratio between fitted and measured minority species fraction, principal components). The terms "raw count profile plot" or "raw profile plot" as used herein refer to a plot of counts in each portion in a region normalized to total counts in a region (e.g., genome, portion, chromosome, chromosome portions of a reference genome or a part of a chromosome). In some cases, a profile can be generated using a static window process, and in certain cases, a profile can be generated using a sliding window process.

A profile generated for a test subject sometimes is compared to a profile generated for one or more reference subjects, to facilitate interpretation of mathematical and/or statistical manipulations of a data set and/or to provide an outcome. In some cases, a profile is generated based on one or more starting assumptions, e.g., assumptions described herein. In certain cases, a test profile often centers around a predetermined value representative of the absence of a copy number alteration, and often deviates from a predetermined value in areas corresponding to the genomic location in which the copy number alteration is located in the test subject, if the test subject possessed the copy number alteration. In test subjects at risk for, or suffering from a medical condition associated with a copy number alteration, the numerical value for a selected portion is expected to vary significantly from the predetermined value for non-affected genomic locations. Depending on starting assumptions (e.g., fixed ploidy or optimized ploidy, fixed fetal fraction or optimized fetal fraction, or combinations thereof) the predetermined threshold or cutoff value or threshold range of values indicative of the presence or absence of a copy number alteration can vary while still providing an outcome useful for determining the presence or absence of a copy number alteration. In some cases, a profile is indicative of and/or representative of a phenotype.

In some cases, the use of one or more reference samples that are substantially free of a copy number alteration in question can be used to generate a reference count profile (e.g., a reference median count profile), which may result in a predetermined value representative of the absence of the copy number alteration, and often deviates from a predetermined value in areas corresponding to the genomic location in which the copy number alteration is located in the test subject, if the test subject possessed the copy number alteration. In test subjects at risk for, or suffering from a medical condition associated with a copy number alteration, the numerical value for the selected portion or sections is expected to vary significantly from the predetermined value for non-affected genomic locations. In certain cases, the use of one or more reference samples known to carry the copy number alteration in question can be used to generate a reference count profile (a reference median count profile), which may result in a predetermined value representative of the presence of the copy number alteration, and often deviates from a predetermined value in areas corresponding to the genomic location in which a test subject does not carry the copy number alteration. In test subjects not at risk for, or suffering from a medical condition associated with a copy number alteration, the numerical value for the selected portion or sections is expected to vary significantly from the predetermined value for affected genomic locations.

By way of a non-limiting example, normalized sample and/or reference count profiles can be obtained from raw sequence read data by (a) calculating reference median counts for selected chromosomes, portions or parts thereof from a set of references known not to carry a copy number alteration, (b) removal of uninformative portions from the reference sample raw counts (e.g., filtering); (c) normalizing the reference counts for all remaining portions of a reference genome to the total residual number of counts (e.g., sum of remaining counts after removal of uninformative portions of a reference genome) for the reference sample selected chromosome or selected genomic location, thereby generating a normalized reference subject profile; (d) removing the corresponding portions from the test subject sample; and (e) normalizing the remaining test subject counts for one or more selected genomic locations to the sum of the residual reference median counts for the chromosome or chromosomes containing the selected genomic locations, thereby generating a normalized test subject profile. In certain cases, an additional normalizing step with respect to the entire genome, reduced by the filtered portions in (b), can be included between (c) and (d).

In some cases a read density profile is determined. In some cases a read density profile comprises at least one read density, and often comprises two or more read densities (e.g., a read density profile often comprises multiple read densities). In some cases, a read density profile comprises a suitable quantitative value (e.g., a mean, a median, a Z-score, or the like). A read density profile often comprises values resulting from one or more read densities. A read density profile sometimes comprises values resulting from one or more manipulations of read densities based on one or more adjustments (e.g., normalizations). In some cases a read density profile comprises unmanipulated read densities. In some cases, one or more read density profiles are generated from various aspects of a data set comprising read densities, or a derivation thereof (e.g., product of one or more mathematical and/or statistical data processing steps known in the art and/or described herein). In certain cases, a read density profile comprises normalized read densities. In some cases a read density profile comprises adjusted read densities. In certain cases a read density profile comprises raw read densities (e.g., unmanipulated, not adjusted or normalized), normalized read densities, weighted read densities, read densities of filtered portions, z-scores of read densities, p-values of read densities, integral values of read densities (e.g., area under the curve), average, mean or median read densities, principal components, the like, or combinations thereof. Often read densities of a read density profile and/or a read density profile is associated with a measure of uncertainty (e.g., a MAD). In certain cases, a read density profile comprises a distribution of median read densities. In some cases a read density profile comprises a relationship (e.g., a fitted relationship, a regression, or the like) of a plurality of read densities. For example, sometimes a read density profile comprises a relationship between read densities (e.g., read densities value) and genomic locations (e.g., portions, portion locations). In some cases, a read density profile is generated using a static window process, and in certain cases, a read density profile is generated using a sliding window process. In some cases a read density profile is sometimes printed and/or displayed (e.g., displayed as a visual representation, e.g., a plot or a graph).

In some cases, a read density profile corresponds to a set of portions (e.g., a set of portions of a reference genome, a set of portions of a chromosome or a subset of portions of a part of a chromosome). In some cases a read density profile comprises read densities and/or counts associated with a collection (e.g., a set, a subset) of portions. In some cases, a read density profile is determined for read densities of portions that are contiguous. In some cases, contiguous portions comprise gaps comprising regions of a reference sequence and/or sequence reads that are not included in a density profile (e.g., portions removed by a filtering). Sometimes portions (e.g., a set of portions) that are contiguous represent neighboring regions of a genome or neighboring regions of a chromosome or gene. For example, two or more contiguous portions, when aligned by merging the portions end to end, can represent a sequence assembly of a DNA sequence longer than each portion. For example two or more contiguous portions can represent an intact genome, chromosome, gene, intron, exon or part thereof. Sometimes a read density profile is determined from a collection (e.g., a set, a subset) of contiguous portions and/or non-contiguous portions. In some cases, a read density profile comprises one or more portions, which portions can be weighted, removed, filtered, normalized, adjusted, averaged, derived as a mean, added, subtracted, processed or transformed by any combination thereof.

A read density profile is often determined for a sample and/or a reference (e.g., a reference sample). A read density profile is sometimes generated for an entire genome, one or more chromosomes, or for a part of a genome or a chromosome. In some cases, one or more read density profiles are determined for a genome or part thereof. In some cases, a read density profile is representative of the entirety of a set of read densities of a sample, and in certain cases, a read density profile is representative of a part or subset of read densities of a sample. That is, sometimes a read density profile comprises or is generated from read densities representative of data that has not been filtered to remove any data, and sometimes a read density profile includes or is generated from data points representative of data that has been filtered to remove unwanted data.

In some cases a read density profile is determined for a reference (e.g., a reference sample, a training set). A read density profile for a reference is sometimes referred to herein as a reference profile. In some cases a reference profile comprises a read densities obtained from one or more references (e.g., reference sequences, reference samples). In some cases a reference profile comprises read densities determined for one or more (e.g., a set of) known euploid samples. In some cases a reference profile comprises read densities of filtered portions. In some cases a reference profile comprises read densities adjusted according to the one or more principal components.

### Performing a comparison

In some cases, a processing step comprises preforming a comparison (e.g., comparing a test profile to a reference profile). Two or more data sets, two or more relationships and/or two or more profiles can be compared by a suitable method. Non-limiting examples of statistical methods suitable for comparing data sets, relationships and/or profiles include Behrens-Fisher approach, bootstrapping, Fisher's method for combining independent tests of significance, Neyman-Pearson testing, confirmatory data analysis, exploratory data analysis, exact test, F-test, Z-test, T-test, calculating and/or comparing a measure of uncertainty, a null hypothesis, counternulls and the like, a chi-square test, omnibus test, calculating and/or comparing level of significance (e.g., statistical significance), a meta analysis, a multivariate analysis, a regression, simple linear regression, robust linear regression, the like or combinations of the foregoing. In certain cases comparing two or more data sets, relationships and/or profiles comprises determining and/or comparing a measure of uncertainty. A "measure of uncertainty" as used herein refers to a measure of significance (e.g., statistical significance), a measure of error, a measure of variance, a measure of confidence, the like or a combination thereof. A measure of uncertainty can be a value (e.g., a threshold) or a range of values (e.g., an interval, a confidence interval, a Bayesian confidence interval, a threshold range). Non-limiting examples of a measure of uncertainty include p-values, a suitable measure of deviation (e.g., standard deviation, sigma, absolute deviation, mean absolute deviation, the like), a suitable measure of error (e.g., standard error, mean squared error, root mean squared error, the like), a suitable measure of variance, a suitable standard score (e.g., standard deviations, cumulative percentages, percentile equivalents, Z-scores, T-scores, R-scores, standard nine (stanine), percent in stanine, the like), the like or combinations thereof. In some cases determining the level of significance comprises determining a measure of uncertainty (e.g., a p-value). In certain cases, two or more data sets, relationships and/or profiles can be analyzed and/or compared by utilizing multiple (e.g., 2 or more) statistical methods (e.g., least squares regression, principal component analysis, linear discriminant analysis, quadratic discriminant analysis, bagging, neural networks, support vector machine models, random forests, classification tree models, K-nearest neighbors, logistic regression and/or loss smoothing) and/or any suitable mathematical and/or statistical manipulations (e.g., referred to herein as manipulations).

In some cases, a processing step comprises a comparison of two or more profiles (e.g., two or more read density profiles). Comparing profiles may comprise comparing profiles generated for a selected region of a genome. For example, a test profile may be compared to a reference profile where the test and reference profiles were determined for a region of a genome (e.g., a reference genome) that is substantially the same region. Comparing profiles sometimes comprises comparing two or more subsets of portions of a profile (e.g., a read density profile). A subset of portions of a profile may represent a region of a genome (e.g., a chromosome, or region thereof). A profile (e.g., a read density profile) can comprise any amount of subsets of portions. Sometimes a profile (e.g., a read density profile) comprises two or more, three or more, four or more, or five or more subsets. In certain cases, a profile (e.g., a read density profile) comprises two subsets of portions where each portion represents regions of a reference genome that are adjacent. In some cases, a test profile can be compared to a reference profile where the test profile and reference profile both comprise a first subset of portions and a second subset of portions where the first and second subsets represent different regions of a genome. Some subsets of portions of a profile may comprise copy number alterations and other subsets of portions are sometimes substantially free of copy number alterations. Sometimes all subsets of portions of a profile (e.g., a test profile) are substantially free of a copy number alteration. Sometimes all subsets of portions of a profile (e.g., a test profile) comprise a copy number alteration. In some cases a test profile can comprise a first subset of portions that comprise a copy number alteration and a second subset of portions that are substantially free of a copy number alteration.

In certain cases, comparing two or more profiles comprises determining and/or comparing a measure of uncertainty for two or more profiles. Profiles (e.g., read density profiles) and/or associated measures of uncertainty are sometimes compared to facilitate interpretation of mathematical and/or statistical manipulations of a data set and/or to provide an outcome. A profile (e.g., a read density profile) generated for a test subject sometimes is compared to a profile (e.g., a read density profile) generated for one or more references (e.g., reference samples, reference subjects, and the like). In some cases, an outcome is provided by comparing a profile (e.g., a read density profile) from a test subject to a profile (e.g., a read density profile) from a reference for a chromosome, portions or parts thereof, where a reference profile is obtained from a set of reference subjects known not to possess a copy number alteration (e.g., a reference). In some cases an outcome is provided by comparing a profile (e.g., a read density profile) from a test subject to a profile (e.g., a read density profile) from a reference for a chromosome, portions or parts thereof, where a reference profile is obtained from a set of reference subjects known to possess a specific copy number alteration (e.g., a chromosome aneuploidy, a microduplication, a microdeletion).

In certain cases, a profile (e.g., a read density profile) of a test subject is compared to a predetermined value representative of the absence of a copy number alteration, and sometimes deviates from a predetermined value at one or more genomic locations (e.g., portions) corresponding to a genomic location in which a copy number alteration is located. For example, in test subjects (e.g., subjects at risk for, or suffering from a medical condition associated with a copy number alteration), profiles are expected to differ significantly from profiles of a reference (e.g., a reference sequence, reference subject, reference set) for selected portions when a test subject comprises a copy number alteration in question. Profiles (e.g., read density profiles) of a test subject are often substantially the same as profiles (e.g., read density profiles) of a reference (e.g., a reference sequence, reference subject, reference set) for selected portions when a test subject does not comprise a copy number alteration in question. Profiles (e.g., read density profiles) may be compared to a predetermined threshold and/or threshold range. The term "threshold" as used herein refers to any number that is calculated using a qualifying data set and serves as a limit of diagnosis of a copy number alteration (e.g., an aneuploidy, a microduplication, a microdeletion, and the like). In certain cases a threshold is exceeded by results obtained by methods described herein and a subject is diagnosed with a copy number alteration. In some cases, a threshold value or range of values may be calculated by mathematically and/or statistically manipulating sequence read data (e.g., from a reference and/or subject). A predetermined threshold or threshold range of values indicative of the presence or absence of a copy number alteration can vary while still providing an outcome useful for determining the presence or absence of a copy number alteration. In certain cases, a profile (e.g., a read density profile) comprising normalized read densities and/or normalized counts is generated to facilitate classification and/or providing an outcome. An outcome can be provided based on a plot of a profile (e.g., a read density profile) comprising normalized counts (e.g., using a plot of such a read density profile).

### Decision Analysis

In some cases, a determination of an outcome (e.g., making a call) or a determination of the presence or absence of a copy number alteration (e.g., chromosome aneuploidy, microduplication, microdeletion) is made according to a decision analysis. Certain decision analysis features are described in International Patent Application Publication No. WO2014/190286. For example, a decision analysis sometimes comprises applying one or more methods that produce one or more results, an evaluation of the results, and a series of decisions based on the results, evaluations and/or the possible consequences of the decisions and terminating at some juncture of the process where a final decision is made. In some cases a decision analysis is a decision tree. A decision analysis, in some cases, comprises coordinated use of one or more processes (e.g., process steps, e.g., algorithms). A decision analysis can be performed by a person, a system, an apparatus, software (e.g., a module), a computer, a processor (e.g., a microprocessor), the like or a combination thereof. In some cases a decision analysis comprises a method of determining the presence or absence of a copy number alteration (e.g., chromosome aneuploidy, microduplication or microdeletion) with reduced false negative and reduced false positive determinations, compared to an instance in which no decision analysis is utilized (e.g., a determination is made directly from normalized counts). In some cases a decision analysis comprises determining the presence or absence of a condition associated with one or more copy number alterations.

In some cases a decision analysis comprises generating a profile for a genome or a region of a genome (e.g., a chromosome or part thereof). A profile can be generated by any suitable method, known or described herein. In some cases, a decision analysis comprises a segmenting process. Segmenting can modify and/or transform a profile thereby providing one or more decomposition renderings of a profile. A profile subjected to a segmenting process often is a profile of normalized counts mapped to portions in a reference genome or part thereof. As addressed herein, raw counts mapped to the portions can be normalized by one or more suitable normalization processes (e.g., LOESS, GC-LOESS, principal component normalization, or combination thereof) to generate a profile that is segmented as part of a decision analysis. A decomposition rendering of a profile is often a transformation of a profile. A decomposition rendering of a profile is sometimes a transformation of a profile into a representation of a genome, chromosome or part thereof.

In certain cases, a segmenting process utilized for the segmenting locates and identifies one or more levels within a profile that are different (e.g., substantially or significantly different) than one or more other levels within a profile. A level identified in a profile according to a segmenting process that is different than another level in the profile, and has edges that are different than another level in the profile, is referred to herein as a level for a discrete segment. A segmenting process can generate, from a profile of normalized counts or levels, a decomposition rendering in which one or more discrete segments can be identified. A discrete segment generally covers fewer portions than what is segmented (e.g., chromosome, chromosomes, autosomes).

In some cases, segmenting locates and identifies edges of discrete segments within a profile. In certain cases, one or both edges of one or more discrete segments are identified. For example, a segmentation process can identify the location (e.g., genomic coordinates, e.g., portion location) of the right and/or the left edges of a discrete segment in a profile. A discrete segment often comprises two edges. For example, a discrete segment can include a left edge and a right edge. In some cases, depending upon the representation or view, a left edge can be a 5'-edge and a right edge can be a 3'-edge of a nucleic acid segment in a profile. In some cases, a left edge can be a 3'-edge and a right edge can be a 5'-edge of a nucleic acid segment in a profile. Often the edges of a profile are known prior to segmentation and therefore, in some cases, the edges of a profile determine which edge of a level is a 5'-edge and which edge is 3'-edge. In some cases one or both edges of a profile and/or discrete segment is an edge of a chromosome.

In some cases, the edges of a discrete segment are determined according to a decomposition rendering generated for a reference sample (e.g., a reference profile). In some cases a null edge height distribution is determined according to a decomposition rendering of a reference profile (e.g., a profile of a chromosome or part thereof). In certain cases, the edges of a discrete segment in a profile are identified when the level of the discrete segment is outside a null edge height distribution. In some cases, the edges of a discrete segment in a profile are identified according a Z-score calculated according to a decomposition rendering for a reference profile.

In some instances, segmenting generates two or more discrete segments (e.g., two or more fragmented levels, two or more fragmented segments) in a profile. In some cases, a decomposition rendering derived from a segmenting process is over-segmented or fragmented and comprises multiple discrete segments. Sometimes discrete segments generated by segmenting are substantially different and sometimes discrete segments generated by segmenting are substantially similar. Substantially similar discrete segments (e.g., substantially similar levels) often refers to two or more adjacent discrete segments in a segmented profile each having a level that differs by less than a predetermined level of uncertainty. In some cases, substantially similar discrete segments are adjacent to each other and are not separated by an intervening segment. In some cases, substantially similar discrete segments are separated by one or more smaller segments. In some cases substantially similar discrete segments are separated by about 1 to about 20, about 1 to about 15, about 1 to about 10 or about 1 to about 5 portions where one or more of the intervening portions have a level significantly different than the level of each of the substantially similar discrete segments. In some cases, the level of substantially similar discrete segments differs by less than about 3 times, less than about 2 times, less than about 1 time or less than about 0.5 times a level of uncertainty. Substantially similar discrete segments, in some cases, comprise a median level that differs by less than 3 MAD (e.g., less than 3 sigma), less than 2 MAD, less than 1 MAD or less than about 0.5 MAD, where a MAD is calculated from a median level of each of the segments. Substantially different discrete segments, in some cases, are not adjacent or are separated by 10 or more, 15 or more or 20 or more portions. Substantially different discrete segments generally have substantially different levels. In certain cases, substantially different discrete segments comprises levels that differ by more than about 2.5 times, more than about 3 times, more than about 4 times, more than about 5 times, more than about 6 times a level of uncertainty. Substantially different discrete segments, in some cases, comprise a median level that differs by more than 2.5 MAD (e.g., more than 2.5 sigma), more than 3 MAD, more than 4 MAD, more than about 5 MAD or more than about 6 MAD, where a MAD is calculated from a median level of each of the discrete segments.

In some cases, a segmentation process comprises determining (e.g., calculating) a level (e.g., a quantitative value, e.g., a mean or median level), a level of uncertainty (e.g., an uncertainty value), Z-score, Z-value, p-value, the like or combinations thereof for one or more discrete segments in a profile or part thereof. In some cases a level (e.g., a quantitative value, e.g., a mean or median level), a level of uncertainty (e.g., an uncertainty value), Z-score, Z-value, p-value, the like or combinations thereof are determined (e.g., calculated) for a discrete segment.

Segmenting can be performed, in full or in part, by one or more decomposition generating processes. A decomposition generating process may provide, for example, a decomposition rendering of a profile. Any decomposition generating process described herein or known in the art may be used. Non-limiting examples of a decomposition generating process include circular binary segmentation (CBS) (see e.g., Olshen et al. (2004) Biostatistics 5(4):557-72; Venkatraman, ES, Olshen, AB (2007) Bioinformatics 23(6):657-63); Haar wavelet segmentation (see e.g., Haar, Alfred (1910) Mathematische Annalen 69(3):331-371); maximal overlap discrete wavelet transform (MODWT) (see e.g., Hsu et al. (2005) Biostatistics 6 (2):211-226); stationary wavelet (SWT) (see e.g., Y. Wang and S. Wang (2007) International Journal of Bioinformatics Research and Applications 3(2):206-222); dual-tree complex wavelet transform (DTCWT) (see e.g., Nguyen et al. (2007) Proceedings of the 7th IEEE International Conference, Boston MA, on October 14-17, 2007, pages 137-144); maximum entropy segmentation, convolution with edge detection kernel, Jensen Shannon Divergence, Kullback-Leibler divergence, Binary Recursive Segmentation, a Fourier transform, the like or combinations thereof.

In some cases, segmenting is accomplished by a process that comprises one process or multiple sub-processes, non-limiting examples of which include a decomposition generating process, thresholding, leveling, smoothing, polishing, the like or combination thereof. Thresholding, leveling, smoothing, polishing and the like can be performed in conjunction with a decomposition generating process, for example.

In some cases, a decision analysis comprises identifying a candidate segment in a decomposition rendering. A candidate segment is determined as being the most significant discrete segment in a decomposition rendering. A candidate segment may be the most significant in terms of the number of portions covered by the segment and/or in terms of the absolute value of the level of normalized counts for the segment. A candidate segment sometimes is larger and sometimes substantially larger than other discrete segments in a decomposition rendering. A candidate segment can be identified by a suitable method. In some cases, a candidate segment is identified by an area under the curve (AUC) analysis. In certain cases, where a first discrete segment has a level and/or covers a number of portions substantially larger than for another discrete segment in a decomposition rendering, the first segment comprises a larger AUC. Where a level is analyzed for AUC, an absolute value of a level often is utilized (e.g., a level corresponding to normalized counts can have a negative value for a deletion and a positive value for a duplication). In certain cases, an AUC is determined as an absolute value of a calculated AUC (e.g., a resulting positive value). In certain cases, a candidate segment, once identified (e.g., by an AUC analysis or by a suitable method) and optionally after it is validated, is selected for a z-score calculation, or the like, to determine if the candidate segment represents a genetic variation (e.g., an aneuploidy, microdeletion or microduplication).

In some cases, a decision analysis comprises a comparison. In some cases, a comparison comprises comparing at least two decomposition renderings. In some cases, a comparison comprises comparing at least two candidate segments. In certain cases, each of the at least two candidate segments is from a different decomposition rendering. For example, a first candidate segment can be from a first decomposition rendering and a second candidate segment can be from a second decomposition rendering. In some cases, a comparison comprises determining if two decomposition renderings are substantially the same or different. In some cases, a comparison comprises determining if two candidate segments are substantially the same or different. Two candidate segments can be determined as substantially the same or different by a suitable comparison method, non-limiting examples of which include by visual inspection, by comparing levels or Z-scores of the two candidate segments, by comparing the edges of the two candidate segments, by overlaying either the two candidate segments or their corresponding decomposition renderings, the like or combinations thereof.

### Outcome

Methods described herein can provide a determination of the presence or absence of a genetic variation for a sample (e.g., fetal aneuploidy), thereby providing an outcome (e.g., thereby providing an outcome determinative of the presence or absence of a genetic variation). Methods herein sometimes provide a classification as to the presence or absence of a genetic variation for a sample (i.e., a classification outcome). A genetic variation often includes a gain, a loss and/or alteration (e.g., duplication, deletion, fusion, insertion, mutation, reorganization, substitution or aberrant methylation) of genetic information (e.g., chromosomes, parts of chromosomes, polymorphic regions, translocated regions, altered nucleotide sequence, the like or combinations of the foregoing) that results in a detectable change in the genome or genetic information of a test subject with respect to a reference. Presence or absence of a genetic variation can be determined by transforming, analyzing and/or manipulating sequence reads that have been mapped to portions (e.g., counts, counts of genomic portions of a reference genome). In certain cases, an outcome is determined according to normalized counts, read densities, read density profiles, and the like.

In some cases, reference samples are euploid for a selected part of a genome (e.g., region), and a measure of uncertainty between a test profile and a reference profile is assessed for the selected region. In some cases, a determination of the presence or absence of a genetic variation is according to the number of deviations (e.g., measures of deviations, MAD) between a test profile and a reference profile for a selected region of a genome (e.g., a chromosome, or part thereof). In some cases, the presence of a genetic variation is determined when the number of deviations between a test profile and a reference profile is greater than about 1, greater than about 1.5, greater than about 2, greater than about 2.5, greater than about 2.6,greater than about 2.7, greater than about 2.8, greater than about 2.9,greater than about 3,greater than about 3.1, greater than about 3.2, greater than about 3.3, greater than about 3.4, greater than about 3.5, greater than about 4, greater than about 5, or greater than about 6. For example, sometimes a test profile and a reference profile differ by more than 3 measures of deviation (e.g., 3 sigma, 3 MAD) and the presence of a genetic variation is determined. A deviation of greater than three between a test profile and a reference profile often is indicative of a non-euploid test subject (e.g., presence of a genetic variation) for a selected region. A test profile significantly greater than a reference profile for a selected region, which reference is euploid for the selected region, sometimes is determinative of a trisomy. Test profiles significantly below a reference profile, which reference profile is indicative of euploidy, sometimes are determinative of a monosomy.

In some cases, the absence of a genetic variation is determined when the number of deviations between a test profile and reference profile for a selected region of a genome is less than about 3.5, less than about 3.4, less than about 3.3, less than about 3.2, less than about 3.1,less than about 3.0,less than about 2.9,less than about 2.8,less than about 2.7,less than about 2.6, less than about 2.5, less than about 2.0, less than about 1.5, or less than about 1.0. For example, sometimes a test profile differs from a reference profile by less than 3 measures of deviation (e.g., 3 sigma, 3 MAD) and the absence of a genetic variation is determined. In some cases, deviation of less than three between test profiles and reference profiles (e.g., 3-sigma for standard deviation) often is indicative of a region that is euploid (e.g., absence of a genetic variation). A measure of deviation between test profiles for a test sample and reference profiles for one or more reference subjects can be plotted and visualized (e.g., z-score plot).

In some cases, a determination of the presence or absence of a genetic variation is determined according to a call zone. In certain cases, a call is made (e.g., a call determining the presence or absence of a genetic variation) when a value (e.g., a profile, a read density profile and/or a measure of uncertainty) or collection of values falls within a pre-defined range (e.g., a zone, a call zone). In some cases, a call zone is defined according to a collection of values (e.g., profiles, read density profiles and/or measures of uncertainty) that are obtained from the same patient sample. In certain cases, a call zone is defined according to a collection of values that are derived from the same chromosome or part thereof. In some cases, a call zone based on a genetic variation determination is defined according a measure of uncertainty (e.g., high level of confidence, e.g., low measure of uncertainty) and/or a quantification of a minority nucleic acid species (e.g.,fetal fraction; e.g., 2%, 3%, 4% or higher minority species fraction).

In some cases, a call zone is defined by a confidence level of about 99% or greater, about 99.1% or greater, about 99.2% or greater, about 99.3% or greater, about 99.4% or greater, about 99.5% or greater, about 99.6% or greater, about 99.7% or greater, about 99.8% or greater or about 99.9% or greater. In some cases, a call is made without using a call zone. In some cases, a call is made using a call zone and additional data or information. In some cases, a call is made based on a comparison without the use of a call zone. In some cases, a call is made based on visual inspection of a profile (e.g., visual inspection of read densities).

In some cases, a no-call zone is where a call is not made. In some cases, a no-call zone is defined by a value or collection of values that indicate low accuracy, high risk, high error, low level of confidence, high measure of uncertainty, the like or a combination thereof. In some cases, a no-call zone is defined, in part, by a minority species fraction of about 5% or less, about 4% or less, about 3% or less, about 2.5% or less, about 2.0% or less, about 1.5% or less or about 1.0% or less.

A genetic variation sometimes is associated with medical condition. An outcome determinative of a genetic variation is sometimes an outcome determinative of the presence or absence of a condition (e.g., a medical condition), disease, syndrome or abnormality, or includes, detection of a condition, disease, syndrome or abnormality. In certain cases, a diagnosis comprises assessment of an outcome. An outcome determinative of the presence or absence of a condition (e.g., a medical condition), disease, syndrome or abnormality by methods described herein can sometimes be independently verified by further testing (e.g., by scan (e.g., CT scan, MRI), karyotyping and/or amniocentesis). Analysis and processing of data can provide one or more outcomes. The term "outcome" as used herein can refer to a result of data processing that facilitates determining the presence or absence of a genetic variation (e.g., an aneuploidy, a copy number variation). In certain cases, the term "outcome" as used herein refers to a conclusion that predicts and/or determines the presence or absence of a genetic variation. In certain cases, the term "outcome" as used herein refers to a conclusion that predicts and/or determines a risk or probability of the presence or absence of a genetic variation in a subject.

A diagnosis sometimes comprises use of an outcome. For example, a health practitioner may analyze an outcome and provide a diagnosis based on, or based in part on, the outcome. In some cases, determination, detection or diagnosis of a condition, syndrome or abnormality comprises use of an outcome determinative of the presence or absence of a genetic variation. In some cases, an outcome based on counted mapped sequence reads or transformations thereof is determinative of the presence or absence of a genetic variation. In certain cases, an outcome generated utilizing one or more methods (e.g., data processing methods) described herein is determinative of the presence or absence of one or more conditions, syndromes or abnormalities. In certain cases, a diagnosis comprises a determination of a presence or absence of a condition, syndrome or abnormality. In certain instances, a diagnosis comprises a determination of a genetic variation as the nature and/or cause of a condition, syndrome or abnormality. In certain cases, an outcome is not a diagnosis. An outcome often comprises one or more numerical values generated using a processing method described herein in the context of one or more considerations of probability. A consideration of risk or probability can include, but is not limited to: a measure of uncertainty, a confidence level, sensitivity, specificity, standard deviation, coefficient of variation (CV) and/or confidence level, Z-scores, Chi values, Phi values, ploidy values, fitted minority species fraction, area ratios, median level, the like or combinations thereof. A consideration of probability can facilitate determining whether a subject is at risk of having, or has, a genetic variation, and an outcome determinative of a presence or absence of a genetic disorder often includes such a consideration.

An outcome sometimes is a phenotype. An outcome sometimes is a phenotype with an associated level of confidence (e.g., a measure of uncertainty, e.g., a fetus is positive for trisomy 21 with a confidence level of 99%, a test subject is negative for a cancer associated with a genetic variation at a confidence level of 95%). Different methods of generating outcome values sometimes can produce different types of results. Generally, there are four types of possible scores or calls that can be made based on outcome values generated using methods described herein: true positive, false positive, true negative and false negative. The terms "score," "scores," "call" and "calls" as used herein refer to calculating the probability that a particular genetic variation is present or absent in a subject/sample. The value of a score may be used to determine, for example, a variation, difference, or ratio of mapped sequence reads that may correspond to a genetic variation. For example, calculating a positive score for a selected genetic variation or portion from a data set, with respect to a reference genome can lead to an identification of the presence or absence of a genetic variation, which genetic variation sometimes is associated with a medical condition (e.g., preeclampsia, trisomy, monosomy, and the like). In some cases, an outcome comprises a read density, a read density profile and/or a plot (e.g., a profile plot). In those cases in which an outcome comprises a profile, a suitable profile or combination of profiles can be used for an outcome. Non-limiting examples of profiles that can be used for an outcome include z-score profiles, p-value profiles, chi value profiles, phi value profiles, the like, and combinations thereof.

An outcome generated for determining the presence or absence of a genetic variation sometimes includes a null result (e.g., a data point between two clusters, a numerical value with a standard deviation that encompasses values for both the presence and absence of a genetic variation, a data set with a profile plot that is not similar to profile plots for subjects having or free from the genetic variation being investigated). In some cases, an outcome indicative of a null result still is a determinative result, and the determination can include the need for additional information and/or a repeat of the data generation and/or analysis for determining the presence or absence of a genetic variation.

An outcome can be generated after performing one or more processing steps described herein, in some cases. In certain cases, an outcome is generated as a result of one of the processing steps described herein, and in some cases, an outcome can be generated after each statistical and/or mathematical manipulation of a data set is performed. An outcome pertaining to the determination of the presence or absence of a genetic variation can be expressed in a suitable form, which form comprises without limitation, a probability (e.g., odds ratio, p-value), likelihood, value in or out of a cluster, value over or under a threshold value, value within a range (e.g., a threshold range), value with a measure of variance or confidence, or risk factor, associated with the presence or absence of a genetic variation for a subject or sample. In certain cases, comparison between samples allows confirmation of sample identity (e.g., allows identification of repeated samples and/or samples that have been mixed up (e.g., mislabeled, combined, and the like)).

In some cases, an outcome comprises a value above or below a predetermined threshold or cutoff value and/or a measure of uncertainty or a confidence level associated with the value. In certain cases, a predetermined threshold or cutoff value is an expected level or an expected level range. An outcome also can describe an assumption used in data processing. In certain cases, an outcome comprises a value that falls within or outside a predetermined range of values (e.g., a threshold range) and the associated uncertainty or confidence level for that value being inside or outside the range. In some cases, an outcome comprises a value that is equal to a predetermined value (e.g., equal to 1, equal to zero), or is equal to a value within a predetermined value range, and its associated uncertainty or confidence level for that value being equal or within or outside a range. An outcome sometimes is graphically represented as a plot (e.g., profile plot).

As noted above, an outcome can be characterized as a true positive, true negative, false positive or false negative. The term "true positive" as used herein refers to a subject correctly diagnosed as having a genetic variation. The term "false positive" as used herein refers to a subject wrongly identified as having a genetic variation. The term "true negative" as used herein refers to a subject correctly identified as not having a genetic variation. The term "false negative" as used herein refers to a subject wrongly identified as not having a genetic variation. Two measures of performance for any given method can be calculated based on the ratios of these occurrences: (i) a sensitivity value, which generally is the fraction of predicted positives that are correctly identified as being positives; and (ii) a specificity value, which generally is the fraction of predicted negatives correctly identified as being negative.

In certain cases, one or more of sensitivity, specificity and/or confidence level are expressed as a percentage. In some cases, the percentage, independently for each variable, is greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). Coefficient of variation (CV) in some cases is expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01% or less)). A probability (e.g., that a particular outcome is not due to chance) in certain cases is expressed as a Z-score, a p-value, or the results of a t-test. In some cases, a measured variance, confidence interval, sensitivity, specificity and the like (e.g., referred to collectively as confidence parameters) for an outcome can be generated using one or more data processing manipulations described herein. Specific examples of generating outcomes and associated confidence levels are described, for example, in International Patent Application Publication No. WO2013/0529 13.

The term "sensitivity" as used herein refers to the number of true positives divided by the number of true positives plus the number of false negatives, where sensitivity (sens) may be within the range of 0 ≤ sens ≤ 1. The term "specificity" as used herein refers to the number of true negatives divided by the number of true negatives plus the number of false positives, where sensitivity (spec) may be within the range of 0 ≤ spec ≤ 1. In some cases, a method that has sensitivity and specificity equal to one, or 100%, or near one (e.g., between about 90% to about 99%) sometimes is selected. In some cases, a method having a sensitivity equaling 1, or 100% is selected, and in certain cases, a method having a sensitivity near 1 is selected (e.g., a sensitivity of about 90%, a sensitivity of about 91%, a sensitivity of about 92%, a sensitivity of about 93%, a sensitivity of about 94%, a sensitivity of about 95%, a sensitivity of about 96%, a sensitivity of about 97%, a sensitivity of about 98%, or a sensitivity of about 99%). In some cases, a method having a specificity equaling 1, or 100% is selected, and in certain cases, a method having a specificity near 1 is selected (e.g., a specificity of about 90%, a specificity of about 91%, a specificity of about 92%, a specificity of about 93%, a specificity of about 94%, a specificity of about 95%, a specificity of about 96%, a specificity of about 97%, a specificity of about 98%, or a specificity of about 99%).

A genetic variation or an outcome determinative of a genetic variation identified by methods described herein can be independently verified by further testing (e.g., by targeted sequencing of nucleic acid). An outcome typically is provided to a health care professional (e.g., laboratory technician or manager; physician or assistant). In certain cases, an outcome is provided on a suitable visual medium (e.g., a peripheral or component of a machine, e.g., a printer or display). In some cases, an outcome determinative of the presence or absence of a genetic variation is provided to a healthcare professional in the form of a report, and in certain cases the report comprises a display of an outcome value and an associated confidence parameter. Generally, an outcome can be displayed in a suitable format that facilitates determination of the presence or absence of a genetic variation and/or medical condition. Non-limiting examples of formats suitable for use for reporting and/or displaying data sets or reporting an outcome include digital data, a graph, a 2D graph, a 3D graph, and 4D graph, a picture (e.g., a jpg, bitmap (e.g., bmp), pdf, tiff, gif, raw, png, the like or suitable format), a pictograph, a chart, a table, a bar graph, a pie graph, a diagram, a flow chart, a scatter plot, a map, a histogram, a density chart, a function graph, a circuit diagram, a block diagram, a bubble map, a constellation diagram, a contour diagram, a cartogram, spider chart, Venn diagram, nomogram, and the like, and combination of the foregoing.

Generating an outcome often can be viewed as a transformation of nucleic acid sequence reads, or the like, into a representation of a subject's cellular nucleic acid. For example, transmuting sequence reads of nucleic acid from a subject by a method described herein, and generating an outcome can be viewed as a transformation of relatively small sequence read fragments to a representation of relatively large and complex structure of nucleic acid in the subject. In some cases, an outcome results from a transformation of sequence reads from a subject into a representation of an existing nucleic acid structure present in the subject (e.g., a genome, a chromosome, chromosome segment, mixture of CF nucleic acid fragments in the subject).

### Use of outcomes

A health care professional, or other qualified individual, receiving a report comprising one or more outcomes determinative of the presence or absence of a genetic variation can use the displayed data in the report to make a call regarding the status of the test subject or patient. The healthcare professional can make a recommendation based on the provided outcome, in some cases. A health care professional or qualified individual can provide a test subject or patient with a call or score with regards to the presence or absence of the genetic variation based on the outcome value or values and associated confidence parameters provided in a report, in some cases. In certain cases, a score or call is made manually by a healthcare professional or qualified individual, using visual observation of the provided report. In certain cases, a score or call is made by an automated routine, sometimes embedded in software, and reviewed by a healthcare professional or qualified individual for accuracy prior to providing information to a test subject or patient. The term "receiving a report" as used herein refers to obtaining, by a communication means, a written and/or graphical representation comprising an outcome, which upon review allows a healthcare professional or other qualified individual to make a determination as to the presence or absence of a genetic variation in a test subject or patient. The report may be generated by a computer or by human data entry, and can be communicated using electronic means (e.g., over the internet, via computer, via fax, from one network location to another location at the same or different physical sites), or by a other method of sending or receiving data (e.g., mail service, courier service and the like). In some cases, the outcome is transmitted to a health care professional in a suitable medium, including, without limitation, in verbal, document, or file form. The file may be, for example, but not limited to, an auditory file, a computer readable file, a paper file, a laboratory file or a medical record file.

The term "providing an outcome" and grammatical equivalents thereof, as used herein also can refer to a method for obtaining such information, including, without limitation, obtaining the information from a laboratory (e.g., a laboratory file). A laboratory file can be generated by a laboratory that carried out one or more assays or one or more data processing steps to determine the presence or absence of the medical condition. The laboratory may be in the same location or different location (e.g., in another country) as the personnel identifying the presence or absence of the medical condition from the laboratory file. For example, the laboratory file can be generated in one location and transmitted to another location in which the information therein will be transmitted to the subject. The laboratory file may be in tangible form or electronic form (e.g., computer readable form), in certain cases.

In some cases, an outcome can be provided to a health care professional, physician or qualified individual from a laboratory and the health care professional, physician or qualified individual can make a diagnosis based on the outcome. In some cases, an outcome can be provided to a health care professional, physician or qualified individual from a laboratory and the health care professional, physician or qualified individual can make a diagnosis based, in part, on the outcome along with additional data and/or information and other outcomes.

A healthcare professional or qualified individual can provide a suitable recommendation based on the outcome or outcomes provided in the report. Non-limiting examples of recommendations that can be provided based on the provided outcome report includes, surgery, radiation therapy, chemotherapy, genetic counseling, after birth treatment solutions (e.g., life planning, long term assisted care, medicaments, symptomatic treatments), pregnancy termination, organ transplant, blood transfusion, the like or combinations of the foregoing. In some cases, the recommendation is dependent on the outcome based classification provided (e.g., cancer, stage and/or type of cancer, Down's syndrome, Turner syndrome, medical conditions associated with genetic variations in T13, medical conditions associated with genetic variations in T18).

Laboratory personnel (e.g., a laboratory manager) can analyze values (e.g., test profiles, reference profiles, level of deviation) underlying a determination of the presence or absence of a genetic variation. For calls pertaining to presence or absence of a genetic variation that are close or questionable, laboratory personnel can re-order the same test, and/or order a different test, that makes use of the same or different sample nucleic acid from a test subject.

### Machines, software and interfaces

Certain processes and methods described herein (e.g., mapping, counting, normalizing, range setting, adjusting, categorizing and/or determining sequence reads, counts, levels and/or profiles) often cannot be performed without a computer, microprocessor, software, module or other machine. Methods described herein typically are computer-implemented methods, and one or more portions of a method sometimes are performed by one or more processors (e.g., microprocessors), computers, systems, apparatuses, or machines (e.g., microprocessor-controlled machine).

Computers, systems, apparatuses, machines and computer program products suitable for use often include, or are utilized in conjunction with, computer readable storage media. Non-limiting examples of computer readable storage media include memory, hard disk, CD-ROM, flash memory device and the like. Computer readable storage media generally are computer hardware, and often are non-transitory computer-readable storage media. Computer readable storage media are not computer readable transmission media, the latter of which are transmission signals per se.

Provided herein are computer readable storage media with an executable program stored thereon, where the program instructs a microprocessor to perform a method described herein. Provided also are computer readable storage media with an executable program module stored thereon, where the program module instructs a microprocessor to perform part of a method described herein. Also provided herein are systems, machines, apparatuses and computer program products that include computer readable storage media with an executable program stored thereon, where the program instructs a microprocessor to perform a method described herein. Provided also are systems, machines and apparatuses that include computer readable storage media with an executable program module stored thereon, where the program module instructs a microprocessor to perform part of a method described herein.

Also provided are computer program products. A computer program product often includes a computer usable medium that includes a computer readable program code embodied therein, the computer readable program code adapted for being executed to implement a method or part of a method described herein. Computer usable media and readable program code are not transmission media (i.e., transmission signals per se). Computer readable program code often is adapted for being executed by a processor, computer, system, apparatus, or machine.

In some cases, methods described herein (e.g., quantifying, counting, filtering, normalizing, transforming, clustering and/or determining sequence reads, counts, levels, profiles and/or outcomes) are performed by automated methods. In some cases, one or more steps of a method described herein are carried out by a microprocessor and/or computer, and/or carried out in conjunction with memory. In some cases, an automated method is embodied in software, modules, microprocessors, peripherals and/or a machine comprising the like, that perform methods described herein. As used herein, software refers to computer readable program instructions that, when executed by a microprocessor, perform computer operations, as described herein.

Sequence reads, counts, levels and/or profiles sometimes are referred to as "data" or "data sets." In some cases, data or data sets can be characterized by one or more features or variables (e.g., sequence based (e.g., GC content, specific nucleotide sequence, the like), function specific (e.g., expressed genes, cancer genes, the like), location based (genome specific, chromosome specific, portion or portion-specific), the like and combinations thereof). In certain cases, data or data sets can be organized into a matrix having two or more dimensions based on one or more features or variables. Data organized into matrices can be organized using any suitable features or variables. In certain cases, data sets characterized by one or more features or variables sometimes are processed after counting.

Machines, software and interfaces may be used to conduct methods described herein. Using machines, software and interfaces, a user may enter, request, query or determine options for using particular information, programs or processes (e.g., mapping sequence reads, processing mapped data and/or providing an outcome), which can involve implementing statistical analysis algorithms, statistical significance algorithms, statistical algorithms, iterative steps, validation algorithms, and graphical representations, for example. In some cases, a data set may be entered by a user as input information, a user may download one or more data sets by suitable hardware media (e.g., flash drive), and/or a user may send a data set from one system to another for subsequent processing and/or providing an outcome (e.g., send sequence read data from a sequencer to a computer system for sequence read mapping; send mapped sequence data to a computer system for processing and yielding an outcome and/or report).

A system typically comprises one or more machines. Each machine comprises one or more of memory, one or more microprocessors, and instructions. Where a system includes two or more machines, some or all of the machines may be located at the same location, some or all of the machines may be located at different locations, all of the machines may be located at one location and/or all of the machines may be located at different locations. Where a system includes two or more machines, some or all of the machines may be located at the same location as a user, some or all of the machines may be located at a location different than a user, all of the machines may be located at the same location as the user, and/or all of the machine may be located at one or more locations different than the user.

A system sometimes comprises a computing machine and a sequencing apparatus or machine, where the sequencing apparatus or machine is configured to receive physical nucleic acid and generate sequence reads, and the computing apparatus is configured to process the reads from the sequencing apparatus or machine. The computing machine sometimes is configured to determine a classification outcome from the sequence reads.

A user may, for example, place a query to software which then may acquire a data set via internet access, and in certain cases, a programmable microprocessor may be prompted to acquire a suitable data set based on given parameters. A programmable microprocessor also may prompt a user to select one or more data set options selected by the microprocessor based on given parameters. A programmable microprocessor may prompt a user to select one or more data set options selected by the microprocessor based on information found via the internet, other internal or external information, or the like. Options may be chosen for selecting one or more data feature selections, one or more statistical algorithms, one or more statistical analysis algorithms, one or more statistical significance algorithms, iterative steps, one or more validation algorithms, and one or more graphical representations of methods, machines, apparatuses, computer programs or a non-transitory computer-readable storage medium with an executable program stored thereon.

Systems addressed herein may comprise general components of computer systems, such as, for example, network servers, laptop systems, desktop systems, handheld systems, personal digital assistants, computing kiosks, and the like. A computer system may comprise one or more input means such as a keyboard, touch screen, mouse, voice recognition or other means to allow the user to enter data into the system. A system may further comprise one or more outputs, including, but not limited to, a display screen (e.g., CRT or LCD), speaker, FAX machine, printer (e.g., laser, ink jet, impact, black and white or color printer), or other output useful for providing visual, auditory and/or hardcopy output of information (e.g., outcome and/or report).

In a system, input and output components may be connected to a central processing unit which may comprise among other components, a microprocessor for executing program instructions and memory for storing program code and data. In some cases, processes may be implemented as a single user system located in a single geographical site. In certain cases, processes may be implemented as a multi-user system. In the case of a multi-user implementation, multiple central processing units may be connected by means of a network. The network may be local, encompassing a single department in one portion of a building, an entire building, span multiple buildings, span a region, span an entire country or be worldwide. The network may be private, being owned and controlled by a provider, or it may be implemented as an internet based service where the user accesses a web page to enter and retrieve information. Accordingly, in certain cases, a system includes one or more machines, which may be local or remote with respect to a user. More than one machine in one location or multiple locations may be accessed by a user, and data may be mapped and/or processed in series and/or in parallel. Thus, a suitable configuration and control may be utilized for mapping and/or processing data using multiple machines, such as in local network, remote network and/or "cloud" computing platforms.

A system can include a communications interface in some cases. A communications interface allows for transfer of software and data between a computer system and one or more external devices. Non-limiting examples of communications interfaces include a modem, a network interface (such as an Ethernet card), a communications port, a PCMCIA slot and card, and the like. Software and data transferred via a communications interface generally are in the form of signals, which can be electronic, electromagnetic, optical and/or other signals capable of being received by a communications interface. Signals often are provided to a communications interface via a channel. A channel often carries signals and can be implemented using wire or cable, fiber optics, a phone line, a cellular phone link, an RF link and/or other communications channels. Thus, in an example, a communications interface may be used to receive signal information that can be detected by a signal detection module.

Data may be input by a suitable device and/or method, including, but not limited to, manual input devices or direct data entry devices (DDEs). Non-limiting examples of manual devices include keyboards, concept keyboards, touch sensitive screens, light pens, mouse, tracker balls, joysticks, graphic tablets, scanners, digital cameras, video digitizers and voice recognition devices. Non-limiting examples of DDEs include bar code readers, magnetic strip codes, smart cards, magnetic ink character recognition, optical character recognition, optical mark recognition, and turnaround documents.

In some cases, output from a sequencing apparatus or machine may serve as data that can be input via an input device. In certain cases, mapped sequence reads may serve as data that can be input via an input device. In certain cases, nucleic acid fragment size (e.g., length) may serve as data that can be input via an input device. In certain cases, output from a nucleic acid capture process (e.g., genomic region origin data) may serve as data that can be input via an input device. In certain cases, a combination of nucleic acid fragment size (e.g., length) and output from a nucleic acid capture process (e.g., genomic region origin data) may serve as data that can be input via an input device. In certain cases, simulated data is generated by an in silico process and the simulated data serves as data that can be input via an input device. The term "in silico" refers to research and experiments performed using a computer. In silico processes include, but are not limited to, mapping sequence reads and processing mapped sequence reads according to processes described herein.

A system may include software useful for performing a process or part of a process described herein, and software can include one or more modules for performing such processes (e.g., sequencing module, logic processing module, data display organization module). The term "software" refers to computer readable program instructions that, when executed by a computer, perform computer operations. Instructions executable by the one or more microprocessors sometimes are provided as executable code, that when executed, can cause one or more microprocessors to implement a method described herein. A module described herein can exist as software, and instructions (e.g., processes, routines, subroutines) embodied in the software can be implemented or performed by a microprocessor. For example, a module (e.g., a software module) can be a part of a program that performs a particular process or task. The term "module" refers to a self-contained functional unit that can be used in a larger machine or software system. A module can comprise a set of instructions for carrying out a function of the module. A module can transform data and/or information. Data and/or information can be in a suitable form. For example, data and/or information can be digital or analogue. In certain cases, data and/or information sometimes can be packets, bytes, characters, or bits. In some cases, data and/or information can be any gathered, assembled or usable data or information. Non-limiting examples of data and/or information include a suitable media, pictures, video, sound (e.g. frequencies, audible or non-audible), numbers, constants, a value, objects, time, functions, instructions, maps, references, sequences, reads, mapped reads, levels, ranges, thresholds, signals, displays, representations, or transformations thereof. A module can accept or receive data and/or information, transform the data and/or information into a second form, and provide or transfer the second form to a machine, peripheral, component or another module. A module can perform one or more of the following non-limiting functions: mapping sequence reads, providing counts, assembling portions, providing or determining a level, providing a count profile, normalizing (e.g., normalizing reads, normalizing counts, and the like), providing a normalized count profile or levels of normalized counts, comparing two or more levels, providing uncertainty values, providing or determining expected levels and expected ranges(e.g., expected level ranges, threshold ranges and threshold levels), providing adjustments to levels (e.g., adjusting a first level, adjusting a second level, adjusting a profile of a chromosome or a part thereof, and/or padding), providing identification (e.g., identifying a copy number alteration, genetic variation or aneuploidy), categorizing, plotting, and/or determining an outcome, for example. A microprocessor can, in certain cases, carry out the instructions in a module. In some cases, one or more microprocessors are required to carry out instructions in a module or group of modules. A module can provide data and/or information to another module, machine or source and can receive data and/or information from another module, machine or source.

A computer program product sometimes is embodied on a tangible computer-readable medium, and sometimes is tangibly embodied on a non-transitory computer-readable medium. A module sometimes is stored on a computer readable medium (e.g., disk, drive) or in memory (e.g., random access memory). A module and microprocessor capable of implementing instructions from a module can be located in a machine or in a different machine. A module and/or microprocessor capable of implementing an instruction for a module can be located in the same location as a user (e.g., local network) or in a different location from a user (e.g., remote network, cloud system). In cases in which a method is carried out in conjunction with two or more modules, the modules can be located in the same machine, one or more modules can be located in different machine in the same physical location, and one or more modules may be located in different machines in different physical locations.

A machine, in some cases, comprises at least one microprocessor for carrying out the instructions in a module. Sequence read quantifications (e.g., counts) sometimes are accessed by a microprocessor that executes instructions configured to carry out a method described herein. Sequence read quantifications that are accessed by a microprocessor can be within memory of a system, and the counts can be accessed and placed into the memory of the system after they are obtained. In some cases, a machine includes a microprocessor (e.g., one or more microprocessors) which microprocessor can perform and/or implement one or more instructions (e.g., processes, routines and/or subroutines) from a module. In some cases, a machine includes multiple microprocessors, such as microprocessors coordinated and working in parallel. In some cases, a machine operates with one or more external microprocessors (e.g., an internal or external network, server, storage device and/or storage network (e.g., a cloud)). In some cases, a machine comprises a module (e.g., one or more modules). A machine comprising a module often is capable of receiving and transferring one or more of data and/or information to and from other modules.

In certain cases, a machine comprises peripherals and/or components. In certain cases, a machine can comprise one or more peripherals or components that can transfer data and/or information to and from other modules, peripherals and/or components. In certain cases, a machine interacts with a peripheral and/or component that provides data and/or information. In certain cases, peripherals and components assist a machine in carrying out a function or interact directly with a module. Non-limiting examples of peripherals and/or components include a suitable computer peripheral, I/O or storage method or device including but not limited to scanners, printers, displays (e.g., monitors, LED, LCT or CRTs), cameras, microphones, pads (e.g., ipads, tablets), touch screens, smart phones, mobile phones, USB I/O devices, USB mass storage devices, keyboards, a computer mouse, digital pens, modems, hard drives, jump drives, flash drives, a microprocessor, a server, CDs, DVDs, graphic cards, specialized I/O devices (e.g., sequencers, photo cells, photo multiplier tubes, optical readers, sensors, etc.), one or more flow cells, fluid handling components, network interface controllers, ROM, RAM, wireless transfer methods and devices (Bluetooth, WiFi, and the like,), the world wide web (www), the internet, a computer and/or another module.

Software often is provided on a program product containing program instructions recorded on a computer readable medium, including, but not limited to, magnetic media including floppy disks, hard disks, and magnetic tape; and optical media including CD-ROM discs, DVD discs, magneto-optical discs, flash memory devices (e.g., flash drives), RAM, floppy discs, the like, and other such media on which the program instructions can be recorded. In online implementation, a server and web site maintained by an organization can be configured to provide software downloads to remote users, or remote users may access a remote system maintained by an organization to remotely access software. Software may obtain or receive input information. Software may include a module that specifically obtains or receives data (e.g., a data receiving module that receives sequence read data and/or mapped read data) and may include a module that specifically processes the data (e.g., a processing module that processes received data (e.g., filters, normalizes, provides an outcome and/or report). The terms "obtaining" and "receiving" input information refers to receiving data (e.g., sequence reads, mapped reads) by computer communication means from a local, or remote site, human data entry, or any other method of receiving data. The input information may be generated in the same location at which it is received, or it may be generated in a different location and transmitted to the receiving location. In some cases, input information is modified before it is processed (e.g., placed into a format amenable to processing (e.g., tabulated)).

Software can include one or more algorithms in certain cases. An algorithm may be used for processing data and/or providing an outcome or report according to a finite sequence of instructions. An algorithm often is a list of defined instructions for completing a task. Starting from an initial state, the instructions may describe a computation that proceeds through a defined series of successive states, eventually terminating in a final ending state. The transition from one state to the next is not necessarily deterministic (e.g., some algorithms incorporate randomness). By way of example, and without limitation, an algorithm can be a search algorithm, sorting algorithm, merge algorithm, numerical algorithm, graph algorithm, string algorithm, modeling algorithm, computational genometric algorithm, combinatorial algorithm, machine learning algorithm, cryptography algorithm, data compression algorithm, parsing algorithm and the like. An algorithm can include one algorithm or two or more algorithms working in combination. An algorithm can be of any suitable complexity class and/or parameterized complexity. An algorithm can be used for calculation and/or data processing, and in some cases, can be used in a deterministic or probabilistic/predictive approach. An algorithm can be implemented in a computing environment by use of a suitable programming language, non-limiting examples of which are C, C++, Java, Perl, Python, Fortran, and the like. In some cases, an algorithm can be configured or modified to include margin of errors, statistical analysis, statistical significance, and/or comparison to other information or data sets (e.g., applicable when using a neural net or clustering algorithm).

In certain cases, several algorithms may be implemented for use in software. These algorithms can be trained with raw data in some cases. For each new raw data sample, the trained algorithms may produce a representative processed data set or outcome. A processed data set sometimes is of reduced complexity compared to the parent data set that was processed. Based on a processed set, the performance of a trained algorithm may be assessed based on sensitivity and specificity, in some cases. An algorithm with the highest sensitivity and/or specificity may be identified and utilized, in certain cases.

In certain cases, simulated (or simulation) data can aid data processing, for example, by training an algorithm or testing an algorithm. In some cases, simulated data includes hypothetical various samplings of different groupings of sequence reads. Simulated data may be based on what might be expected from a real population or may be skewed to test an algorithm and/or to assign a correct classification. Simulated data also is referred to herein as "virtual" data. Simulations can be performed by a computer program in certain cases. One possible step in using a simulated data set is to evaluate the confidence of identified results, e.g., how well a random sampling matches or best represents the original data. One approach is to calculate a probability value (p-value), which estimates the probability of a random sample having better score than the selected samples. In some cases, an empirical model may be assessed, in which it is assumed that at least one sample matches a reference sample (with or without resolved variations). In some cases, another distribution, such as a Poisson distribution for example, can be used to define the probability distribution.

A system may include one or more microprocessors in certain cases. A microprocessor can be connected to a communication bus. A computer system may include a main memory, often random access memory (RAM), and can also include a secondary memory. Memory in some cases comprises a non-transitory computer-readable storage medium. Secondary memory can include, for example, a hard disk drive and/or a removable storage drive, representing a floppy disk drive, a magnetic tape drive, an optical disk drive, memory card and the like. A removable storage drive often reads from and/or writes to a removable storage unit. Non-limiting examples of removable storage units include a floppy disk, magnetic tape, optical disk, and the like, which can be read by and written to by, for example, a removable storage drive. A removable storage unit can include a computer-usable storage medium having stored therein computer software and/or data.

A microprocessor may implement software in a system. In some cases, a microprocessor may be programmed to automatically perform a task described herein that a user could perform. Accordingly, a microprocessor, or algorithm conducted by such a microprocessor, can require little to no supervision or input from a user (e.g., software may be programmed to implement a function automatically). In some cases, the complexity of a process is so large that a single person or group of persons could not perform the process in a timeframe short enough for determining the presence or absence of a genetic variation.

In some cases, secondary memory may include other similar means for allowing computer programs or other instructions to be loaded into a computer system. For example, a system can include a removable storage unit and an interface device. Non-limiting examples of such systems include a program cartridge and cartridge interface (such as that found in video game devices), a removable memory chip (such as an EPROM, or PROM) and associated socket, and other removable storage units and interfaces that allow software and data to be transferred from the removable storage unit to a computer system.

Fig. 1 illustrates a non-limiting example of a computing environment 110 in which various systems, methods, algorithms, and data structures described herein may be implemented. The computing environment 110 is only one example of a suitable computing environment and is not intended to suggest any limitation as to the scope of use or functionality of the systems, methods, and data structures described herein. Neither should computing environment 110 be interpreted as having any dependency or requirement relating to any one or combination of components illustrated in computing environment 110. A subset of systems, methods, and data structures shown in Fig. 1 can be utilized in certain cases. Systems, methods, and data structures described herein are operational with numerous other general purpose or special purpose computing system environments or configurations. Examples of known computing systems, environments, and/or configurations that may be suitable include, but are not limited to, personal computers, server computers, thin clients, thick clients, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The operating environment 110 of Fig. 1 includes a general purpose computing device in the form of a computer 120, including a processing unit 121, a system memory 122, and a system bus 123 that operatively couples various system components including the system memory 122 to the processing unit 121. There may be only one or there may be more than one processing unit 121, such that the processor of computer 120 includes a single central-processing unit (CPU), or a plurality of processing units, commonly referred to as a parallel processing environment. The computer 120 may be a conventional computer, a distributed computer, or any other type of computer.

The system bus 123 may be any of several types of bus structures including a memory bus or memory controller, a peripheral bus, and a local bus using any of a variety of bus architectures. The system memory may also be referred to as simply the memory, and includes read only memory (ROM) 124 and random access memory (RAM). A basic input/output system (BIOS) 126, containing the basic routines that help to transfer information between elements within the computer 120, such as during start-up, is stored in ROM 124. The computer 120 may further include a hard disk drive interface 127 for reading from and writing to a hard disk, not shown, a magnetic disk drive 128 for reading from or writing to a removable magnetic disk 129, and an optical disk drive 130 for reading from or writing to a removable optical disk 131 such as a CD ROM or other optical media.

The hard disk drive 127, magnetic disk drive 128, and optical disk drive 130 are connected to the system bus 123 by a hard disk drive interface 132, a magnetic disk drive interface 133, and an optical disk drive interface 134, respectively. The drives and their associated computer-readable media provide nonvolatile storage of computer-readable instructions, data structures, program modules and other data for the computer 120. Any type of computer-readable media that can store data that is accessible by a computer, such as magnetic cassettes, flash memory cards, digital video disks, Bernoulli cartridges, random access memories (RAMs), read only memories (ROMs), and the like, may be used in the operating environment.

A number of program modules may be stored on the hard disk, magnetic disk 129, optical disk 131, ROM 124, or RAM, including an operating system 135, one or more application programs 136, other program modules 137, and program data 138. A user may enter commands and information into the personal computer 120 through input devices such as a keyboard 140 and pointing device 142. Other input devices (not shown) may include a microphone, joystick, game pad, satellite dish, scanner, or the like. These and other input devices are often connected to the processing unit 121 through a serial port interface 146 that is coupled to the system bus, but may be connected by other interfaces, such as a parallel port, game port, or a universal serial bus (USB). A monitor 147 or other type of display device is also connected to the system bus 123 via an interface, such as a video adapter 148. In addition to the monitor, computers typically include other peripheral output devices (not shown), such as speakers and printers.

The computer 120 may operate in a networked environment using logical connections to one or more remote computers, such as remote computer 149. These logical connections may be achieved by a communication device coupled to or a part of the computer 120, or in other manners. The remote computer 149 may be another computer, a server, a router, a network PC, a client, a peer device or other common network node, and typically includes many or all of the elements described above relative to the computer 120, although only a memory storage device 150 has been illustrated in Fig. 1. The logical connections depicted in Fig. 1 include a local-area network (LAN) 151 and a wide-area network (WAN) 152. Such networking environments are commonplace in office networks, enterprise-wide computer networks, intranets and the Internet, which all are types of networks.

When used in a LAN-networking environment, the computer 120 is connected to the local network 151 through a network interface or adapter 153, which is one type of communications device. When used in a WAN-networking environment, the computer 120 often includes a modem 154, a type of communications device, or any other type of communications device for establishing communications over the wide area network 152. The modem 154, which may be internal or external, is connected to the system bus 123 via the serial port interface 146. In a networked environment, program modules depicted relative to the personal computer 120, or portions thereof, may be stored in the remote memory storage device. It is appreciated that the network connections shown are non-limiting examples and other communications devices for establishing a communications link between computers may be used.

Thus, also provided in this disclosure is a system for determining the presence or absence of a genetic variation. The system comprises a component for amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome, a component for determining the amount of each amplified paralogous polynucleotide species in each of the sets, a component for determining a paralog ratio for each of the sets between the amount of each amplified paralogous polynucleotide species in each of the sets, thereby generating a plurality of paralog ratios; and a component for determining the presence or absence of the genetic variation based on the plurality of paralog ratios. In some cases, one or more components described above comprise a computer processor. For example, the system may comprise a computer processor that is configured to determine the amount of each amplified paralogous polynucleotide species in each of the sets; a computer processor that is configured to determine a paralog ratio for each of the sets between the amount of each amplified paralogous polynucleotide species in each of the sets, thereby generating a plurality of paralog ratios; and/or a computer processor that is configured to determine the presence or absence of the genetic variation based on the plurality of paralog ratios.

### Transformations

As noted above, data sometimes is transformed from one form into another form. The terms "transformed," "transformation," and grammatical derivations or equivalents thereof, as used herein refer to an alteration of data from a physical starting material (e.g., test subject and/or reference subject sample nucleic acid) into a digital representation of the physical starting material (e.g., sequence read data), and in some cases includes a further transformation into one or more numerical values or graphical representations of the digital representation that can be utilized to provide an outcome. In certain cases, the one or more numerical values and/or graphical representations of digitally represented data can be utilized to represent the appearance of a test subject's physical genome (e.g., virtually represent or visually represent the presence or absence of a genomic insertion, duplication or deletion; represent the presence or absence of a variation in the physical amount of a sequence associated with medical conditions). A virtual representation sometimes is further transformed into one or more numerical values or graphical representations of the digital representation of the starting material. These methods can transform physical starting material into a numerical value or graphical representation, or a representation of the physical appearance of a test subject's nucleic acid.

In some cases, transformation of a data set facilitates providing an outcome by reducing data complexity and/or data dimensionality. Data set complexity sometimes is reduced during the process of transforming a physical starting material into a virtual representation of the starting material (e.g., sequence reads representative of physical starting material). A suitable feature or variable can be utilized to reduce data set complexity and/or dimensionality. Non-limiting examples of features that can be chosen for use as a target feature for data processing include GC content, fetal gender prediction, fragment size (e.g., length of CCF fragments, reads or a suitable representation thereof (e.g., FRS)), fragment sequence, identification of a copy number alteration, identification of chromosomal aneuploidy, identification of particular genes or proteins, identification of cancer, diseases, inherited genes/traits, chromosomal abnormalities, a biological category, a chemical category, a biochemical category, a category of genes or proteins, a gene ontology, a protein ontology, co-regulated genes, cell signaling genes, cell cycle genes, proteins pertaining to the foregoing genes, gene variants, protein variants, co-regulated genes, co-regulated proteins, amino acid sequence, nucleotide sequence, protein structure data and the like, and combinations of the foregoing. Non-limiting examples of data set complexity and/or dimensionality reduction include; reduction of a plurality of sequence reads to profile plots, reduction of a plurality of sequence reads to numerical values (e.g., normalized values, Z-scores, p-values); reduction of multiple analysis methods to probability plots or single points; principal component analysis of derived quantities; and the like or combinations thereof.

### Genetic variations and medical conditions

The presence or absence of a genetic variation can be determined using a method or apparatus described herein. In certain cases, the presence or absence of one or more genetic variations is determined according to an outcome provided by methods and apparatuses described herein. A genetic variation generally is a particular genetic phenotype present in certain individuals, and often a genetic variation is present in a statistically significant subpopulation of individuals. In some embodiments, a genetic variation is a chromosome abnormality or copy number alteration (e.g., aneuploidy, duplication of one or more chromosomes, loss of one or more chromosomes, partial chromosome abnormality or mosaicism (e.g., loss or gain of one or more regions of a chromosome), translocation, inversion, each of which is described in greater detail herein). Non-limiting examples of genetic variations include one or more copy number alterations, deletions (e.g., microdeletions), duplications (e.g., microduplications), insertions, mutations (e.g., single nucleotide variations), polymorphisms (e.g., single-nucleotide polymorphisms), fusions, repeats (e.g., short tandem repeats), distinct methylation sites, distinct methylation patterns, the like and combinations thereof. An insertion, repeat, deletion, duplication, mutation or polymorphism can be of any length, and in some cases, is about 1 base or base pair (bp) to about 250 megabases (Mb) in length. In some cases, an insertion, repeat, deletion, duplication, mutation or polymorphism is about 1 base or base pair (bp) to about 50,000 kilobases (kb) in length (e.g., about 10 bp, 50 bp, 100 bp, 500 bp, 1 kb, 5 kb, 10kb, 50 kb, 100 kb, 500 kb, 1000 kb, 5000 kb or 10,000 kb in length).

A genetic variation is sometime a deletion. In certain instances, a deletion is a mutation (e.g., a genetic aberration) in which a part of a chromosome or a sequence of DNA is missing. A deletion is often the loss of genetic material. Any number of nucleotides can be deleted. A deletion can comprise the deletion of one or more entire chromosomes, a region of a chromosome, an allele, a gene, an intron, an exon, any non-coding region, any coding region, a part thereof or combination thereof. A deletion can comprise a microdeletion. A deletion can comprise the deletion of a single base.

A genetic variation is sometimes a duplication. In certain instances, a duplication is a mutation (e.g., a genetic aberration) in which a part of a chromosome or a sequence of DNA is copied and inserted back into the genome. In certain cases, a genetic duplication (e.g., duplication) is any duplication of a region of DNA. In some cases, a duplication is a nucleic acid sequence that is repeated, often in tandem, within a genome or chromosome. In some cases, a duplication can comprise a copy of one or more entire chromosomes, a region of a chromosome, an allele, a gene, an intron, an exon, any non-coding region, any coding region, part thereof or combination thereof. A duplication can comprise a microduplication. A duplication sometimes comprises one or more copies of a duplicated nucleic acid. A duplication sometimes is characterized as a genetic region repeated one or more times (e.g., repeated 1, 2, 3, 4, 5, 6, 7, 8, 9 or 10 times). Duplications can range from small regions (thousands of base pairs) to whole chromosomes in some instances. Duplications frequently occur as the result of an error in homologous recombination or due to a retrotransposon event. Duplications have been associated with certain types of proliferative diseases. Duplications can be characterized using genomic microarrays or comparative genetic hybridization (CGH).

A genetic variation is sometimes an insertion. An insertion is sometimes the addition of one or more nucleotide base pairs into a nucleic acid sequence. An insertion is sometimes a microinsertion. In certain cases, an insertion comprises the addition of a region of a chromosome into a genome, chromosome, or part thereof. In certain cases, an insertion comprises the addition of an allele, a gene, an intron, an exon, any non-coding region, any coding region, part thereof or combination thereof into a genome or part thereof. In certain cases, an insertion comprises the addition (e.g., insertion) of nucleic acid of unknown origin into a genome, chromosome, or part thereof. In certain cases, an insertion comprises the addition (e.g., insertion) of a single base.

As used herein a "copy number alteration" generally is a class or type of genetic variation or chromosomal aberration. A copy number alteration also may be referred to as a copy number variation, and can be a deletion (e.g., microdeletion), duplication (e.g., a microduplication) or insertion (e.g., a microinsertion). Often, the prefix "micro" as used herein sometimes is a region of nucleic acid less than 5 Mb in length. A copy number alteration can include one or more deletions (e.g., microdeletion), duplications and/or insertions(e.g., a microduplication, microinsertion) of a part of a chromosome. In certain cases, a duplication comprises an insertion. In certain cases, an insertion is a duplication. In certain cases, an insertion is not a duplication.

In some cases, a copy number alteration is a fetal copy number alteration. Often, a fetal copy number alteration is a copy number alteration in the genome of a fetus. In some cases, a copy number alteration is a maternal and/or fetal copy number alteration. In certain cases, a maternal and/or fetal copy number alteration is a copy number alteration within the genome of a pregnant female (e.g., a female subject bearing a fetus), a female subject that gave birth or a female capable of bearing a fetus. A copy number alteration can be a heterozygous copy number alteration where the alteration (e.g., a duplication or deletion) is present on one allele of a genome. A copy number alteration can be a homozygous copy number alteration where the alteration is present on both alleles of a genome. In some cases, a copy number alteration is a heterozygous or homozygous fetal copy number alteration. In some cases, a copy number alteration is a heterozygous or homozygous maternal and/or fetal copy number alteration. A copy number alteration sometimes is present in a maternal genome and a fetal genome, a maternal genome and not a fetal genome, or a fetal genome and not a maternal genome.

"Ploidy" is a reference to the number of chromosomes present in a subject. In certain cases, "ploidy" is the same as "chromosome ploidy." In humans, for example, autosomal chromosomes are often present in pairs. For example, in the absence of a genetic variation, most humans have two of each autosomal chromosome (e.g., chromosomes 1-22). The presence of the normal complement of 2 autosomal chromosomes in a human is often referred to as euploid or diploid. "Microploidy" is similar in meaning to ploidy. "Microploidy" often refers to the ploidy of a part of a chromosome. The term "microploidy" sometimes is a reference to the presence or absence of a copy number alteration (e.g., a deletion, duplication and/or an insertion) within a chromosome (e.g., a homozygous or heterozygous deletion, duplication, or insertion, the like or absence thereof).

A genetic variation for which the presence or absence is identified for a subject is associated with a medical condition in certain cases. Thus, technology described herein can be used to identify the presence or absence of one or more genetic variations that are associated with a medical condition or medical state. Non-limiting examples of medical conditions include those associated with intellectual disability (e.g., Down Syndrome), aberrant cell-proliferation (e.g., cancer), presence of a micro-organism nucleic acid (e.g., virus, bacterium, fungus, yeast), and preeclampsia.

Non-limiting examples of genetic variations, medical conditions and states are described hereafter.

### Chromosome abnormalities

In some cases, the presence or absence of a chromosome abnormality can be determined by using a method and/or apparatus described herein. Chromosome abnormalities include, without limitation, copy number alterations, and a gain or loss of an entire chromosome or a region of a chromosome comprising one or more genes. Chromosome abnormalities include monosomies, trisomies, polysomies, loss of heterozygosity, translocations, deletions and/or duplications of one or more nucleotide sequences (e.g., one or more genes), including deletions and duplications caused by unbalanced translocations. The term "chromosomal abnormality" or "aneuploidy" as used herein refer to a deviation between the structure of the subject chromosome and a normal homologous chromosome. The term "normal" refers to the predominate karyotype or banding pattern found in healthy individuals of a particular species, for example, a euploid genome (e.g., diploid in humans, e.g., 46,XX or 46,XY). As different organisms have widely varying chromosome complements, the term "aneuploidy" does not refer to a particular number of chromosomes, but rather to the situation in which the chromosome content within a given cell or cells of an organism is abnormal. In some embodiments, the term "aneuploidy" herein refers to an imbalance of genetic material caused by a loss or gain of a whole chromosome, or part of a chromosome. An "aneuploidy" can refer to one or more deletions and/or insertions of a region of a chromosome. The term "euploid," in some cases, refers a normal complement of chromosomes.

The term "monosomy" as used herein refers to lack of one chromosome of the normal complement. Partial monosomy can occur in unbalanced translocations or deletions, in which only a part of the chromosome is present in a single copy. Monosomy of sex chromosomes (45, X) causes Turner syndrome, for example. The term "disomy" refers to the presence of two copies of a chromosome. For organisms such as humans that have two copies of each chromosome (those that are diploid or "euploid"), disomy is the normal condition. For organisms that normally have three or more copies of each chromosome (those that are triploid or above), disomy is an aneuploid chromosome state. In uniparental disomy, both copies of a chromosome come from the same parent (with no contribution from the other parent).

The term "trisomy" as used herein refers to the presence of three copies, instead of two copies, of a particular chromosome. The presence of an extra chromosome 21, which is found in human Down syndrome, is referred to as "Trisomy 21." Trisomy 18 and Trisomy 13 are two other human autosomal trisomies. Trisomy of sex chromosomes can be seen in females (e.g., 47, XXX in Triple X Syndrome) or males (e.g., 47, XXY in Klinefelter's Syndrome; or 47,XYY in Jacobs Syndrome). In some cases, a trisomy is a duplication of most or all of an autosome. In certain cases, a trisomy is a whole chromosome aneuploidy resulting in three instances (e.g., three copies) of a particular type of chromosome (e.g., instead of two instances (e.g., a pair) of a particular type of chromosome for a euploid).

The terms "tetrasomy" and "pentasomy" as used herein refer to the presence of four or five copies of a chromosome, respectively. Although rarely seen with autosomes, sex chromosome tetrasomy and pentasomy have been reported in humans, including XXXX, XXXY, XXYY, XYYY, XXXXX, XXXXY, XXXYY, XXYYY and XYYYY.

The term "mosaicism" as used herein refers to aneuploidy in some cells, but not all cells, of an organism. Certain chromosome abnormalities can exist as mosaic and non-mosaic chromosome abnormalities. For example, certain trisomy 21 individuals have mosaic Down syndrome and some have non-mosaic Down syndrome. Different mechanisms can lead to mosaicism. For example, (i) an initial zygote may have three 21st chromosomes, which normally would result in simple trisomy 21, but during the course of cell division one or more cell lines lost one of the 21st chromosomes; and (ii) an initial zygote may have two 21st chromosomes, but during the course of cell division one of the 21st chromosomes were duplicated. Somatic mosaicism likely occurs through mechanisms distinct from those typically associated with genetic syndromes involving complete or mosaic aneuploidy. Methods and protocols described herein can identify presence or absence of non-mosaic and mosaic chromosome abnormalities.

Following is a non-limiting list of chromosome abnormalities that can be potentially identified by methods, processes, machines and apparatuses described herein.

| **Chromosome** | **Abnormality** | **Disease Association** |
|---|---|---|
| X | XO | Turner's Syndrome |
| Y | XXY | Klinefelter syndrome |
| Y | XYY | Double Y syndrome |
| Y | XXX | Trisomy X syndrome |
| Y | XXXX | Four X syndrome |
| Y | Xp21 deletion | Duchenne's/Becker syndrome, congenital adrenal hypoplasia, chronic granulomatus disease |
| Y | Xp22 deletion | steroid sulfatase deficiency |
| Y | Xq26 deletion | X-linked lymphproliferative disease |
| 1 | 1p (somatic) monosomy trisomy | neuroblastoma |
| 2 | monosomy trisomy 2q | growth retardation, developmental and mental delay, and minor physical abnormalities |
| 3 | monosomy trisomy (somatic) | Non-Hodgkin's lymphoma |
| 4 | monosomy trsiomy (somatic) | Acute non lymphocytic leukaemia (ANLL) |
| 5 | 5p | Cri du chat; Lejeune syndrome |
| 5 | 5q (somatic) monosomy trisomy | myelodysplastic syndrome |
| 6 | monosmy trisomy (somatic) | clear-cell sarcoma |
| 7 | 7 q 11.23 deletion | William's syndrome |
| 7 | monosomy trisomy | monosomy 7 syndrome of childhood; somatic: renal cortical adenomas; myelodysplastic syndrome |
| 8 | 8q24.1 deletion | Langer-Giedon syndrome |
| 8 | monosomy trisomy | myelodysplastic syndrome; Warkany syndrome; somatic: chronic myelogenous leukemia |
| 9 | monosomy 9p | Alfi's syndrome |
| 9 | monosomy 9p partial trisomy | Rethore syndrome |
| 9 | trisomy | complete trisomy 9 syndrome; mosaic trisomy 9 syndrome |
| 10 | Monosomy trisomy (somatic) | ALL or ANLL |
| 11 | 11p- | Aniridia; Wilms tumor |
| 11 | 11q- | Jacobson Syndrome |
| 11 | monosomy (somatic) trisomy | myeloid lineages affected (ANLL, MDS) |
| 12 | monosomy trisomy (somatic) | CLL, Juvenile granulosa cell tumor (JGCT) |
| 13 | 13q- | 13q-syndrome; Orbeli syndrome |
| 13 | 13q14 deletion | retinoblastoma |
| 13 | monosomy trisomy | Patau's syndrome |
| 14 | monsomy trisomy (somatic) | myeloid disorders (MDS, ANLL, atypical CML) |
| 15 | 15q11-q13 deletion monosomy | Prader-Willi, Angelman's syndrome |
| 15 | trisomy (somatic) | myeloid and lymphoid lineages affected, e.g., MDS, ANLL, ALL, CLL) |
| 16 | 16q13.3 deletion | Rubenstein- Taybi |
| | monosomy trisomy (somatic) | papillary renal cell carcinomas (malignant) |
| 17 | 17p-(somatic) | 17p syndrome in myeloid malignancies |
| 17 | 17q11.2 deletion | Smith-Magenis |
| 17 | 17q13.3 | Miller-Dieker |
| 17 | monosomy trisomy (somatic) | renal cortical adenomas |
| 17 | 17p11.2-12 trisomy | Charcot-Marie Tooth Syndrome type 1; HNPP |
| 18 | 18p- | 18p partial monosomy syndrome or Grouchy Lamy Thieffry syndrome |
| 18 | 18q- | Grouchy Lamy Salmon Landry Syndrome |
| 18 | monosomy trisomy | Edwards Syndrome |
| 19 | monosomy trisomy | |
| 20 | 20p- | trisomy 20p syndrome |
| 20 | 20p11.2-12 deletion | Alagille |
| 20 | 20q- | somatic: MDS, ANLL, polycythemia vera, chronic neutrophilic leukemia |
| 20 | monosomy trisomy (somatic) | papillary renal cell carcinomas (malignant) |
| 21 | monosomy trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
| 22 | monosomy trisomy | complete trisomy 22 syndrome |

In certain cases, presence or absence of a fetal chromosome abnormality is identified (e.g., trisomy 21, trisomy 18 and/or trisomy 13). Presence or absence of one or more of the chromosome abnormalities described in the table above may be identified in some cases.

### Medical disorders and medical conditions

Methods described herein can be applicable to any suitable medical disorder or medical condition. Non-limiting examples of medical disorders and medical conditions include cell proliferative disorders and conditions, wasting disorders and conditions, degenerative disorders and conditions, autoimmune disorders and conditions, pre-eclampsia, chemical or environmental toxicity, liver damage or disease, kidney damage or disease, vascular disease, high blood pressure, and myocardial infarction.

### Preeclampsia

In some cases, the presence or absence of preeclampsia is determined by using a method or apparatus described herein. Preeclampsia is a condition in which hypertension arises in pregnancy (e.g., pregnancy-induced hypertension) and is associated with significant amounts of protein in the urine. In certain instances, preeclampsia may be associated with elevated levels of extracellular nucleic acid and/or alterations in methylation patterns. For example, a positive correlation between extracellular fetal-derived hypermethylated RASSF1A levels and the severity of pre-eclampsia has been observed. In certain instances, increased DNA methylation is observed for the H19 gene in preeclamptic placentas compared to normal controls.

### Markers

In certain instances, a polynucleotide in abnormal or diseased cells is modified with respect to nucleic acid in normal or non-diseased cells (e.g., single nucleotide variation, copy number variation). In some instances, a polynucleotide is present in abnormal or diseased cells and not present in normal or non-diseased cells, and sometimes a polynucleotide is not present in abnormal or diseased cells and is present in normal or non-diseased cells. Thus, a marker sometimes is a single nucleotide variation and/or a copy number variation (e.g., a differentially expressed DNA or RNA (e.g., mRNA)). For example, patients with metastatic diseases may be identified by cancer-specific markers and/or certain single nucleotide polymorphisms or short tandem repeats, for example. Non-limiting examples of cancer types that may be positively correlated with elevated levels of circulating DNA include breast cancer, colorectal cancer, gastrointestinal cancer, hepatocellular cancer, lung cancer, melanoma, non-Hodgkin lymphoma, leukemia, multiple myeloma, bladder cancer, hepatoma, cervical cancer, esophageal cancer, pancreatic cancer, and prostate cancer. Various cancers can possess, and can sometimes release into the bloodstream, nucleic acids with characteristics that are distinguishable from nucleic acids from non-cancerous healthy cells, such as, for example, epigenetic state and/or sequence variations, duplications and/or deletions. Such characteristics can, for example, be specific to a particular type of cancer. Accordingly, methods described herein sometimes provide an outcome based on determining the presence or absence of a particular marker, and sometimes an outcome is presence or absence of a particular type of condition (e.g., a particular type of cancer).

### Compositions

Also provided herein are compositions for detecting the presence or absence of a genetic variation, comprising a mixture of a plurality of amplification primer pairs that can amplify the plurality of sets of paralogous polynucleotide species disclosed herein. Each primer of the plurality of amplification primer pairs is complementary to less than 20 positions in a human genome. In some embodiments, the amplification primers for each of the paralogous polynucleotide species sets produces two equal-sized amplicons with a size between 60 to 100 bp. In some embodiments, the amplification primers for each of the sets produces zero off-target amplification events allowing maximal 3-base pair mis-priming. In some embodiments, the amplification primers for each of the sets do not overlap with any annotated single nucleotide variant with greater than 1% minor allele frequency in dbSNP build137.

In certain cases, certain molecules (e.g., nucleic acid primers, amplified DNA products, etc.) used in accordance with and/or provided by the disclosure comprise one or more detectable entities or moieties, i.e., such molecules are "labeled" with such entities or moieties.

Any of a wide variety of detectable agents can be used in the practice of the disclosure. Suitable detectable agents include, but are not limited to: various ligands, radionuclides; fluorescent dyes; chemiluminescent agents (such as, for example, acridinum esters, stabilized dioxetanes, and the like); bioluminescent agents; spectrally resolvable inorganic fluorescent semiconductors nanocrystals (i.e., quantum dots); microparticles; metal nanoparticles (e.g., gold, silver, copper, platinum, etc.); nanoclusters; paramagnetic metal ions; enzymes; colorimetric labels (such as, for example, dyes, colloidal gold, and the like); biotin; dioxigenin; haptens; and proteins for which antisera or monoclonal antibodies are available.

In some cases, the detectable moiety is biotin. Biotin can be bound to avidins (such as streptavidin), which are typically conjugated (directly or indirectly) to other moieties (e.g., fluorescent moieties) that are detectable themselves.

Below are described some non-limiting examples of some detectable moieties that may be used.

### Fluorescent dyes

In certain cases, a detectable moiety is a fluorescent dye. Numerous known fluorescent dyes of a wide variety of chemical structures and physical characteristics are suitable for use in the practice of the disclosure. A fluorescent detectable moiety can be stimulated by a laser with the emitted light captured by a detector. The detector can be a charge-coupled device (CCD) or a confocal microscope, which records its intensity.

Suitable fluorescent dyes include, but are not limited to, fluorescein and fluorescein dyes (e.g., fluorescein isothiocyanine or FITC, naphthofluorescein, 4',5'-dichloro-2',7'-dimethoxyfluorescein, 6-carboxyfluorescein or FAM, etc.), carbocyanine, merocyanine, styryl dyes, oxonol dyes, phycoerythrin, erythrosin, eosin, rhodamine dyes (e.g., carboxytetramethyl-rhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (e.g., methoxycoumarin, dialkylaminocoumarin, hydroxycoumarin, aminomethylcoumarin (AMCA), etc.), Q-DOTS. Oregon Green Dyes (e.g., Oregon Green 488, Oregon Green 500, Oregon Green 514., etc.), Texas Red, Texas Red-X, SPECTRUM REDTM, SPECTRUM GREENTM, cyanine dyes (e.g., CY-3TM, CY-5TM, CY-3.5TM, CY5.5TM, etc.), ALEXA FLUORTM dyes (e.g., ALEXA FLUORTM 350, ALEXA FLUORTM 488, ALEXA FLUORTM 532, ALEXA FLUORTM 546, ALEXA FLUORTM 568, ALEXA FLUORTM 594, ALEXA FLUORTM 633, ALEXA FLUORTM 660, ALEXA FLUORTM 680, etc.), BODIPYTM dyes (e.g., BODIPYTM FL, BODIPYTM R6G, BODIPYTM TMR, BODIPYTM TR, BODIPYTM 530/550, BODIPYTM 558/568, BODIPYTM 564/570, BODIPYTM 576/589, BODIPYTM 581/591, BODIPYTM 630/650, BODIPYTM 650/665, etc.), IRDyes (e.g., IRD40, IRD 700, IRD 800, etc.), and the like. For more examples of suitable fluorescent dyes and methods for coupling fluorescent dyes to other chemical entities such as proteins and peptides, see, for example, "The Handbook of Fluorescent Probes and Research Products", 9th Ed., Molecular Probes, Inc., Eugene, OR. Favorable properties of fluorescent labeling agents include high molar absorption coefficient, high fluorescence quantum yield, and photostability. In some cases, labeling fluorophores exhibit absorption and emission wavelengths in the visible (i.e., between 400 and 750 nm) rather than in the ultraviolet range of the spectrum (i.e., lower than 400 nm).

A detectable moiety may include more than one chemical entity such as in fluorescent resonance energy transfer (FRET). Resonance transfer results an overall enhancement of the emission intensity. For instance, see Ju et. al. (1995) Proc. Nat'l Acad. Sci. (USA) 92:4347. To achieve resonance energy transfer, the first fluorescent molecule (the "donor" fluor) absorbs light and transfers it through the resonance of excited electrons to the second fluorescent molecule (the "acceptor" fluor). In one approach, both the donor and acceptor dyes can be linked together and attached to the oligo primer. Methods to link donor and acceptor dyes to a nucleic acid have been described, for example, in U.S. Pat. No. 5,945,526 to Lee et al.. Donor/acceptor pairs of dyes that can be used include, for example, fluorescein/tetramethylrohdamine, IAEDANS/fluroescein, EDANS/DABCYL, fluorescein/fluorescein, BODIPY FL/BODIPY FL, and Fluorescein/ QSY 7 dye. See, e.g., U.S. Pat. No. 5,945,526 to Lee et al. Many of these dyes also are commercially available, for instance, from Molecular Probes Inc. (Eugene, Oreg.). Suitable donor fluorophores include 6- carboxyfluorescein (FAM), tetrachloro-6-carboxyfluorescein (TET), 2'-chloro-7'-phenyl-1,4- dichloro-6-carboxyfluorescein (VIC), and the like.

### Radioactive isotopes

In certain cases, a detectable moiety is a radioactive isotope. For example, a molecule may be isotopically-labeled (i.e., may contain one or more atoms that have been replaced by an atom having an atomic mass or mass number different from the atomic mass or mass number usually found in nature) or an isotope may be attached to the molecule. Non-limiting examples of isotopes that can be incorporated into molecules include isotopes of hydrogen, carbon, fluorine, phosphorous, copper, gallium, yttrium, technetium, indium, iodine, rhenium, thallium, bismuth, astatine, samarium, and lutetium (i.e., 3H, 13C, 14C, 18F, 19F, 32P, 35S, 64Cu, 67Cu, 67Ga, 90Y, 99mTc, 111In, 1251, 1231, 1291, 1311, 1351, 186Re, 187Re, 201T1, 212Bi, 213Bi, 21lAt, 153Sm, 177Lu).

In some cases, signal amplification is achieved using labeled dendrimers as the detectable moiety (see, e.g., Physiol Genomics 3:93-99, 2000). Fluorescently labeled dendrimers are available from Genisphere (Montvale, N.J.). These may be chemically conjugated to the oligonucleotide primers by methods

### Kits

Also provided herein are kits for determining the presence of absence of a genetic variation. The kit comprises a mixture of a plurality of pairs of amplification primer pairs, wherein each pair amplifies a set of paralogous sequences disclosed herein. Each primer of the amplification primer pairs is complementary to less than 20 positions in a human genome. In certain embodiments, the plurality of amplification primer pairs are selected from the amplification primer pairs disclosed in Figure 8. In some embodiments, the kit comprises a DNA polymerase. The kit may optionally comprise reagents for amplifying a plurality set of paralogous sequences.

In some cases, kits comprise paralog sequences and associated primers of interest. In some cases, kits are provided in a form of an array such as a microarray or a mutation panel. In some cases, provided kits further comprise reagents for carrying out various detection methods described herein (e.g., sequencing, hybridization, etc.). For example, kits may optionally contain buffers, enzymes, containers and/or reagents for use in methods described herein.

In some cases, provided kits further comprise a control indicative of a healthy individual, e.g., a nucleic acid and/or protein sample from an individual who does not have the genetic variant, and/or genetic disease and/or syndrome of interest. Kits may also contain instructions on how to determine if an individual has the disease and/or syndrome of interest, or is at risk of developing the disease and/or syndrome of interest.

In some cases, provided is a computer readable medium encoding information corresponding to the biomarker of interest. Such computer readable medium may be included in a kit.

### Methods of Making Paralog Contig Assay Systems

Also provided are methods of generating paralog assay systems. The method may comprise: identifying paralogous contigs on a first reference chromosome and a second target chromosome; extracting those sequences which are almost identical in sequence and that map to exactly two regions, one region on the first reference chromosome and one region on the second target chromosome; and merging the sequences to form paralog contigs.

In an case, all potential paralogous contigs located on the second target chromosome (e.g., chromosome 21) and alternative autosomes (reference chromosomes) are identified *in silico* by sampling each target chromosome and reference chromosome at 10 base pair intervals in 100 base pair segments. Or, other similar intervals (e.g., 10, 15, 20 base pairs, etc.) and segments (e.g., 75, 100, 150, 200 base pairs) may be used. In an case, each segment may then be aligned to the human reference genome (e.g., hg19, GRCh37). In an case, the alignment may comprise using Bowtie version 2.1.0 [34]. Or, other alignment methods may be used. Segments which map to exactly two regions, one on the target chromosome and one on a reference chromosome, may then be extracted and the corresponding regions merged to obtain paralogous contigs. In certain cases, each paralogous pair may be globally aligned to locate nucleotide differences distinguishing target and reference paralogs and to obtain the consensus sequence. For example, in certain cases the sequences may be aligned using the Biostrings function 'pairwise Alignment' [32] in R version 3.0 [36]. Or, other alignment methods may be used.

The method may further comprise designing primers that amplify, but differentiate, the contigs from both the reference and the target chromosome. Primer design may include consideration of GC content, melting temperature, and multiplexing optimization. In an case, to ensure that all primer pairs are specific to target and reference paralogs and to accommodate the short length of cfDNA, only the primer pairs that produce two equal-sized amplicons with a size between 60-100 bp are selected. In an case, the designed primers may be filtered to ensure desired sequence properties and to reduce possible sources of variation in the context of multiplexed PCR. In certain cases, the final filtered set of paralog targeting primers satisfy at least some, if not all of the following selection criteria: (1) each primer pair is predicted to produce exactly two equal-sized amplicons between 60-100 bp *in silico,* (2) each primer pair is predicted to produce zero off-target amplification events allowing maximal 3-base mis-priming , (3) each individual primer sequence is complementary to less than 20 positions in the genome, (4) no primer overlaps with any annotated single nucleotide variant with >1% minor allele frequency (e.g., using dbSNP build137 [39] or other similar analysis), (5) primers flank at least one position that distinguished target from reference paralogs, and (6) all independent amplicons are at least 100-bp apart.

Certain methods described herein may be performed in conjunction with methods described, for example in International Patent Application Publication No. WO2013/052913, International Patent Application Publication No. WO2013/052907, International Patent Application Publication No. WO2013/055817, International Patent Application Publication No. WO2013/109981, International Patent Application Publication No. WO2013/177086, International Patent Application Publication No. WO2013/192562, International Patent Application Publication No. WO2014/116598, International Patent Application Publication No. WO2014/055774, International Patent Application Publication No. WO2014/190286, International Patent Application Publication No. WO2014/205401, International Patent Application Publication No. WO2015/051163, International Patent Application Publication No. WO2015/138774, International Patent Application Publication No. WO2015/054080, International Patent Application Publication No. WO2015/183872, International Patent Application Publication No. WO2016/019042, and International Patent Application Publication No. WO 2016/057901.

### EXAMPLES

The examples set forth below illustrate certain embodiments and do not limit the technology.

### Example 1: Targeted paralog sequencing

The following example describes a method for NIPT based on a targeted sequencing strategy specifically enriching for paralogous motifs on chromosomes of interest for fetal aneuploidy detection, chromosome 18 and 21. In this study, targeted paralog assay libraries were prepared for 1334 total samples as part of both classification training and a blinded evaluation dataset. Of these, 480 samples were fully blinded with an unknown composition of fetal euploid and aneuploid samples. Using a conservative classification strategy, all fetal aneuploidy cases were correctly classified, resulting in 100% sensitivity and specificity for fetal chromosome 21 trisomies and 87.5% sensitivity and 100% specificity for fetal chromosome 18 trisomies. Notably, these results were achieved at less than 25% of the standard depth of sequencing for a single sample using genome-wide sequencing strategies for aneuploidy detection.

### MATERIALS AND METHODS

### Identification and filtering of paralogous contigs

All potential paralogous contigs located on either chromosome 18 or chromosome 21 (target chromosomes) and alternative autosomes (reference chromosomes) were identified *in silico* by sampling each target chromosome at 10 base pair intervals in 100 base pair segments. Each segment was aligned to the human reference genome (hg19, GRCh37) using Bowtie version 2.1.0 [34]. Segments which mapped to exactly two regions, one on the target chromosome and one on a reference chromosome, were extracted and the corresponding regions were merged to obtain paralogous contigs. Each paralogous pair was then globally aligned using the Biostrings function 'pairwiseAlignment' [32] in R version 3.0 [36] to locate nucleotide differences distinguishing target and reference paralogs and to obtain the consensus sequence.

### Design of multiplexed PCR primer sequences

Primer 3 version 2.2.3 [37:38] was used to design PCR primer pairs surrounding each nucleotide that differentiates the target and reference paralogs. The primer design parameters were: primer GC content = 30-70%, primer size = 18-24 bp, optimal size = 20 bp, primer melting temperature = 52-64°C, optimal Tm = 60°C. All resulting primer pairs were aligned as paired-end reads to the reference genome (hg19, GRCh37) using Bowtie version 2.1.0 [34] and required perfect primer matching to the reference. To ensure that all primer pairs were specific to target and reference paralogs and to accommodate the short length of cfDNA, only the primer pairs that produced two equal-sized amplicons with a size between 60-100bp were selected.

The designed primers were then filtered to ensure desired sequence properties and to reduce possible sources of variation in the context of multiplexed PCR. The final filtered set of paralog targeting primers satisfied the following selection criteria: (1) each primer pair was predicted to produce exactly two equal-sized amplicons between 60-100bp *in silico,* (2) each primer pair was predicted to produce zero off-target amplification events allowing maximal 3-base mis-priming , (3) each individual primer sequence was complementary to less than 20 positions in the genome, (4) no primer overlapped with any annotated single nucleotide variant with >1% minor allele frequency in dbSNP build137 [39], (5) primers flanked at least one position that distinguished target from reference paralogs, and (6) all independent amplicons were at least 100-bp apart.

Additional PCR primers were designed to amplify selected chrY regions and single nucleotide polymorphisms (SNPs) for the determination of fetal sex, the amount of input DNA, and estimation of the fraction of fetal origin DNA, respectively. All primer specifications were similar to those described above with the exception of filter criteria specific to paralog state of amplicons.

To minimize undesired primer-primer interactions in the multiplexed PCR reaction, *in silico* multiplexing optimization was performed. The reverse complement of each primer sequence was locally aligned to all the other primers using the Biostrings function 'pairwiseAlignment' [35] in R version 3.0 [36] and a primer-specific alignment score was calculated. A "hub" assay, defined as the assay with the largest total alignment score, was identified and removed from the primer pool. Hub assays with the maximum predicted primer-primer alignment score were iteratively eliminated from the primer pool until the number of remaining assays reached a minimum remaining assay threshold of 450, 450, 10, and 150 assays for chromosome 21 paralogs, chromosome 18 paralogs, chromosome Y fetal sex assays, and SNPs, respectively.

### Sample acquisition and blood processing

Whole blood (10 mL per aliquot) was collected and plasma separated as previously described [15]. Two aliquots of plasma were obtained from each individual and were processed independently.

### Nucleic acid extraction

After blood processing, cfDNA was extracted using an automated system capable of extracting 96 samples in parallel using an optimized version of the manufacturer's protocol (AMPure XP; Beckman Coulter).

### Library preparation

Library preparation was accomplished through a two-phase PCR-based targeted enrichment process. Extracted cfDNA was used as template for single-well, 1060-plex PCR reactions. Following amplification, samples were purified using AMPure XP magnetic beads (Beckman Coulter) in a semi-automated process implemented on Zephyr liquid handling platforms (Caliper LifeSciences). Purified samples were quantified as described previously [8]. Purified and normalized products were then used as template for a subsequent PCR reaction using partial sequencing adapter motifs introduced during multiplexed PCR as priming sites. Final library samples were then purified, quantified, and normalized as described above.

### Massively parallel sequencing

Isomolar paralog-targeted libraries (n=96) were sequenced together on two lanes of a HiSeq Rapid V1 flowcell on a HiSeq2500 instrument (Illumina). Sequencing by synthesis was performed for 80 cycles followed by 8 additional cycles to read each sample index. Data were merged across both lanes of each flowcell prior to further processing.

### Sequencing metrics

All BCL output files from the sequencing instrument were converted to FASTQ format and aligned to the human reference genome (hg19, GRCh37) using Bowtie version 2.1.0 [34]. Subsequent processing to evaluate paralog targeting library quality and chromosome 18 or chromosome 21 dosage was performed using custom scripts to calculate the following metrics:

### On-target rate

On-target rate was calculated as the fraction of reads mapped to expected genomic coordinates out of all sequencing reads. On-target reads were additionally filtered to require that (1) read length ≥45 bp, (2) mapped position of the 5'-end of the read was within 5 bp of the 5'-end of a designated target region, and (3) that reads were not ambiguously aligned.

### Ambiguous reads

Ambiguously aligned reads were defined as reads that could be aligned to more than one position with equivalent confidence (i.e. the best alignment and the second best alignment had the same alignment score). Ambiguously aligned reads may have arisen due to reference genome uncertainty in paralogous regions, unpredicted amplification targets for paralog primers, or process errors. All ambiguously aligned reads were removed from further analysis.

### Assay paralog ratio

The paralog ratio of an assay was calculated as the ratio of target paralog read depth to reference paralog read depth. Ratios were logarithm-transformed to zero-center values for additional analyses.

### Assay MAD

Assay median absolute deviation (MAD) was calculated in the base R package [36] as the median absolute deviation of logarithm-transformed assay ratios in euploid samples.

### Aneuploidy classification approaches

### Z-score calculation

To calculate the z-score of a selected assay, the logarithm-transformed paralog assay ratio was first z-scaled by offsetting by the median and scaling by the MAD values derived from reference euploid samples. The sample level z-score was then calculated by combining individual assay z-scores with the exclusion of outliers of z > 4^{∗}MAD or < -4MAD from the distribution center: $Z = \frac{{\sum }_{i = 1}^{N} {z}_{i}}{\sqrt{N}}$, where *zᵢ* is the z-score of a non-outlier assay.

A variation to the above calculation is the weighted z-score, which was calculated by weighting individual non-outlier z-scores according to assay MAD or discriminatory power. ${Z}_{w} = \frac{{\sum }_{i = 1}^{N} {w}_{i} {z}_{i}}{\sqrt{{\sum }_{i = 1}^{N} {w}_{i}^{2}}}$, where *wᵢ* is the weight for each individual assay.

### Machine learning based aneuploidy classification

An artificial neural network approach was additionally implemented for aneuploidy classification using R package neuralnet [40] in R version 3.0 [36]. Prior to training of classification models, all assays were first filtered to require a minimum of 150 reads and no observed copy number variant (CNV) indication. Potential batch-level process bias was removed by offsetting assay ratios by plate-specific medians. To reduce model complexity and avoid over fitting, principal component analysis (PCA) was applied to transform the data into principal coordinate space. Two to fifteen high dimension principal components were then used to train alternative classification models and three-fold cross validation was used to select the best classification model.

### Fetal sex determination

Fetal sex was determined based on the proportion of sequencing reads aligning to chromosome Y, calculated as the total sequencing read depth of chromosome Y assays normalized by total depth of all assays. The chromosome Y percentage is expected to be elevated in male, relative to female, samples.

### Estimation of the fraction of fetal fraction

The fraction of fetal (placental) DNA for each sample was calculated based on the sequencing read counts of the 150 SNP assays. All SNP assays with fewer than 100 sequencing reads were removed from analysis. Maternal homozygous and fetal heterozygous SNP loci were identified by k-means clustering implemented in the kmeans base function in R version 3.0 [36]. The fetal fraction was then estimated using the median observed frequency of all the identified SNPs with the above genotype. Estimates of fetal fraction for each sample were compared against estimates from whole-genome MaterniT21^{®} PLUS laboratory developed test sequence data using published methods [41].

### RESULTS

### Blinded paralog assay performance evaluation

The performance of targeted sequencing of paralogs for fetal aneuploidy detection was evaluated in 768 maternal plasma DNA samples with results available from genome-wide sequencing using the MaterniT21^{®} PLUS laboratory developed test. Of these samples, 288 were fetal euploid samples as determined by MaterniT21^{®} PLUS results and provided a null distribution of assay performance parameters. An additional 480 samples were blinded, research consented samples containing unknown numbers of fetal chromosome 21 and chromosome 18 trisomies to determine assay performance.

Global sequencing metrics for performance evaluation samples are summarized in Table 1. 576 independent samples were processed in addition to the 768 samples described above for the purpose of assay selection and training of classification models. Data include a set of euploid samples to provide a null distribution of assay performance and a set of blinded fully blinded samples of varied fetal genotype. MaterniT21^{®} PLUS Assay ground truth data were obtained for all included samples. Sequencing read depth of performance evaluation samples was slightly reduced relative to the assay selection and classification model training dataset. Similarly, assay variance was slightly increased; however, the paralog assay ratio remained constant between the two datasets.

**TABLE 1.**

| | Total Reads (millions) | Aligned reads (%) | On-target reads (%) |
|---|---|---|---|
| Presumed euploids (N=240) | 2.121 | 95.3 | 83.3 |
| Blinded samples (N=480) | 2.265 | 95.2 | 83.4 |

### Trisomy classification results

Based on previous experimental and empirical observations, the following acceptance criteria were used for sample quality control: sequencing read counts >1.2 million, on-target rate >75%, and fetal DNA fraction >4%. Of the 480 blinded samples, two were excluded due to insufficient sequencing read counts and five samples were excluded due to insufficient on-target rate. Notably, on-target rates for the five on-target rate excluded samples ranged from 72-75% and could potentially have been classified; however, due to the lack of empirical data to determine a statistically significant threshold for on-target rate, a heuristic threshold was utilized. The exclusion of these seven samples left 473 total blinded samples for classification.

The union of z-scores and artificial neural network results were utilized to classify samples as fetal euploid, fetal trisomy 21, or fetal trisomy 18. All classifications were determined independently by two laboratory directors who evaluated the z-scores and artificial neural network results independently to provide curated classification results prior to sample unblinding. After laboratory director classification, z-scores and fetal fraction values of all putative fetal trisomy samples were used to create linear models for chromosomes 18 and 21. All putative non-trisomy samples with differences in expected z-scores and observed z-score that are within three times the MAD of the residual model distributions were reported without classification. In total, five samples classified by lab directors were reassigned as unclassified using this method. The final classification was thus based on a conservative classification strategy such that only concordant calls were reported. Classification results were considered with respect to MaterniT21^{®} PLUS Assay results as ground truth.

The conservative classification scheme pursued here resulted in 41 of 473 blinded samples without unambiguous fetal aneuploidy classification results, or a "no-call" rate of 8.7%. Of these ambiguously classified samples, 8, 3, and 30 were identified as fetal trisomy 21, fetal trisomy 18, and fetal euploid cases, respectively; consequently, the set of samples without unambiguous fetal aneuploidy classification results was enriched for 26.8% fetal trisomy cases versus 12.3% fetal trisomy cases in the study population. For samples with fetal aneuploidy classification results, the union of all statistical metrics provided perfectly concordant classification results for fetal trisomy 21 samples with zero false positive or false negative samples (Table 2 - Fetal aneuploidy classification results). Of the eight samples defined by the MaterniT21^{®} PLUS LDT as fetal trisomy 21 cases that could not be unambiguously classified using the targeted paralog strategy described here, six had a relatively low estimated fetal fraction (<8%), which would be expected to reduce signal, but were otherwise undistinguished; the remaining two samples without classification results were flagged due to ambiguity in the fetal trisomy 18 classification and might have otherwise been correctly classified. Similarly, the classification of fetal trisomy 18 samples yielded zero false positives, but two false negatives which both had marginally low fetal fraction (<8%) and average z-score classification results (2.45-2.47). Classification results are summarized in Figure 2.

**TABLE 2.**

| Targeted Paralog Result | | MaterniT21^{®} PLUS Result | | |
|---|---|---|---|---|
| SAMPLE ACCEPTANCE | Total | Euploid | T18 | T21 |
| Failed fetal DNA fraction | 0 | 0 | 0 | 0 |
| Failed read count | 2 | 2 | 0 | 0 |
| Failed on-target rate | 5 | 3 | 1 | 1 |
| Total Samples | 480 | 420 | 20 | 40 |
| Accepted samples | 473 | 415 | 19 | 39 |
| | | | | |

| SAMPLE CLASSIFICATION | Total | Euploid | T18 | T21 |
|---|---|---|---|---|
| Ambiguous Chr 21 and 18 | 2 | 1 | 0 | 1 |
| Ambiguous Chr 21 | 25 | 19 | 0 | 6 |
| Ambiguous Chr 18 | 14 | 10 | 3 | 1 |
| Fetal Chr 21 trisomy | 31 | 0 | 0 | 31 |
| Fetal Chr 18 trisomy | 14 | 0 | 14 | 0 |
| Fetal euploid | 387 | 385 | 2 | 0 |

The reduced sensitivity in classification of fetal trisomy 18 relative to fetal trisomy 21 may be due to increased noise observed in chromosome 18 assays and inadequately trained classification models for fetal trisomy 18 detection due to the small training set sample size. In comparison, MaterniT21^{®} PLUS Assay results yielded z-scores of 10.66 and 13.99 for the two abovementioned unclassified samples and the overall z-scores were higher from the MaterniT21 ^{®} PLUS LDT than those generated from targeted paralog assays, representative of a larger classification metric gap between affected and unaffected samples when using a genome-wide approach (Figure 2). These observations illustrate the challenges with sub-genomic targeted approaches compared to a genome-wide sequencing strategy for noninvasive fetal aneuploidy classification.

### Fetal sex determination

Chromosome Y representation was transformed into a standard normal distribution by z-scaling. The offset and scaling factors were set by the flowcell specific median and MAD of chromosome Y sequencing read representation of all samples <0.07%, respectively. Of the 473 total blinded samples passing acceptance criteria, one discordant fetal sex classification and four ambiguous fetal sex classifications based on z-scores were observed (Table 3- Fetal sex classification results). The sample with discordant fetal sex classification was classified as unreportable based on discordant z-score and risk-score trisomy classification results. Summary of classification results with respect to MaterniT21^{®} PLUS. Reported results are derived from a heuristic threshold for percentage of sequencing reads mapped to chromosome Y assays. All four unclassified fetal sex samples had fetal fraction <7.5% (Figure 3).

**TABLE 3.**

| Targeted Paralog Result | | MaterniT21^{®} PLUS Result | |
|---|---|---|---|
| SAMPLE ACCEPTANCE | Total | Male fetus | Female fetus |
| Failed fetal DNA fraction | 0 | 0 | 0 |
| Failed read count | 2 | 2 | 0 |
| Failed on-target rate | 5 | 1 | 4 |
| Total Samples | 480 | 240 | 240 |
| Accepted samples | 473 | 237 | 236 |

| SAMPLE CLASSIFICATION | Total | Male fetus | Female fetus |
|---|---|---|---|
| Ambiguous fetal sex | 4 | 4 | 0 |
| Male fetus | 234 | 233 | 1 |
| Female fetus | 235 | 0 | 235 |

### Reproducibility of targeted paralog based fetal aneuploidy detection

The blinded sample targeted paralog libraries were re-sequenced to test the reproducibility of observed results and to evaluate the impact of variable sequencing depth on assay performance. The original experiment yielded a mean of 2.2 million sequencing reads per sample while the re-sequenced experiment yielded a mean of 1.27 million sequencing reads with similar on-target rates of 83%. Chromosome Y fraction was highly concordant between the two sequencing experiments (r² = 0.99, p<10⁻¹⁵ , Figure 4A). A similarly high concordance of estimated fraction of fetal origin DNA was observed (r² = 0.97, p<10⁻¹⁵, Figure 4B). In both of the above cases, discordant data points can be attributed to QC failures due to insufficient read count and/or on-target rate. Finally, the calculated chromosome 21 and chromosome 18 z-scores show high concordance from experiment to experiment (Chromosome 21: r² = 0.87, p<10⁻¹⁵, Figure 4C. Chromosome 18: r² = 0.75, p<10⁻¹⁵ , Figure 4D). Jointly, these results suggest that assay performance is robust to variation in sequencing performance.

### DISCUSSION

This study applied a targeted sequencing strategy specifically enriching for paralogous motifs anchored on chromosomes of interest (chromosomes 18 and 21) for fetal aneuploidy detection.

In this study, targeted paralog assay libraries for 1334 total samples were prepared as part of both classification training and a blinded evaluation dataset. Of these, 480 samples were fully blinded with an unknown composition of fetal euploid and aneuploid samples. Using a conservative classification strategy, all fetal aneuploidy cases were correctly classified, resulting in 100% sensitivity and specificity for fetal chromosome 21 trisomies and 87.5% sensitivity and 100% specificity for fetal chromosome 18 trisomies. Notably, these results were achieved at less than 25% of the standard depth of sequencing for a single sample using genome-wide sequencing strategies for aneuploidy detection. However, an unambiguous classification result was returned for only 432 of 480 fully blinded samples, reflecting a combined 10% QC failure and ambiguous classification rate. While this result is similar to some other published methods for targeted sequencing based non-invasive fetal aneuploidy detection [14, 42], it remains an order of magnitude higher than published genome-wide sequencing fetal aneuploidy detection strategies [12].

The targeted paralog sequencing strategy described here offers several distinct advantages for non-invasive fetal aneuploidy detection. First and foremost, the sequencing requirements and cost per sample are reduced relative to a genome-wide sequencing assay. Further, the workflow for preparation and sequencing of such libraries is amenable to full automation, potentially facilitating extremely high-throughput screening of cfDNA samples in a clinical laboratory environment. Finally, paralog ratio testing provides additional content relative to simple counting metrics, is a more direct strategy than SNP-based testing, and offers the capacity to effectively self-normalize each targeted paralog through the calculation of locus-specific paralog ratios to reduce some of the noise inherent in sequencing datasets.

The assay described herein specifically enriches for paralog targets used for fetal aneuploidy detection in maternal cfDNA. This assay was found to have high sensitivity and specificity for fetal chromosome 18 and 21 trisomies, but to suffer from a relatively high rate of ambiguous classification. These data demonstrate that a target enrichment strategy selecting for paralogous motifs in cfDNA samples is technically feasible for high-throughput classification of fetal aneuploidies from maternal blood samples, correctly classifying >95% of all fetal aneuploidies tested with no false positive results observed.

### Example 2: Assay selection and classification model training

The performance of all available paralog assays was first assessed in a training data set consisting of NGS libraries previously prepared from 341 fetal euploid, 200 fetal chromosome 21 triploid, and 35 fetal chromosome 18 triploid cfDNA samples. The objectives for these data were four-fold: (1) to evaluate the ability to determine fetal sex, (2) to evaluate the capacity to estimate fetal fraction, (3) to select the best performing assays, and (4) to train fetal aneuploidy classification models. Global sequencing metrics for samples included in the assay selection and classification model training set are included in Table 4. Data include model training set with MaterniT21^{®} PLUS Assay ground truth data known through the course of data analysis.

**Table 4**

| | Total Reads (millions) | Aligned reads (%) | On-target reads (%) |
|---|---|---|---|
| Model Training (N=576) | 2.892 | 93.7 | 72.4 |

### Fetal sex determination

A small fraction of total assays designed were targeted to chromosome Y; for each sample, the percentage of total sequencing reads mapping to chromosome Y was calculated using assays uniquely targeting chromosome Y regions. Good separation between male and female samples were observed (Figure 5). The percentage of chromosome Y reads from male fetus samples was >0.0435% and the percentage of chromosome Y sequencing reads from female fetus samples was <0.0159% with one discordant case.

### Relative quantitation of fetal DNA

Fetal fraction in each sample were estimated using allele frequencies measured from a panel of 150 segregating SNP loci and compared to an estimation method based on whole genome sequence data [41] from independent aliquots of each sample processed using the MaterniT21^{®} PLUS Assay (Sequenom). Two samples exhibited outlier behavior and displayed high variance in the SNP loci fetal fraction estimation. Nevertheless, estimation of relative abundance of fetal DNA in all samples was highly correlated with the orthogonal whole-genome sequence based estimate (r² = 0.82, p<10⁻¹⁵ , F-test, Figure 6).

### Z-score classification

At minimum, optimal assays for aneuploidy classification require sufficient depth of sequencing coverage, small variance expressed as assay MAD, and measurable signal differentiating aneuploid and euploid populations as measured by the p-value from the Wilcoxon rank sum test on log-transformed assay ratios. Using a grid search approach, the optimal assay sets were determined independently for z-score based fetal trisomy 21 and trisomy 18 classification. Specifically, 305 chromosome 21 assays with coverage > 700, MAD < 0.28, p-value < 0.1, and no evidence for CNV were identified as the optimal feature set for z-score based fetal trisomy 21 classification. The optimal thresholds for chromosome 18 assays were relaxed due to the small number of training chromosome 18 fetal trisomy samples. 296 chromosome 18 assays with coverage>200, MAD<0.3 and p-value<0.3 were selected as the optimal feature set for z-score based fetal trisomy 18 classification. The specific chromosome 21 and chromosome 18 assays utilized for classification are designated in Fig. 8 by a "+" in the last column of the table (included in final).

Optimal features were used to calculate z-scores for all training set samples. A trisomy sample was identified when the z-score exceeded 4 for chromosome 21 or 4.5 for chromosome 18; no positive or negative classification of fetal trisomy could be made when the z-score was between 2.5 and 4.0 for chromosome 21 or between 3.0 and 4.5 for chromosome 18. The resulting filtered training data were validated against independent aliquots of each sample processed using the MaterniT21^{®} PLUS Assay (Sequenom) and suggested sensitivity >96% and specificity >99% for fetal aneuploidy detection with indeterminate results in <3% of samples could be achieved (Figure 7). Several variations of the z-score based classification methods were also evaluated. These included weighting z-scores by assay variance or discriminatory power and merging assays based on physical location and assay correlations prior to z-score calculation. These alternative z-score based classification methods yield, at best similar performance to the standard z-score method described and were not pursued further.

### Artificial neural network based classification

Implementation of artificial neural networks with seven principal components for aneuploidy classification yielded the maximum sensitivity and specificity among all of the models tested, at 97% and 99% predicted, respectively. Using artificial neural network classification, samples containing 5-10% fetal DNA were ambiguously assigned; consequently, no positive or negative classification of fetal trisomy could be made when the artificial neural network regression model was between 0.05 and 0.95.

### Classification model performance

The final optimal classification of fetal aneuploidies employed the union of z-score and artificial neural network results to identify fetal trisomies only when both approaches were concordant. Fetal aneuploidy classification results for classification model training are shown in Table 5. Classification model training data were used to establish heuristic thresholds for subsequent blinded classification. Classifications herein therefore represent best case classification scenarios with respect to MaterniT21^{®} PLUS Assay ground truth data. Seventeen samples with estimated fetal (placental) origin DNA fraction <4% and ten samples for which identity could not be unambiguously assigned were excluded from the final analysis. No classification could be made in 5% and 3.6% of fetal trisomy 21 and fetal trisomy 18 cases, respectively. All other samples could be unambiguously and correctly classified in the model training dataset (Table 5).

**Table 5**

| Targeted Paralog Result | | MaterniT21^{®} PLUS Result | | |
|---|---|---|---|---|
| SAMPLE ACCEPTANCE | Total | Euploid | T18 | T21 |
| Failed fetal DNA fraction | 17 | 10 | 4 | 3 |
| Total Samples | 566 | 341 | 31 | 194 |
| Accepted samples | 549 | 331 | 27 | 191 |

| T21 SAMPLE CLASSIFICATION | Total | Euploid | T18 | T21 |
|---|---|---|---|---|
| Ambiguous classification | 26 | 14 | 0 | 12 |
| Discordant classification | 2 | 1 | 0 | 1 |
| Concordant classification | 521 | 316 | 27 | 178 |

| T18 SAMPLE CLASSIFICATION | Total | Euploid | T18 | T21 |
|---|---|---|---|---|
| Ambiguous classification | 19 | 11 | 5 | 3 |
| Discordant classification | 1 | 1 | 0 | 0 |
| Concordant classification | 529 | 319 | 22 | 188 |

### REFERENCES

1. Lo YM, Corbetta N, Chamberlain PF, Rai V, Sargent IL, et al. (1997) Presence of fetal DNA in maternal plasma and serum. Lancet 350: 485-487. doi: 10.1016/s0140-6736(97)02174-0
2. Lo YM, Tein MS, Lau TK, Haines CJ, Leung TN, et al. (1998) Quantitative analysis of fetal DNA in maternal plasma and serum: implications for noninvasive prenatal diagnosis. Am J Hum Genet 62: 768-775. doi: 10.1086/301800
3. Nygren AO, Dean J, Jensen TJ, Kruse S, Kwong W, et al. (2010) Quantification of fetal DNA by use of methylation-based DNA discrimination. Clin Chem 56: 1627-1635. doi: 10.1373/clinchem.2010.146290
4. Lo YM, Fun FM, Chan KC, Tsui NB, Chong KC, et al. (2007) Digital PCR for the molecular detection of fetal chromosomal aneuploidy. Proc Natl Acad Sci USA 104:13116-13121. doi: 10.1073/pnas.0705765104
5. Fan HC, Quake SR (2007) Detection of aneuploidy with digital polymerase chain reaction. Anal Chem 79:7576-7579. doi: 10.1021/ac0709394
6. Chiu RW, Chan KC, Gao Y, Lau VY, Zheng W, et al. (2008) Noninvasive prenatal diagnosis of fetal chromosomal aneuploidy by massively parallel genomic sequencing of DNA in maternal plasma. Proc Natl Acad Sci U S A 105: 20458-20463. doi: 10.1073/pnas.0810641105
7. Fan HC, Blumenfeld YJ, Chitkara U, Hudgins L, Quake SR (2008) Noninvasive diagnosis of fetal aneuploidy by shotgun sequencing DNA from maternal blood. Proc Natl Acad Sci U S A 105: 16266-16271. doi: 10.1073/pnas.0808319105
8. Ehrich M, Deciu C, Zwiefelhofer T, Tynan JA, Cagasan L, et al. (2011) Noninvasive detection of fetal trisomy 21 by sequencing of DNA in maternal blood: a study in a clinical setting. Am J Obstet Gynecol 204: 205 e1-11. doi: 10.1016/j.ajog.2010.12.060
9. Palomaki GE, Kloza EM, Lambert-Messerlian GM, Haddow JE, Neveux LM, et al. (2011) DNA sequencing of maternal plasma to detect Down syndrome: An international clinical validation study. Genet Med 13: 913-920. doi: 10.1097/gim.0b013e3182368a0e
10. Chiu RW, Akolekar R, Zheng YW, Leung TY, Sun H, et al. (2011) Non-invasive prenatal assessment of trisomy 21 by multiplexed maternal plasma DNA sequencing: large scale validity study. BMJ 342: c7401. doi: http://dx.doi.org/10.1136/bmj.c7401
11. Sehnert AJ, Rhees B, Comstock D, de Feo E, Heilek G, et al. (2011) Optimal detection of fetal chromosomal abnormalities by massively parallel DNA sequencing of cell-free fetal DNA from maternal blood. Clin Chem doi:10.1373/clinchem.2011.165910.
12. Palomaki GE, Deciu C, Kloza EM, Lambert-Messerlian GM, Haddow JE, et al. (2012) DNA sequencing of maternal plasma reliably identifies trisomy 18 and trisomy 13 as well as Down syndrome: an international collaborative study. Genet Med 14: 296-305. doi: 10.1038/gim.2011.73
13. Dan S, Wang W, Ren J, Li Y, Hu H, Xu Z, et al. (2012) Clinical application of massively parallel sequencing-based prenatal noninvasive fetal trisomy test for trisomies 21 and 18 in 11,105 pregnancies with mixed risk factors. Prenat Diagn 232: 1225-32.
14. Bianchi DW, Platt LD, Goldberg JD, Abuhamad AZ, Sehnert AJ, et al. (2012) Genome-wide fetal aneuploidy detection by maternal plasma DNA sequencing. Obstet Gynecol 119: 890-901. doi: 10.1002/pd.4002
15. Jensen TJ, Zwiefelhofer T, Tim RC, Džakula Ž, Kim SK, et al. (2013) High-throughput massively parallel sequencing for fetal aneuploidy detection from maternal plasma. PLoS ONE 8: e57381. doi: 10.1371/journal.pone.0057381
16. Gil MM, Akolekar R, Quezada MS, Bregant B, Nicolaides KH (2014) Analysis of cell-free DNA in maternal blood in screening for aneuploidies: meta-analysis. Fetal Diagn Ther 35:156-173. doi: 10.1002/uog.14791
17. Dondorp W, deWert G, Bombard Y, Bianchi DW, Bergmann C, et al. (2015) Non-invasive prenatal testing for aneuploidy and beyond: challenges of responsible innovation in prenatal testing. European J Human Genetics 23(11):1438-1450. DOI: 10.1038/ejhg.2015.57
18. Fan HC, Quake SR (2010) Sensitivity of noninvasive prenatal detection of fetal aneuploidy from maternal plasma using shotgun sequencing is limited only by counting statistics. PLoS ONE 5: e10439. doi: 10.1371/journal.pone.0010439
19. Zhao C, Tynan J, Ehrich M, Hannum G, McCullough R, et al. (2015) Detection of fetal subchromosomal abnormalities by sequencing circulating cell-free DNA from maternal plasma. Clin Chem 61(4):608-616. doi: 10.1373/clinchem.2014.233312
20. Bianchi DW, Wilkins-Haug L (2014) Integration of noninvasive DNA testing for aneuploidy into prenatal care: What has happened since the rubber met the road? Clin Chem 60:78-87. doi: 10.1373/clinchem.2013.202663
21. Sparks AB, Wang ET, Struble CA, Barrett W, Stokowski R, et al. (2012) Selective analysis of cell-free DNA in maternal blood for evaluation of fetal trisomy. Prenat Diagn 32: 3-9. doi: 10.1002/pd.2922
22. Sparks AB, Struble CA, Wang ET, Song K, Oliphant A. (2012) Noninvasive prenatal detection and selective analysis of cell-free DNA obtained from maternal blood: evaluation for trisomy 21 and trisomy 18. Am J Obstet Gynecol 206: 319.e1-9. doi: 10.1016/j.ajog.2012.01.030
23. Ashoor G, Syngelaki A, Wagner M, Birdir C, Nicolaides KH. (2012) Chromosome-selective sequencing of maternal plasma cell-free DNA for first-trimester detection of trisomy 21 and trisomy 18. Am J Obstet Gynecol 206:322.e1-5. doi: 10.1016/j.ajog.2012.01.029
24. Zimmerman B, Hill M, Gemelos G, Demko Z, Banjevic M, et al. (2012) Noninvasive prenatal aneuploidy testing of chromosomes 13, 18, 21, X, and Y using targeted sequencing of polymorphic loci. Prenat Diagn 32:1233-1231. doi: 10.1002/pd.3993
25. Norton ME, Brar H, Weiss J, Karimi A, Laurent LC, et al. (2012) Non-Invasive Chromosomal Evaluation (NICE) Study: results of a multicenter prospective cohort study for detection of fetal trisomy 21 and trisomy 18. Am J Obstet Gynecol 207: 137 e1-8. doi: 10.1016/j.ajog.2012.05.021
26. Nicolaides KH, Syngelaki A, Gil M, Atanasova V, Markova D (2013) Validation of targeted sequencing of single-nucleotide polymorphisms for non-invasive prenatal detection of aneuploidy of chromosomes 13, 18, 21, X, and Y. Prenat Diagn 33:575-579. doi: 10.1002/pd.4103
27. Norton ME, Jacobsson B, Swarmy GK, Laurent LC, Ranzini AC, et al. (2015) Cell-free DNA analysis for noninvasive examination of trisomy. New Eng J Med doi: 10.1056/NEJMoa1407349
28. Redon R, Ishikawa S, Fitch KR, Feuk L, Perry GH, et al. (2006) Global variation in copy number in the human genome. Nature 444:444-454. doi:10.1038/nature05329
29. McCarroll SA, Altshuler DM (2007) Copy-number variation and association studies of human disease. Nat Genet 39: S37-S42. doi:10.1038/ng2080
30. Walker S, Janyakhantikul S, Armour JAL (2008) Multiplex paralogue ratio tests for accurate measurement of multiallelic CNVs. Genomics 93: 98-103 doi:10.1016/j.ygeno.2008.09.004
31. Ohno S (1970) Evolution by gene duplication. Springer, New York. ISBN 3642866611
32. Deutsch, S, Choudhury U, Merla G, Howald C, Sylvan A, et al. (2004) Detection of aneuploidies by paralogous sequence quantification. J Med Genet 41:908-915. doi:10.1136/jmg.2004.023184
33. Howald C, Merla G, Digilio MC, Amenta S, Lyle R, et al. (2006) Two high throughput technologies to detect aneuploidies identify new Williams-Beuren syndrome patients with atypical deletions. J Med Genet 43: 266-273. doi: 10.1136/jmg.2005.034009
34. Langmead B, Salzberg S (2012) Fast gapped-read alignment with Bowtie 2. Nature Methods 9:357-359. doi: 10.1038/nmeth.1923
35. Pages H, Aboyoun P, Gentleman R, DebRoy S (). Biostrings: String objects representing biological sequences, and matching algorithms. R package version 2.29.0.
36. R Core Team (2013). R: A language and environment for statistical computing. R Foundation for Statistical Computing, Vienna, Austria. ISBN 3-900051-07-0, URL http: //www.R-proj ect.org/.
37. Untergrasser A, Cutcutache I, Koressaar T, Ye J, Faircloth BC, Remm M, Rozen SG (2012) Primer3 - new capabilities and interfaces. Nucleic Acids Research 40(15):e115. doi: 10.1093/nar/gks596
38. Koressaar T, Remm M (2007) Enhancements and modifications of primer design program Primer3. Bioinformatics 23(10):1289-91
39. Database of Single Nucleotide Polymorphisms (dbSNP). Bethesda (MD): National Center for Biotechnology Information, National Library of Medicine. (dbSNP Build ID:137)
40. Guenther F (2012) Package 'neuralnet'. R package version 1.32.
41. Kim SK, Hannum G, Geis J, Tynan J, Hogg G, et al. (2015) Determination of fetal DNA fraction from the plasma of pregnanat women using sequence read counts. Prenat Diagn 35(8):810-805. doi: 10.1002/pd.4615
42. Nicolaides KH, Syngelaki A, Ashoor G, Birdir C, Touzet G (2012) Noninvasive prenatal testing for fetal trisomies in a routinely screened first-trimester population. Am J Obstet Gynecol 207:374.e1-6. doi: 10.1016/j.ajog.2012.08.033
43. Chandrananda D, Thorne NP, Bahlo M. (2015) High-resolution characterization of sequence signatures due to non-random cleavage of cell-free DNA. BMC Med Genomics 8:29. doi: 10.1186/s12920-015-0107-z
44. Snyder MW, Kircher M, Hill AJ, Daza RM, Shendure J. (2016) Cell-free DNA comprises an in vivo nucleosome footprint that informs its tissues-of-origin. Cell 164:57-68. doi: 10.1016/j.cell/2015.11.050
45. Yu SCY, Chan KCA, Zheng YWL, Jiang P, Liao GJW, Sun H, Akolekar R, et al. (2014) Size-based molecular diagnostics using plasma DNA for noninvasive prenatal testing. Proc Natl Acad Sci 111(23): 8583-8588. doi/10.1073/pnas.
46. Lo YMD, Chan KCA, Sun H, Chen EZ, Jiang P, Lun FMF, et al (2010) Maternal Plasma DNA Sequencing Reveals the Genome-Wide Genetic and Mutational Profile of the Fetus. Sci Trans Med 2(61):61ra91. DOI: 10.1126/scitranslmed.3001720
47. Lanman RB, Mortimr SA, Zill OA, Sebisanovic D, Lopez R, Blau S, et al. (2015) Analytical and clinical validation of a digital sequencing panel for quantitative, highly accurate evaluation of cell-free circulating tumor DNA. PLoS ONE 10(10):e0140712. doi: 10.1371/joumal.pone.0140712
   Citation of the above-mentioned patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

## Claims

1. A method for determining the presence or absence of a genetic variation, comprising:
(a) amplifying in a single reaction a plurality of sets of paralogous polynucleotide species from nucleic acid in a sample, the amplifying comprising contacting the nucleic acid with a plurality of amplification primers under amplification conditions to obtain a plurality of amplified sets of paralogous polynucleotide species, wherein
(i) each set of the plurality of amplified sets of paralogous polynucleotide species includes paralogous polynucleotide species located on a target chromosome and one or more reference chromosomes; and
(ii) each set of the plurality of paralogous polynucleotide species is amplified by a different single pair of amplification primers of the plurality of amplification primers and each primer of the plurality of amplification primers is complementary to less than 20 positions in a human genome;
(b) determining, for each set of the plurality of amplified sets of paralogous polynucleotide species the amount of the paralogous polynucleotide species from the target chromosome and the amount of the paralogous polynucleotide species from the one or more reference chromosomes;
(c) determining, for each set of the plurality of amplified sets, a paralog ratio between the amount of the paralogous polynucleotide species from the target chromosome and the amount of the paralogous polynucleotide species from the one or more reference chromosomes;
(d) determining, for the paralog ratio determined for each set of the plurality of amplified sets of paralogous polynucleotide species, a z-score;
(e) determining, for the sample, a sample z-score based on the z-score determined for each set of the plurality of amplified sets of paralogous polynucleotide species; and
(f) determining, for the sample, the presence or absence of the genetic variation based on the sample z-score.

2. The method of claim 1, wherein the nucleic acid is
(a) circulating cell free nucleic acid; and/or
(b) from a pregnant female comprising fetally derived and maternally derived nucleic acid.

3. The method of any one of claims 1 or 2, wherein the genetic variation is
(a) a copy number alteration; and/or
(b) a single nucleotide variation; and/or
(c) aneuploidy.

4. The method of any one of claims 1-3, wherein the plurality of sets of paralogous polynucleotide species comprises a number of sets so as to provide statistical accuracy for samples comprising minority DNA as the target, optionally at least 100, 250, or 500 sets.

5. The method of any one of claims 1 to 4, wherein the paralogous polynucleotide species in each of the sets
(i) have primer hybridization sequences with a degree of sequence similarity that a single pair of amplification primers hybridizes to the paralogous polynucleotide species of each of the sets; and/or
(ii) differ by one or more mismatch nucleotides, wherein preferably the determining the amount of each amplified paralogous polynucleotide species in each of the sets is by detecting the one or more mismatch nucleotides.

6. The method of any one of claims 1 to 5, wherein amplifying in a single reaction in (a) is at least 500 sets of paralogous polynucleotide species.

7. The method of any one of claims 2 to 6, comprising determining fetal sex by a process comprising contacting the circulating cell free nucleic acid in the single reaction in (a) with primers specific for Y-chromosome polynucleotides under amplification conditions and amplifying Y-chromosome polynucleotides; wherein there are preferably at least 10 Y-chromosome polynucleotides are amplified.

8. The method of any one of claims 2 to 7, comprising determining fetal fraction by a process comprising contacting the circulating cell free nucleic acid in the single reaction in (a) with primers specific for polynucleotides flanking or comprising single nucleotide polymorphic (SNP) loci under amplification conditions, and amplifying polynucleotides containing the SNP loci, wherein there are preferably at least 150 polynucleotides comprising single nucleotide polymorphic (SNP) loci that are amplified.

9. The method of any one of claims 1 to 8, wherein the target chromosome is
(i) chromosome 21 and the reference chromosome is an autosome other than chromosome 21; or
(ii) chromosome 18 and the reference chromosome is an autosome other than chromosome 18.

10. The method of any one of claims 1 to 8, wherein the sets of paralogous polynucleotide species comprise sets wherein the target chromosome is chromosome 21 and sets wherein the target chromosome is chromosome 18;
wherein there are preferably at least 250 sets of paralogous polynucleotide species for target chromosome 21 and there are at least 250 sets of paralogous polynucleotide species for target chromosome 18; more preferred at least 500 sets of paralogous polynucleotide species for target chromosome 21 and there are at least 500 sets of paralogous polynucleotide species for target chromosome 18.

11. The method of claim 10, comprising amplifying Y-chromosome polynucleotides and amplifying polynucleotides comprising single nucleotide polymorphic (SNP) loci; wherein there are preferably at least 10 Y-chromosome polynucleotides that are amplified and at least 150 polynucleotides comprising single nucleotide polymorphic (SNP) loci that are amplified.

12. The method of any one of claims 1 to 11, wherein the amplification primers for each of the paralogous polynucleotide species sets
(i) produce two equal-sized amplicons with a size between 60 to 100 bp; and/or
(ii) produce zero off-target amplification events allowing maximal 3-base pair mis-priming; and/or
(iii) do not overlap with any annotated single nucleotide variant with greater than 1% minor allele frequency; and/or
(iv) flank at least one position where the nucleotide sequence species in a set differ by one or more mismatch nucleotides; and/or
(v) amplify a paralogous polynucleotide species set that is at least 100-bp apart from each of the other sets.

13. The method of any one of claims 1 to 12, wherein after (a) each of the amplified sets of paralogous polynucleotide species are further amplified by universal PCR.

14. The method of any one of claims 1 to 13, comprising generating sequence reads from each of the amplified paralogous polynucleotide species sets by a sequencing process; wherein the sequence reads are preferably filtered for a minimum number of reads and no copy number variants; and preferably quantified to obtain counts; and
wherein determining a paralog ratio in (c) is preferably calculated as the ratio of the counts of the paralogous polynucleotide species on a target chromosome to the counts of the paralogous polynucleotide species on a reference chromosome; or logarithm-transformed to zero-center values.

15. The method of any one of claims 1 to 14, comprising determining a statistic for each of the paralog ratios or logarithm-transformed paralog ratios, wherein the statistic is preferably a z-score, wherein the z-score is a quotient of (a) subtraction product of (i) the logarithm-transformed paralog ratio, less (ii) a median of the logarithm-transformed paralog ratio of reference euploid samples, divided by (b) a MAD value derived from reference euploid samples; or a weighted z-score, wherein the weighted z-score is preferably calculated by weighting individual non-outlier z-scores paralog ratios according to assay MAD or discriminatory power.

16. The method of claim 15, comprising determining a sample statistic according to the statistic for each of the paralog ratios or logarithm-transformed paralog ratios, preferably comprising summing statistics for each of the sets; and preferably
(I) wherein a sample z-score is calculated by z-scores of a plurality paralogous polynucleotide species sets, with the exclusion of outliers of z greater than 4 MAD or less than -4 MAD; or
(II) comprising determining a sample z-score according to $Z = \frac{{\sum }_{i = 1}^{N} {z}_{i}}{\sqrt{N}} ,$ where *zᵢ* is the z-score of a non-outlier paralog ratio and N is the number of non-outlier paralogs; or
(III) wherein a sample z-score is calculated by weighted z-scores of a plurality paralogous polynucleotide species sets; or
(IV) comprising determining a sample weighted z-score according to ${Z}_{w} = \frac{{\sum }_{i = 1}^{N} {w}_{i} {z}_{i}}{\sqrt{{\sum }_{i = 1}^{N} {w}_{i}^{2}}} ,$ where *wᵢ* is the weight for each paralog ratio.

17. The method of any one of claims 1 to 16, wherein the paralogous polynucleotide species sets comprise one or more of the sets in Fig. 8.

18. The method of any one of claims 1 to 17, wherein the amplification primers comprise one or more pairs of the amplification primers in Figure 8.

19. The method of any one of claims 1, 2, 3(a), (b), 4-6, and 12-18, wherein the genetic variation is
(a) cancer;
(b) an inherited mutation; or
(c) a somatic mutation.

20. Use of a composition in the method of any one of claims 1 to 19, wherein the composition comprises a mixture of a plurality of amplification primer pairs that can amplify the plurality of sets of paralogous polynucleotide species of any of claims 1-19, wherein each primer of the plurality of amplification primer pairs is complementary to less than 20 positions in a human genome,
wherein the amplification primers for each of the paralogous polynucleotide species sets produces two equal-sized amplicons with a size between 60 to 100 bp;
wherein the amplification primers for each of the sets produces zero off-target amplification events allowing maximal 3-base pair mis-priming; and/or
wherein the amplification primers for each of the sets do not overlap with any annotated single nucleotide variant with greater than 1% minor allele frequency in dbSNP build137.

21. The use of claim 20, wherein the composition comprises a plurality of amplification primer pairs in Figure 8.

22. Use of a kit in the method of any one of claims 1 to 19, wherein the kit comprises the composition of claim 20 or 21 and a DNA polymerase.

23. A system for determining the presence or absence of a genetic variation, comprising
(a) a component for amplifying in a single reaction a plurality of sets of paralogous polynucleotide species from nucleic acid in a sample, the amplifying comprising contacting the nucleic acid with a plurality of amplification primers under amplification conditions to obtain a plurality of amplified sets of paralogous polynucleotide species, wherein
(i) each set of the plurality of amplified sets of paralogous polynucleotide species includes paralogous polynucleotide species located on a target chromosome and one or more reference chromosomes; and
(ii) each set of the plurality of paralogous polynucleotide species is amplified by a different single pair of amplification primers of the plurality of amplification primers and each primer of the plurality of amplification primers is complementary to less than 20 positions in a human genome;
(b) a component for determining, for each set of the plurality of amplified sets of paralogous polynucleotide species, the amount of the paralogous polynucleotide species from the target chromosome and the amount of the paralogous polynucleotide species from the one or more reference chromosomes;
(c) a component for:
(i) determining, for each set of the plurality of amplified sets, a paralog ratio between the amount of the paralogous polynucleotide species from the target chromosome and the amount of the paralogous polynucleotide species from the one or more reference chromosomes;
(ii) determining, for the paralog ratio determined for each set of the plurality of amplified sets of paralogous polynucleotide species, a z-score; and
(iii) determining, for the sample, a sample z-score based on the z-score determined for each set of the plurality of amplified sets of paralogous polynucleotide species; and
(d) a component for determining, for the sample, the presence or absence of the genetic variation based on the sample z-score.

24. The system of claim 23, wherein one or more of components (b)-(d) comprise a computer processor.

## Patentansprüche

1. Verfahren zum Bestimmen der Anwesenheit oder Abwesenheit einer genetischen Variation, umfassend:
(a) Amplifizieren, in einer einzigen Reaktion, einer Vielzahl von Sets paraloger Polynukleotidspezies von Nukleinsäure in einer Probe, das Amplifizieren umfassend In-Kontakt-Bringen der Nukleinsäure mit einer Vielzahl von Amplifikationsprimern unter Amplifikationsbedingungen, um eine Vielzahl von amplifizierten Sets paraloger Polynukleotidspezies zu erhalten, wobei
(i) jedes Set der Vielzahl von amplifizierten Sets paraloger Polynukleotidspezies paraloge Polynukleotidspezies beinhaltet, die auf einem Zielchromosom und einem oder mehreren Referenzchromosomen lokalisiert sind; und
(ii) jedes Set der Vielzahl von paralogen Polynukleotidspezies mit einem anderen einzelnen Paar von Amplifikationsprimern aus der Vielzahl von Amplifikationsprimern amplifiziert wird und jeder Primer der Vielzahl von Amplifikationsprimern zu weniger als 20 Positionen in einem menschlichen Genom komplementär ist;
(b) Bestimmen, für jedes Set aus der Vielzahl von amplifizierten Sets paraloger Polynukleotidspezies, der Menge der paralogen Polynukleotidspezies von dem Zielchromosom und der Menge der paralogen Polynukleotidspezies von dem einen oder den mehreren Referenzchromosomen;
(c) Bestimmen, für jedes Set aus der Vielzahl von amplifizierten Sets, eines Paralog-Verhältnisses zwischen der Menge der paralogen Polynukleotidspezies von dem Zielchromosom und der Menge der paralogen Polynukleotidspezies von dem einen oder den mehreren Referenzchromosomen;
(d) Bestimmen eines z-Score für das Paralog-Verhältnis, das für jedes Set aus der Vielzahl von amplifizierten Sets paraloger Polynukleotidspezies bestimmt wurde;
(e) Bestimmen eines Proben-z-Score für die Probe basierend auf dem z-Score, der für jedes Set der Vielzahl von amplifizierten Sets paraloger Polynukleotidspezies bestimmt wurde; und
(f) Bestimmen, für die Probe, der Anwesenheit oder Abwesenheit der genetischen Variation basierend auf dem Proben-z-Score.

2. Verfahren nach Anspruch 1, wobei die Nukleinsäure
(a) zirkulierende Zell-freie Nukleinsäure ist; und/oder
(b) von einer schwangeren Frau stammt, umfassend fötal abgeleitete und von der Mutter abgeleitete Nukleinsäure.

3. Verfahren nach einem der Ansprüche 1 oder 2, wobei die genetische Variation
(a) eine Veränderung der Kopienanzahl; und/oder
(b) eine Einzelnukleotidvariation; und/oder
(c) Aneuploidie;
ist.

4. Verfahren nach einem der Ansprüche 1 bis 3, wobei die Vielzahl von Sets paraloger Polynukleotidspezies eine Anzahl von Sets umfasst, die eine statistische Genauigkeit für Proben bereitstellt, die eine Minderheiten-DNA als Ziel umfassen, gegebenenfalls mindestens 100, 250, oder 500 Sets.

5. Verfahren nach einem der Ansprüche 1 bis 4, wobei die paralogen Polynukleotidspezies in jedem der Sets
(i) Primerhybridisierungssequenzen mit einem Grad von Sequenzähnlichkeit haben, sodass ein einziges Paar von Amplifikationsprimern an die paralogen Polynukleotidspezies von jedem der Sets hybridisiert; und/oder
(ii) sich durch eines oder mehrere Fehlpaarungsnukleotiden unterscheiden, wobei vorzugsweise das Bestimmen der Menge von jeder amplifizierten paralogen Polynukleotidspezies in jedem der Sets durch Detektieren des einen oder der mehreren Fehlpaarungsnukleotid(s/e) bewerkstelligt wirkt.

6. Verfahren nach einem der Ansprüche 1 bis 5, wobei Amplifizieren in einer einzelnen Reaktion in (a) mindestens 500 Sets paraloger Polynukleotidspezies betrifft.

7. Verfahren nach einem der Ansprüche 2 bis 6, umfassend das Bestimmen des fötalen Geschlechts mittels eines Verfahrens umfassend In-Kontakt-Bringen der zirkulierenden Zell-freien Nukleinsäure in der einzelnen Reaktion in (a) mit Primern, die spezifisch für Y-Chromosom-Polynukleotide sind, unter Amplifikationsbedingungen, und Amplifizieren von Y-Chromosom-Polynukleotiden; wobei vorzugsweise mindestens 10 Y-Chromosom-Polynukleotide amplifiziert werden.

8. Verfahren nach einem der Ansprüche 2 bis 7, umfassend das Bestimmen des fötalen Anteils mittels eines Verfahrens umfassend In-Kontakt-Bringen der zirkulierenden Zell-freien Nukleinsäure in der einzelnen Reaktion in (a) mit Primern, die spezifisch für Polynukleotide sind, die Einzelnukleotidpolymorphe ("single nucleotide polymorphic") (SNP)-Loci flankieren oder umfassen, unter Amplifikationsbedingungen, und Amplifizieren von Polynukleotiden, die die SNP-Loci enthalten, wobei vorzugsweise mindestens 150 Polynukleotide, die Einzelnukleotid-polymorphe(SNP)-Loci umfassen, amplifiziert werden.

9. Verfahren nach einem der Ansprüche 1 bis 8, wobei das Zielchromosom
(i) Chromosom 21 und das Referenzchromosom ein anderes Autosom als Chromosom 21 ist; oder
(ii) Chromosom 18 und das Referenzchromosom ein anderes Autosom als Chromosom 18 ist.

10. Verfahren nach einem der Ansprüche 1 bis 8, wobei die Sets paraloger Polynukleotidspezies Sets, in denen das Zielchromosom Chromosom 21 ist, und Sets, in denen das Zielchromosom Chromosom 18 ist, umfassen;
wobei vorzugsweise mindestens 250 Sets paraloger Polynukleotidspezies für Zielchromosom 21 vorliegen und mindestens 250 Sets paraloger Polynukleotidsequenzen für Zielchromosom 18 vorliegen; bevorzugter mindestens 500 Sets paraloger Polynukleotidsequenzen für Zielchrom 21 vorliegen und mindestens 500 Sets paraloger Polynukleotidsequenzen für Zielchromosom 18 vorliegen.

11. Verfahren nach Anspruch 10, umfassend Amplifizieren von Y-Chromosom-Polynukleotiden und Amplifizieren von Polynukleotiden die Einzelnukleotid-polymorphe(SNP)-Loci umfassen; wobei vorzugsweise mindestens 10 Y-Chromosom-Polynukleotide vorliegen, die amplifiziert werden und mindestens 150 Polynukleotide, die Einzelnukleotid-polymorphe(SNP)-Loci umfassen, vorliegen, die amplifiziert werden.

12. Verfahren nach einem der Ansprüche 1 bis 11, wobei die Amplifikationsprimer für jedes der paralogen Polynukleotidspeziessets
(i) zwei gleichgroße Amplikons mit einer Größe zwischen 60 bis 100 bp generieren; und/oder
(ii) null off-target-Amplifikationsereignisse generieren, die maximal 3-Basenpaar-Mispriming erlauben; und/oder
(iii) nicht mit einer annotierten Einzelnukleotidvariante, die eine größere als 1%ige Minderallelhäufigkeit hat, überlappen;
(iv) mindestens eine Stelle flankieren, an welcher sich die Nukleotidsequenzspezies in einem Set durch eine oder mehrere Fehlpaarungsnukleotide unterscheiden; und/oder
(v) ein paraloges Polynukleotidspeziesset amplifizieren, das mindestens 100-bp von jedem der anderen Sets entfernt ist.

13. Verfahren nach einen der Ansprüche 1 bis 12, wobei nach (a) jedes der amplifizierten Sets paraloger Polynukleotidspezies mittels universeller PCR weiteramplifiziert wird.

14. Verfahren nach einem der Ansprüche 1 bis 13, umfassend das Generieren von Sequenzleseeinheiten von jedem der amplifizierten paralogen Polynukleotidspeziessets mittels eines Sequenzierungsverfahrens; wobei die Sequenzleseeinheiten vorzugsweise bezüglich einer Mindestanzahl von Leseeinheiten und unter Ausschluss von Kopienanzahlvarianten gefiltert werden; und vorzugsweise quantifiziert werden, um Anzahlen zu erhalten; und wobei das Bestimmen eines Paralog-Verhältnisses in (c) vorzugsweise als Verhältnis der Anzahlen der paralogen Polynukleotidspezies auf einem Zielchromosom zu den Anzahlen der paralogen Polynukleotidspezies auf einem Referenzchromosom; oder als Logarithmus-transformierte zu Nullzentrierte ("zero-center") Werte berechnet werden.

15. Verfahren nach einem der Ansprüche 1 bis 14, umfassend das Bestimmen einer Statistik für jedes der Paralog-Verhältnisse oder Logarithmus-transformierten Paralog-Verhältnisse, wobei die Statistik vorzugsweise ein z-Score ist, wobei der z-Score ein Quotient von (a) eines Subtraktionsprodukts von (i) dem Logarithmus-transformierten Paralog-Verhältnis, abzüglich (ii) eines Medians von dem Logarithmus-transformierten Paralog-Verhältnis von euploiden Referenzproben, geteilt durch (b) einen MAD-Wert abgeleitet von euploiden Referenzproben ist; oder einem gewichteten z-Score, wobei der gewichtete z-Score vorzugsweise durch Gewichten individueller nicht-Ausreißer-z-Score-Paralog-Verhältnisse gemäß Assay-MAD oder Trennschärfe berechnet wird.

16. Verfahren nach Anspruch 15, umfassend das Bestimmen einer Proben-Statistik entsprechend der Statistik für jedes der Paralog-Verhältnisse oder Logarithmus-transformierten Paralog-Verhältnisse, vorzugsweise umfassend Summieren von Statistiken für jedes der Sets; und vorzugsweise
(I) wobei ein Proben-z-Score mittels z-Score-Werten von einer Vielzahl paraloger Polynukleotidspeziessets, mit dem Ausschluss von Ausreißern von z größer als 4 MAD oder kleiner als -4 MAD, berechnet wird; oder
(II) umfassend das Bestimmen eines Proben-z-Score entsprechend $Z = \frac{{\sum }_{i = 1}^{N} {z}_{i}}{\sqrt{N}} ,$ wobei *zᵢ* der z-Score von einem Nicht-Ausreißer-Paralogverhältnis und N die Anzahl von Nicht-Ausreißer-Paralogen ist; oder
(III) wobei ein Proben-z-Score mittels gewichteter z-Score-Werten von einer Vielzahl paraloger Polynukleotidspeziessets berechnet wird; oder
(IV) umfassend Bestimmen eines Proben-gewichteten z-Score entsprechend ${Z}_{w} = \frac{{\sum }_{i = 1}^{N} {w}_{i} {z}_{i}}{\sqrt{{\sum }_{i = 1}^{N} {w}_{i}^{2}}} ,$ wobei *wᵢ* das Gewicht für jedes Paralog-Verhältnis ist.

17. Verfahren nach einem der Ansprüche 1 bis 16, wobei die paralogen Polynukleotidspeziessets eines oder mehrere der Sets aus Fig. 8 umfassen.

18. Verfahren nach einem der Ansprüche 1 bis 17, wobei die Amplifikationsprimer eines oder mehrere Paare der Amplifikationsprimer aus Fig. 8 umfassen.

19. Verfahren nach einem der Ansprüche 1, 2, 3(a), (b), 4 bis 6 und 12 bis 18 wobei die genetische Variation
(a) Krebs;
(b) eine vererbte Mutation; oder
(c) eine somatische Mutation
ist.

20. Verwendung einer Zusammensetzung in dem Verfahren nach einem der Ansprüche 1 bis 19, wobei die Zusammensetzung ein Gemisch einer Vielzahl von Amplifikationsprimerpaaren umfasst, die die Vielzahl von Sets paraloger Polynukleotidspezies nach einem der Ansprüche 1 bis 19 amplifizieren können, wobei jeder Primer aus der Vielzahl von Amplifikationsprimerpaaren zu weniger als 20 Positionen in einem menschlichen Genom komplementär ist,
wobei die Amplifikationsprimer für jedes der paralogen Polynukleotidspeziessets zwei gleichgroße Amplikons mit einer Größe zwischen 60 bis 100 bp generieren;
wobei die Amplifikationsprimer für jedes der Sets null off-target-Amplifikationsereignisse generieren, und maximal 3-Basenpaar-Misprimings erlauben;
und/oder
wobei die Amplifikationsprimer für jedes der Sets nicht mit einer annotierten Einzelnukleotidvariante die mehr als 1% Minderallelhäufigkeit in der dbSNP build137 hat, überlappen.

21. Verwendung nach Anspruch 20, wobei die Zusammensetzung eine Vielzahl von Amplifikationsprimerpaaren aus Fig. 8 umfasst.

22. Verwendung eines Kits in dem Verfahren nach einem der Ansprüche 1 bis 19, wobei das Kit die Zusammensetzung nach Anspruch 20 oder 21 und eine DNA-Polymerase umfasst.

23. System zum Bestimmen der Anwesenheit oder Abwesenheit einer genetischen Variation, umfassend:
(a) eine Komponente zum Amplifizieren, in einer einzigen Reaktion, einer Vielzahl von Sets paraloger Polynukleotidspezies von Nukleinsäure in einer Probe, das Amplifizieren umfassend In-Kontakt-Bringen der Nukleinsäure mit einer Vielzahl von Amplifikationsprimern unter Amplifikationsbedingungen, um eine Vielzahl von amplifizierten Sets paraloger Polynukleotidspezies zu erhalten, wobei
(i) jedes Set der Vielzahl von amplifizierten Sets paraloger Polynukleotidspezies paraloge Polynukleotidspezies beinhaltet, die auf einem Zielchromosom und einem oder mehreren Referenzchromosomen lokalisiert sind; und
(ii) jedes Set der Vielzahl von paralogen Polynukleotidspezies mit einem anderen einzelnen Paar von Amplifikationsprimern aus der Vielzahl von Amplifikationsprimern amplifiziert wird und jeder Primer der Vielzahl von Amplifikationsprimern zu weniger als 20 Positionen in einem menschlichen Genom komplementär ist;
(b) eine Komponente zum Bestimmen, für jedes Set aus der Vielzahl von amplifizierten Sets paraloger Polynukleotidspezies, der Menge der paralogen Polynukleotidspezies von dem Zielchromosom und der Menge der paralogen Polynukleotidspezies von dem einen oder den mehreren Referenzchromosomen;
(c) eine Komponente zum:
(i) Bestimmen, für jedes Set aus der Vielzahl von amplifizierten Sets, eines Paralog-Verhältnisses zwischen der Menge der paralogen Polynukleotidspezies von dem Zielchromosom und der Menge der paralogen Polynukleotidspezies von dem einen oder den mehreren Referenzchromosomen;
(ii) Bestimmen eines z-Score für das Paralog-Verhältnis, das für jedes Set aus der Vielzahl von amplifizierten Sets paraloger Polynukleotidspezies bestimmt wurde;
(iii) Bestimmen eines Proben-z-Score für die Probe basierend auf dem z-Score, der für jedes Set aus der Vielzahl von amplifizierten Sets paraloger Polynukleotidspezies bestimmt wurde; und
(d) eine Komponente zum Bestimmen, für die Probe, der Anwesenheit oder Abwesenheit einer genetischen Variation basierend auf dem Proben-z-Score.

24. System nach Anspruch 23, wobei eine oder mehrere der Komponenten (b)-(d) einen Computerprozessor umfassen.

## Revendications

1. Méthode de détermination de la présence ou de l'absence d'une variation génétique, comprenant :
(a) l'amplification, dans une seule réaction, d'une pluralité d'ensembles d'espèces de polynucléotides paralogues à partir de l'acide nucléique dans un échantillon, l'amplification comprenant la mise en contact de l'acide nucléique avec une pluralité d'amorces d'amplification dans des conditions d'amplification pour obtenir une pluralité d'ensembles amplifiés d'espèces de polynucléotides paralogues, dans laquelle
(i) chaque ensemble de la pluralité d'ensembles amplifiés d'espèces de polynucléotides paralogues comprend des espèces de polynucléotides paralogues localisées sur un chromosome cible et un ou plusieurs chromosomes de référence ; et
(ii) chaque ensemble de la pluralité d'espèces de polynucléotides paralogues est amplifié par une seule paire différente d'amorces d'amplification de la pluralité d'amorces d'amplification et chaque amorce de la pluralité d'amorces d'amplification est complémentaire à moins de 20 positions dans un génome humain ;
(b) la détermination, pour chaque ensemble de la pluralité d'ensembles amplifiés d'espèces de polynucléotides paralogues, de la quantité d'espèces de polynucléotides paralogues à partir du chromosome cible et de la quantité d'espèces de polynucléotides paralogues à partir de l'un ou de plusieurs chromosomes de référence ;
(c) la détermination, pour chaque ensemble de la pluralité d'ensembles amplifiés, d'un rapport paralogue entre la quantité d'espèces de polynucléotides paralogues à partir du chromosome cible et la quantité d'espèces de polynucléotides paralogues à partir de l'un ou de plusieurs chromosomes de référence ;
(d) la détermination, pour le rapport paralogue déterminé pour chaque ensemble de la pluralité d'ensembles amplifiés d'espèces de polynucléotides paralogues, d'un score z ;
(e) la détermination, pour l'échantillon, d'un score z d'échantillon sur la base du score z déterminé pour chaque ensemble de la pluralité d'ensembles amplifiés d'espèces de polynucléotides paralogues ; et
(f) la détermination, pour l'échantillon, de la présence ou de l'absence de la variation génétique sur la base du score z d'échantillon.

2. Méthode selon la revendication 1, dans laquelle l'acide nucléique est
(a) un acide nucléique acellulaire circulant ; et/ou
(b) d'une femme enceinte, comprenant un acide nucléique dérivé du fœtus et dérivé de la mère.

3. Méthode selon l'une quelconque des revendications 1 ou 2, dans laquelle la variation génétique est
(a) une altération du nombre de copies ; et/ou
(b) une variation d'un seul nucléotide ; et/ou
(c) une aneuploïdie.

4. Méthode selon l'une quelconque des revendications 1 à 3, dans laquelle la pluralité d'ensembles d'espèces de polynucléotides paralogues comprend un certain nombre d'ensembles de manière à fournir une précision statistique pour des échantillons comprenant une minorité d'ADN comme cible, optionnellement au moins 100, 250, ou 500 ensembles.

5. Méthode selon l'une quelconque des revendications 1 à 4, dans laquelle les espèces de polynucléotides paralogues dans chacun des ensembles
(i) ont des séquences d'amorces d'hybridation ayant un degré de similarité de séquence tel qu'une paire unique d'amorces d'amplification s'hybride aux espèces de polynucléotides paralogues de chacun des ensembles ; et/ou
(ii) diffèrent au niveau de l'un ou de plusieurs nucléotides de mésappariement, où la détermination de la quantité de chaque espèce de polynucléotides paralogues amplifiées dans chacun des ensembles s'effectue par détection des un ou plusieurs nucléotides de mésappariement.

6. Méthode selon l'une quelconque des revendications 1 à 5, dans laquelle l'amplification dans une seule réaction à l'étape (a) est d'au moins 500 ensembles d'espèces de polynucléotides paralogues.

7. Méthode selon l'une quelconque des revendications 2 à 6, comprenant la détermination du sexe du fœtus par un procédé comprenant la mise en contact de l'acide nucléique acellulaire circulant dans la seule réaction à l'étape (a) avec des amorces spécifiques pour les polynucléotides du chromosome Y dans des conditions d'amplification et l'amplification des polynucléotides du chromosome Y ; où de préférence au moins 10 polynucléotides du chromosome Y sont amplifiés.

8. Méthode selon l'une quelconque des revendications 2 à 7, comprenant la détermination de la fraction du fœtus par un procédé comprenant la mise en contact de l'acide nucléique acellulaire circulant dans la seule réaction à l'étape (a) avec des amorces spécifiques pour des polynucléotides flanquant ou comprenant des loci de polymorphisme d'un seul nucléotide (PSN) dans des conditions d'amplification et l'amplification des polynucléotides contenant les loci de PSN, où de préférence au moins 150 polynucléotides comprenant des loci de polymorphisme d'un seul nucléotide (PSN) sont amplifiés.

9. Méthode selon l'une quelconque des revendications 1 à 8, dans laquelle le chromosome cible est
(i) le chromosome 21 et le chromosome de référence est un autosome autre que le chromosome 21 ; ou
(ii) le chromosome 18 et le chromosome de référence est un autosome autre que le chromosome 18.

10. Méthode selon l'une quelconque des revendications 1 à 8, dans laquelle les ensembles d'espèces de polynucléotides paralogues comprennent des ensembles où le chromosome cible est le chromosome 21 et des ensembles où le chromosome cible est le chromosome 18 ;
dans laquelle il y a de préférence au moins 250 ensembles d'espèces de polynucléotides paralogues pour le chromosome cible 21 et il y a au moins 250 ensembles d'espèces de polynucléotides paralogues pour le chromosome cible 18 ; de manière encore plus préférée, il y a au moins 500 ensembles d'espèces de polynucléotides paralogues pour le chromosome cible 21 et il y a au moins 500 ensembles d'espèces de polynucléotides paralogues pour le chromosome cible 18.

11. Méthode selon la revendication 10, comprenant l'amplification des polynucléotides du chromosome Y et l'amplification des polynucléotides comprenant des loci de polymorphisme d'un seul nucléotide (PSN) ; dans laquelle il y a de préférence au moins 10 polynucléotides du chromosome Y qui sont amplifiés et au moins 150 polynucléotides comprenant des loci de polymorphisme d'un seul nucléotide (PSN) qui sont amplifiés.

12. Méthode selon l'une quelconque des revendications 1 à 11, dans laquelle les amorces d'amplification pour chacun des ensembles d'espèces de polynucléotides paralogues
(i) produisent deux amplicons de taille égale, ayant une taille entre 60 et 100 bp ; et/ou
(ii) produisent zéro événement d'amplification hors cible permettant le mauvais amorçage d'au maximum 3 paires de base ; et/ou
(iii) ne se chevauchent pas à plus de 1 % de fréquence d'allèle mineure avec un quelconque variant mononucléotidique annoté; et/ou
(iv) flanquent au moins une position où les espèces de séquences de nucléotide dans un ensemble diffèrent au niveau de l'un ou de plusieurs nucléotides de mésappariement ; et/ou
(v) amplifient un ensemble d'espèces de polynucléotides paralogues qui est écarté d'au moins 100 bp de chacun des autres ensembles.

13. Méthode selon l'une quelconque des revendications 1 à 12, dans laquelle, après l'étape (a), chacun des ensembles amplifiés d'espèces de polynucléotides paralogues sont en outre amplifiés par PCR universelle.

14. Méthode selon l'une quelconque des revendications 1 à 13, comprenant la génération de lectures de séquence à partir de chacun des ensembles d'espèces de polynucléotides paralogues par un processus de séquençage ; dans laquelle les lectures de séquence sont de préférence filtrées pour un nombre minimal de lectures et aucun variant de nombre de copie ; et de préférence quantifiées pour obtenir des comptages ; et
dans laquelle la détermination d'un rapport paralogue à l'étape (c) est de préférence calculée comme étant le rapport entre les comptages des espèces de polynucléotides paralogues sur un chromosome cible et les comptages des espèces de polynucléotides paralogues sur un chromosome de référence ; ou transformées logarithmiquement en valeurs de centre zéro.

15. Méthode selon l'une quelconque des revendications 1 à 14, comprenant la détermination d'une statistique pour chacun des rapports paralogues ou des rapports paralogues transformés logarithmiquement, dans laquelle la statistique est de préférence un score z, dans laquelle le score z est un quotient de (a) un produit de soustraction du (i) rapport paralogue transformé logarithmiquement, moins (ii) une médiane du rapport paralogue transformé logarithmiquement d'échantillons euploïdes de référence, divisé par (b) une valeur MAD dérivée des échantillons euploïdes de référence ; ou un score z pondéré, où le score z pondéré est de préférence calculé par pondération des rapports paralogues de scores z non aberrants individuels conformément à l'analyse MAD ou le pouvoir discriminant.

16. Méthode selon la revendication 15, comprenant la détermination d'une statistique d'échantillon conformément à la statistique pour chacun des rapports paralogues ou rapports paralogues transformés logarithmiquement, de préférence comprenant des statistiques de somme pour chacun des ensembles ; et de préférence
(I) dans laquelle un score z d'échantillon est calculé par des scores z d'une pluralité d'ensembles d'espèces de polynucléotides paralogues, à l'exclusion des valeurs aberrantes de z supérieures à 4 MAD ou inférieures à -4 MAD ; ou
(II) comprenant la détermination d'un score z d'échantillon conformément à $Z = \frac{{\sum }_{i = 1}^{N} {z}_{i}}{\sqrt{N}} ,$ où *zᵢ* est le score z d'un rapport paralogue non aberrant et N est le nombre de paralogues non aberrants ; ou
(III) dans laquelle un score z d'échantillon est calculé par des scores z pondérés d'une pluralité d'ensembles d'espèces de polynucléotides paralogues ; ou
(IV) comprenant la détermination d'un score z d'échantillon pondéré conformément à ${Z}_{w} = \frac{{\sum }_{i = 1}^{N} {w}_{i} {z}_{i}}{\sqrt{{\sum }_{i = 1}^{N} {w}_{i}^{2}}} ,$ où *wᵢ* est la pondération pour chaque rapport paralogue.

17. Méthode selon l'une quelconque des revendications 1 à 16, dans laquelle les ensembles d'espèces de polynucléotides paralogues comprennent un ou plusieurs des ensembles de la figure 8.

18. Méthode selon l'une quelconque des revendications 1 à 17, dans laquelle les amorces d'amplification comprennent une ou plusieurs paires des amorces d'amplification de la figure 8.

19. Méthode selon l'une quelconque des revendications 1, 2, 3(a), (b), 4 à 6, et 12 à 18, dans laquelle la variation génétique est
(a) un cancer ;
(b) une mutation héréditaire ; ou
(c) une mutation somatique.

20. Utilisation d'une composition dans la méthode selon l'une quelconque des revendications 1 à 19, dans laquelle la composition comprend un mélange d'une pluralité de paires d'amorces d'amplification qui peut amplifier la pluralité d'ensembles d'espèces de polynucléotides paralogues selon l'une quelconque des revendications 1 à 19, dans laquelle chaque amorce de la pluralité de paires d'amorces d'amplification est complémentaire à moins de 20 positions dans un génome humain,
dans laquelle les amorces d'amplification pour chacun des ensembles d'espèces de polynucléotides paralogues produisent deux amplicons de taille égale, ayant une taille entre 60 et 100 bp ;
dans laquelle les amorces d'amplification pour chacun des ensembles produisent zéro événement d'amplification hors cible permettant le mauvais amorçage d'au maximum 3 paires de base ; et/ou
dans laquelle les amorces d'amplification pour chacun des ensembles ne se chevauchent pas à plus de 1 % de fréquence d'allèle mineure dans le PSNdb buildl37 avec un quelconque variant mononucléotidique annoté.

21. Utilisation selon la revendication 20, dans laquelle la composition comprend une pluralité de paires d'amorces d'amplification de la figure 8.

22. Utilisation d'un kit dans la méthode selon l'une quelconque des revendications 1 à 19, dans laquelle le kit comprend la composition selon la revendication 20 ou 21 et une ADN polymérase.

23. Système de détermination de la présence ou de l'absence d'une variation génétique, comprenant :
(a) un composant pour l'amplification, dans une seule réaction, d'une pluralité d'ensembles d'espèces de polynucléotides paralogues à partir de l'acide nucléique dans un échantillon, l'amplification comprenant la mise en contact de l'acide nucléique avec une pluralité d'amorces d'amplification dans des conditions d'amplification pour obtenir une pluralité d'ensembles amplifiés d'espèces de polynucléotides paralogues, dans laquelle
(i) chaque ensemble de la pluralité d'ensembles amplifiés d'espèces de polynucléotides paralogues comprend des espèces de polynucléotides paralogues localisées sur un chromosome cible et un ou plusieurs chromosomes de référence ; et
(ii) chaque ensemble de la pluralité d'espèces de polynucléotides paralogues est amplifié par une seule paire différente d'amorces d'amplification de la pluralité d'amorces d'amplification et chaque amorce de la pluralité d'amorces d'amplification est complémentaire à moins de 20 positions dans un génome humain ;
(b) un composant pour la détermination, pour chaque ensemble de la pluralité d'ensembles amplifiés d'espèces de polynucléotides paralogues, de la quantité d'espèces de polynucléotides paralogues à partir du chromosome cible et de la quantité d'espèces de polynucléotides paralogues à partir de l'un ou de plusieurs chromosomes de référence ;
(c) un composant pour :
(i) la détermination, pour chaque ensemble de la pluralité d'ensembles amplifiés, d'un rapport paralogue entre la quantité d'espèces de polynucléotides paralogues à partir du chromosome cible et la quantité d'espèces de polynucléotides paralogues à partir de l'un ou de plusieurs chromosomes de référence ;
(ii) la détermination, pour le rapport paralogue déterminé pour chaque ensemble de la pluralité d'ensembles amplifiés d'espèces de polynucléotides paralogues, d'un score z ; et
(iii) la détermination, pour l'échantillon, d'un score z d'échantillon sur la base du score z déterminé pour chaque ensemble de la pluralité d'ensembles amplifiés d'espèces de polynucléotides paralogues ; et
(d) un composant pour la détermination, pour l'échantillon, de la présence ou de l'absence de la variation génétique sur la base du score z d'échantillon.

24. Système selon la revendication 23, dans lequel un ou plusieurs des composants (b) à (d) comprennent un processeur informatique.
